# EUROPEAN PATENT APPLICATION

(11) **EP 4 826 737 A1**
(43) Date of publication of application: **21.10.2026**
(21) Application number: 26167748.8
(22) Date of filing: 25.03.2026
(51) Int. Cl.: G16H 50/20, G06N 3/02, G06N 3/0475, G06N 3/0895, G16H 10/60, G16H 15/00, G16H 20/00, G16H 40/20, G16H 40/60, G16H 50/70, G16H 10/40

(54) **METHODS AND SYSTEMS FOR DECISION MAKER FOCUS AND TREATMENT PLANNING USING LONGITUDINAL PATIENT TIMELINES**

(30) Priority: 17.04.2025 US 202519182538
(71) Applicant: GE Precision Healthcare LLC, Waukesha, WI 53188 (US)
(72) Inventor: DIVINE, Lucas Jason, Waukesha, 53188 (US); KESHET, Renato, Haifa (IL); PALANI, Shanmugam, 560066 Bengaluru (IN); BHARDWAJ, Annamraju Ravi, 560037 Bengaluru (IN); MANI, Sundararajan, Brookfield, 53045 (US)
(74) Representative: Kilburn & Strode LLP

(57) **Abstract**

Medical decision makers are important to healthcare. Systems and methods are proposed herein to provide a decision maker focused user interface (UI). The systems and method also allow, via the innovative UI, treatment planning and decision maker learning by employing longitudinal patient timelines (510, 600). Decision points of the patient timeline are identified, and learnings are provided based on the events and outcomes based on the decision. These learnings include comparisons with other similar patients, decisions by other luminary decision makers, and aha moments.

## Description

### BACKGROUND

The subject disclosure relates to medical treatment of patients using generative artificial intelligence (AI) and user interfaces (UIs) and, more specifically, to systems and methods for decision maker focus and treatment planning using longitudinal patient timelines.

Clinicians making treatment decisions for complex medical conditions, like cancer or rare diseases with ever changing medical guidelines, may not always have all the information to make the best decision in a particular moment for a particular patient. Second opinions are often sought by patients due to the relatively high rate of unsatisfactory treatment plans and new cutting edge potential treatments. Improved technology that provides better diagnosis, better information for clinicians, including retrospective views to help them improve as decision makers, and treatment insights is needed. Intuitive UIs that make absorbing such information easy for humans are also needed.

### SUMMARY

The following presents a summary to provide a basic understanding of one or more embodiments described herein. This summary is not intended to identify key or critical elements, delineate scope of particular embodiments or scope of claims. Its sole purpose is to present concepts in a simplified form as a prelude to the more detailed description that is presented later. In one or more embodiments described herein, systems, computer-implemented methods, apparatus and/or computer program products that enable generative AI-based solutions to evaluate treatment options for patients are discussed.

According to an embodiment, a system is provided. The system can comprise a memory that can store computer-executable components. The system can further comprise a processor that can execute the computer-executable components stored in the memory, where the computer-executable components can comprise a data access component that can access first data comprising first patient data associated with a patient, second patient data associated with one or more additional patients, a set of clinical rules, and treatment information related to a medical condition of the patient. The data access component can further access second data comprising one or more outcomes resulting from a clinical decision associated with the medical condition. The computer-executable components can further comprise a data generation component that identify, based on analysis of the first data and the second data, one or more cohorts to a medical journey of the patient. The data generation component can generate, based at least on the first data, the second data and the one or more cohorts to the medical journey of the patient, third data comprising at least two alternative treatment plans employable for subsequent treatment of the medical condition. The computer-executable components can further comprise a display component that can display, on a user interface (UI) of a device, a patient timeline comprising a temporal view of a medical history of the patient, specific to the medical condition. The display component can highlight, on the UI, the clinical decision and the one or more outcomes via a bounding box. The display component can further display, on the UI, visual connections between the clinical decision and the one or more outcomes to highlight a progression of the medical journey of the patient based on the clinical decision. The display component can further display, on the UI, a visualization of the one or more cohorts to the medical journey of the patient, wherein the visualization can be impactful in evaluating the at least two alternative treatment plans with reference to cohort outcomes, and wherein the visualization can be employable by a medical device to automatically execute, based on at least one cohort of the one or more cohorts to the medical journey of the patient, an action related to the subsequent treatment of the medical condition, if the at least one cohort satisfies a set of defined criteria.

According to another embodiment, a computer-implemented method is provided. The computer-implemented method can comprise accessing, by a system operatively coupled to a processor, first data comprising first patient data associated with a patient, second patient data associated with one or more additional patients, a set of clinical rules, and treatment information related to a medical condition of the patient. The computer-implemented method can further comprise accessing, by the system, second data comprising one or more outcomes resulting from a clinical decision associated with the medical condition. The computer-implemented method can further comprise analyzing, by the system, via generative AI, the first data and the second data. The computer-implemented method can further comprise identifying, by the system, based on analysis of the first data and the second data, via the generative AI, one or more cohorts to a medical journey of the patient. The computer-implemented method can further comprise generating, by the system, based at least on the first data, the second data and the one or more cohorts to the medical journey of the patient, via the generative AI, third data comprising at least two alternative treatment plans employable for subsequent treatment of the medical condition. The computer-implemented method can further comprise displaying, by the system, on a UI of a device, a patient timeline comprising a temporal view of a medical history of the patient, specific to the medical condition. The computer-implemented method can further comprise displaying, by the system, on the UI, a visualization of the one or more cohorts to the medical journey of the patient, where the visualization can be impactful in evaluating the at least two alternative treatment plans with reference to cohort outcomes.

According to yet another embodiment, a computer program product is provided. The computer program product can comprise a non-transitory computer readable memory having program instructions embodied therewith, the program instructions executable by a processor to cause the processor to access first data comprising first patient data associated with a patient, second patient data associated with one or more additional patients, a set of clinical rules, and treatment information related to a medical condition of the patient. The program instructions can be further executable by the processor to cause the processor to access second data comprising one or more outcomes resulting from a clinical decision associated with the medical condition. The program instructions can be further executable by the processor to cause the processor to analyze, via generative AI, the first data and the second data. The program instructions can be further executable by the processor to cause the processor to identify, based on analysis of the first data and the second data, via the generative AI, one or more cohorts to a medical journey of the patient. The program instructions can be further executable by the processor to cause the processor to generate, based at least on the first data, the second data and the one or more cohorts to the medical journey of the patient, via the generative AI, third data comprising at least two alternative treatment plans employable for subsequent treatment of the medical condition. The program instructions can be further executable by the processor to cause the processor to display, on a UI of a device, a patient timeline comprising a temporal view of a medical history of the patient, specific to the medical condition. The program instructions can be further executable by the processor to cause the processor to display, on the UI, a visualization of the one or more cohorts to the medical journey of the patient, where the visualization can be impactful in evaluating the at least two alternative treatment plans with reference to cohort outcomes.

### BRIEF DESCRIPTION OF THE DRAWINGS

One or more embodiments are described below in the Detailed Description section with reference to the following drawings:
FIG. 1A illustrates a diagram of an example, non-limiting system for patient history analysis and time-line segmentation using an electronic health record (EHR) display system, in accordance with one or more embodiments described herein.
FIG. 1B illustrates a diagram of an example, non-limiting method for timeline segmentation and display of the electronic health records (EHRs) of the patient over the display device, in accordance with one or more embodiments described herein.
FIG. 1C illustrates a diagram of an example, non-limiting method for timeline segmentation and display of the EHRs of the patient over the display device, in accordance with one or more embodiments described herein.
FIG. 1D schematically illustrates a diagram of an example, non-limiting process employable to leverage the system of FIG. 1A to improve data access and integration for a multidisciplinary team, in accordance with one or more embodiments described herein.
FIG. 1E illustrates a diagram of an example, non-limiting process for utilizing the system of FIG. 1A to generate longitudinal data elements for a patient for display to a user, in accordance with one or more embodiments described herein.
FIG. 2A illustrates a block diagram of an example, non-limiting system that can employ software and AI to present a decision maker specific visualization of the medical journey of a patient and generate alternative treatment plans employable for subsequent treatment of a disease, in accordance with one or more embodiments described herein.
FIG. 2B illustrates another block diagram of an example, non-limiting system that can present a visualization of the medical journey of a patient, including a decision maker specific visualization, and that can have the ability to generate alternative treatment plans employable for subsequent treatment of a disease, in accordance with one or more embodiments described herein.
FIG. 3 illustrates a diagram of an example, non-limiting UI displaying a temporal view of the medical journey of a patient, in accordance with one or more embodiments described herein.
FIG. 4 illustrates a diagram of an example, non-limiting UI displaying a temporal view of the medical journey of a patient, specific to a medical condition, in accordance with one or more embodiments described herein.
FIG. 5 illustrates diagrams of example, non-limiting patient timelines illustrated in FIGS. 3 and 4 to show a comparison between the two patient timelines, in accordance with one or more embodiments described herein.
FIG. 6 illustrates a diagram of an example, non-limiting patient timeline displaying a portion of the medical journey of a patient, in accordance with one or more embodiments described herein.
FIG. 7 illustrates a diagram of an example, non-limiting UI that can be employed to visualize medical studies related to the medical condition of a patient, in accordance with one or more embodiments described herein.
FIG. 8 illustrates a diagram of an example, non-limiting UI that can be employed to visualize treatment plans employed by medical practitioners specializing in the treatment of the medical condition of a patient, in accordance with one or more embodiments described herein.
FIG. 9 illustrates a diagram of an example, non-limiting UI that can be employed to mark aha moments in the medical journey of a patient, in accordance with one or more embodiments described herein.
FIG. 10 illustrates a diagram of an example, non-limiting patient timeline displaying an aha moment and a parallel view of medical journeys of multiple patients, in accordance with one or more embodiments described herein.
FIG. 11 illustrates a flow diagram of an example, non-limiting process that can generate an AI-based report to summarize the medical journey of a patient, in accordance with one or more embodiments described herein.
FIG. 12 illustrates a flow diagram of an example, non-limiting method that can detect decision points in the medical journey of a patient, in accordance with one or more embodiments described herein.
FIG. 13 illustrates a flow diagram of an example, non-limiting method that can detect events and outcomes resulting from a decision point in the medical journey of a patient, in accordance with one or more embodiments described herein.
FIG. 14 illustrates a flow diagram of an example, non-limiting method that can identify cohorts to the medical journey of a patient, in accordance with one or more embodiments described herein.
FIG. 15 illustrates flow diagrams of example, non-limiting methods that can generate a ranked list of medical practitioners specializing in the treatment of a medical condition and generate additional data corresponding to respective medical practitioners in the ranked list, in accordance with one or more embodiments described herein.
FIGS. 16 and 17 illustrate diagrams of example, non-limiting UIs that can display the medical journey of a patient, in accordance with one or more embodiments described herein.
FIG. 18 illustrates a diagram of an example, non-limiting UI that can display the decision maker specific view of the medical journey of a patient, in accordance with one or more embodiments described herein.
FIG. 19 illustrates a diagram of an example, non-limiting patient timeline that can display a cancer-specific view of the medical journey of a patient, in accordance with one or more embodiments described herein.
FIG. 20 illustrates diagrams of example, non-limiting cohort analysis visuals, in accordance with one or more embodiments described herein.
FIG. 21 illustrates diagrams of example, non-limiting patient timelines that can display aspects of the medical journey of a patient.
FIG. 22 illustrates a flow diagram of an example, non-limiting method that can present a decision maker specific visualization of the medical journey of a patient and generate alternative treatment plans employable for subsequent treatment of a disease, in accordance with one or more embodiments described herein.
FIG. 23 illustrates a flow diagram of an example, non-limiting method that can employ generative AI to present a decision maker specific visualization of the medical journey of a patient and generate alternative treatment plans employable for subsequent treatment of a disease, in accordance with one or more embodiments described herein.
FIG. 24 illustrates a block diagram of an example, non-limiting operating environment in which one or more embodiments described herein can be facilitated.
FIG. 25 illustrates an example networking environment operable to execute various implementations described herein.

### DETAILED DESCRIPTION

The following detailed description is merely illustrative and is not intended to limit embodiments and/or application or uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

In medical practice, physicians often experience dilemmas due to equivalencies of treatment options. For example, a physician can attempt multiple solutions for a patient, but each solution can yield a different outcome even when the solutions appear to be equivalent to each other. That is, treatment options can be equivalent in terms of eligibility, but they are often not equivalent in terms of outcomes. Additionally, medical practitioners often desire to learn about treatment choices employed by other medical practitioners. For example, a first physician may desire to know whether a second physician employs a specific drug, radiation therapy, etc. to treat patients, which ones of the treatment choices employed by the second physician yield the best outcome, and so on. For example, a first oncologist can benefit from knowing whether a second oncologist employs a popular drug for cancer treatment, or whether the second oncologist employs a treatment process that can be replicated by the first oncologist at a different institution. Likewise, a medical practitioner at a hospital may desire to know the outcomes of a treatment plan employed by another medical practitioner at the same medical facility. For example, a doctor can begin practicing at a different hospital and introduce a new drug in the formulary, or a new surgeon can join a hospital and introduce new solutions in their practice, and other medical practitioners may desire to know how the new drug or the new solutions can be employed by them.

In general, information evolves very rapidly, and new data is continuously produced in the field of medicine. In oncology, for example, new cancers are frequently discovered or new updates to cancer guidelines can become available. However, local physicians often do not have such information readily available to make informed clinical decisions for their patients. In fact, due to lack of information, physicians can choose the wrong treatments up to 1/3^{rd} of the time for their patients. As a result, patients often seek second opinions. Some existing solutions to the problems discussed herein include outcome tracking solutions for cancer that analyze clinical outcomes and explore future potential clinical trials to aide clinical decision making. However, such solutions typically do not involve learning from past data such as past clinical decisions, outcomes of past clinical decisions, etc. and accessing third party recommendations. Thus, solutions to assist medical practitioners in making better decisions by providing them access to latest information can be desirable.

Various embodiments of the present disclosure can be implemented to produce a solution to these problems. Embodiments described herein include systems, computer-implemented methods, and computer program products that can provide generative AI-based solutions and interactive UIs to evaluate treatment options for patients. For example, in one or more embodiments, technology is provided that can generate a time graph of a patient, that is, a patient timeline, wherein the patient timeline can display several decision maker specific events in the medical journey of the patient. This may be called a longitudinal patent timeline in some embodiments.

For example, the decision maker specific events can comprise an imaging scan event, the discovery of a suspicious mass, a biopsy to further investigate a suspicious mass, etc. The embodiments herein can also generate additional data such as, for example, a report of the biopsy event that can be digitally attached to the biopsy event on the patient timeline. The patient timeline and the additional data can be displayed by the system, via a display component, on a UI of a device (e.g., a desktop computer, a laptop, a tablet, a smartphone, etc.), within an existing software tool. Using the UI, a physician can traverse the patient timeline to study the medical journey of a patient over time. Specifically, embodiments herein allow a physician/clinician/healthcare worker to explore their past decision making to see how they can improve and learn from situations for the best healthcare of a patient and disease moving forward. The existing software tool can be a software tool that can generate a generic/default patient timeline that is not disease-specific and that cannot generate the type of additional data that the AI model can generate. Thus, the embodiments herein make the existing software tool more valuable for decision-makers as well as patients. Additionally, the data generated can be presented by the display component on the UI in a very visual manner, for example, via colored, patterned and/or otherwise emphasized shapes such as dotted rectangles, blinking points, etc. to distinguish data points, results and other types of information. The display component can also outline related sections of a view on the patient timeline from a first significant clinical decision to a second significant clinical decision in a patient's medical journey, show visual connections between points representing significant clinical decisions on the patient timeline, and present other visual cues to assist clinical decision making.

In one or more embodiments, the system can generate the patient timeline specific to a disease by accessing, via a data access component, information from a larger dataset and developing a more focused view. For example, the medical journey for a cancer patient can comprise events related to immunizations, oncological interventions, encounters with cardiology departments, regular check-ups, encounters with endocrinologists for diabetes monitoring, outpatient visits, etc., wherein only some of the events can be relevant to the cancer-specific medical journey of the patient. In one or more embodiments, the system can identify the oncological interventions or oncology-related events for the cancer patient within the larger dataset to generate the patient timeline, wherein the patient timeline can display the cancer-specific medical journey of the patient, and specific decisions of the decision maker.

In one or more embodiments, the embodiments herein can generate, via a data generation component, alternative treatment plans for the cancer patient based on the cancer-specific medical journey of the patient, as well as on other factors discussed herein. For example, the data generation component can identify the next best steps in the cancer treatment of the cancer patient. For example, the data generation component can evaluate five potential treatment choices in conjunction with the cancer patient's personal preferences and select the top two or top three treatment choices as the best treatment choices for the cancer patient. In one or more embodiments, the data generation component can present alternative treatment options in a non-intrusive manner. For example, during practice, physicians often interact with large amounts of dynamic information (e.g., information related to oncology, cardiology, internal medicine, etc. and other multidisciplinary situations) such as inputs, outputs, updates, and so on. Therefore, instead of suggesting treatment alternatives that intrude with the regular practice of a physician, the data generation component can present data-driven solutions that the physician can employ to determine the subsequent treatment for a patient. For example, the data generation component can identify potential next steps in a patient's treatment, clinicians that can provide a specialized service in a hospital, distributions for a specific type of drug or surgery, etc., wherein such data can assist the physician in determining whether a clinical decision can generate outcomes that fall within a group of desirable outcomes or deviate from the group of desirable outcomes, and wherein the group of desirable outcomes can correspond to a cohort of patients whose medical conditions follow predictable trajectories. If the outcomes resulting from a clinical decision begin to deviate from the group of desirable outcomes, the physician can conclude that the disease has become heterogenous or has changed form, and the physician can adjust the course of the treatment accordingly. For example, the physician can review treatments implemented by other clinicians to determine the best course of action.

In one or more embodiments, the embodiments herein can also generate, via the data generation component, second opinions (e.g., a second opinion view on the UI) within the existing software tool to assist the oncologist in identifying the best treatment solutions specific to the cancer patient. These second opinions can be applicable to current clinical decisions and to past clinical decisions associated with a treatment plan to help decision makers learn from their past clinical decisions.

The embodiments herein show a data density-dependent visualization that can allow human users to more clearly see and understand the patient journey, the medical decisions made, adherence to treatment plans (e.g., whether treatment plans are followed), etc. and to review the medical decisions versus the medical outcomes of the medical decisions. The embodiments herein also provide auto-scaling of space and time based on the patient state (e.g., pre-treatment, active treatment, survivorship). In one or more embodiments, filtering of the data as well as interaction with the data for decision making and potential recommendations can be performed via innovative algorithms and technologies.

In one or more embodiments, the data generation component can employ artificial intelligence, which may include neural networks, machine learning, generative AI, or other data science technologies to provide relevant and accurate data, scores, predictions, etc. that can assist the oncologist in producing a desirable outcome in a patient instead of allowing a disease to lead the treatment journey of a patient to an outcome that cannot be predicted exactly. In some situations, two patients can have the same type of cancer, but one of the patients can also be a diabetes patient and the other patient can be a heart patient. As a result, the outcomes of a cancer treatment can be different for both patients due to the unique attributes of each patient, the treatment attributes, etc. In such situations, the data generation component can tailor the treatment for each patient based on historical data (e.g., solutions employed for diabetic patients versus heart patients), obesity, age, ethnicity, frailty, and other factors specific to the respective patients. Additionally, the data generation component can take these factors into account when comparing past clinical decisions made for a patient to clinical decisions made for other patients with similar characteristics.

Physicians typically make clinical decisions based on data from clusters of patients rather than the experience of a single patient. For example, Neurofibromatosis type 1 (NF-1) is a complex and highly variable disease which makes it difficult to draw conclusions from a single patient's experience. In one or more embodiments, the system can identify, via the data generation component, cohorts to the medical journey of a patient to provide information to a physician about treatment plans implemented on other patients in the past, in a contextual manner. For example, based on the medical context of a patient, the data generation component can identify cohorts to the medical journey of a patient to ensure that the cohorts are relevant to the patient. For example, the data generation component can document outcomes metrics, experiences, toxicities, etc. for a patient, access guidelines applicable to the documented data, employ generative AI to extract the right contextual elements from the documented data, and identify a group of patients with medical journeys similar to that of the patient. The medical journeys of the patients in the group can assist a physician with decision making. For example, the cohorts can inform the physician about guidance or second opinions suggested by other physicians in the past, sources of the second opinions, guidelines on a previous cohort outcome (e.g., favorable results in 60% of the patients subjected to a treatment plan), improvements in outcome metric such as progression-free survival, and so on.

In one or more embodiments, a reporting component can generate a visual report by arranging the overall information available for the cancer patient, normalizing the information and shrinking the information, wherein the report can summarize the medical journey of the cancer patient, learnings from past data and other relevant data. The summary comprised in the report can save the cancer patient's oncologist valuable time by efficiently presenting information that the oncologist would otherwise need to learn by reading numerous reports. In one or more embodiments, such a report may be in a longitudinal patient timeline view.

In summary, the embodiments of the present disclosure can generate specific views of a patient timeline comprising events from the medical journey of a patient (including disease-specific and decision maker specific views), employ AI to present various analyses based on the disease-specific views, and provide graphical updates on the patient timeline to assist a decision maker in exploring alternative clinical paths to those executed in the past and to be executed in the future.

Additionally, the embodiments of the present disclosure can provide business and economic benefits by providing improved diagnoses, providing more accurate information for medical practitioners, and generating higher sales for organizations by improving usage and visual statistics for decision makers (e.g., physicians, surgeons, and/or other end entities). In one or more embodiments, the technologies described herein can provide decision makers with useful and interactive user interfaces that help them review past clinical decisions, learn from comparable data and overlays, and make the best clinical decisions possible moving forward. Such embodiments may also save patients time and money because the system can overlay second opinions and other helpful decision making information on the patient timeline, leading to the patients needing fewer second opinions on treatments. The technology described in various embodiments can be a powerful tool to improve clinical outcomes for patients and reduce expenses resulting from poor clinical decisions for both patients and medical facilities. It should be appreciated that although the examples discussed throughout this specification are focused on oncology, the embodiments of the present disclosure can be applied to various types of medical decision making and to other fields such as cardiology, neurology, pulmonology, orthopedics, etc.

For simplicity of explanation, the computer-implemented and non-computer-implemented methodologies provided herein are depicted and/or described as a series of acts. It is to be understood that the subject innovation is not limited by the acts illustrated and/or by the order of acts, for example acts can occur in one or more orders and/or concurrently, and with other acts not presented and described herein. Furthermore, not all illustrated acts can be utilized to implement the computer-implemented and non-computer-implemented methodologies in accordance with the described subject matter. Additionally, the computer-implemented methodologies described hereinafter and throughout this specification are capable of being stored on an article of manufacture to enable transporting and transferring the computer-implemented methodologies to computers. The term article of manufacture, as used herein, is intended to encompass a computer program accessible from any computer-readable device or storage media and run by computer processing units.

The systems and/or devices have been (and/or will be further) described herein with respect to interaction between one or more components. Such systems and/or components can include those components or sub-components specified therein, one or more of the specified components and/or sub-components, and/or additional components. Sub-components can be implemented as components communicatively coupled to other components rather than included within parent components. One or more components and/or sub-components can be combined into a single component providing aggregate functionality. The components can interact with one or more other components not specifically described herein for the sake of brevity, but known by those of skill in the art.

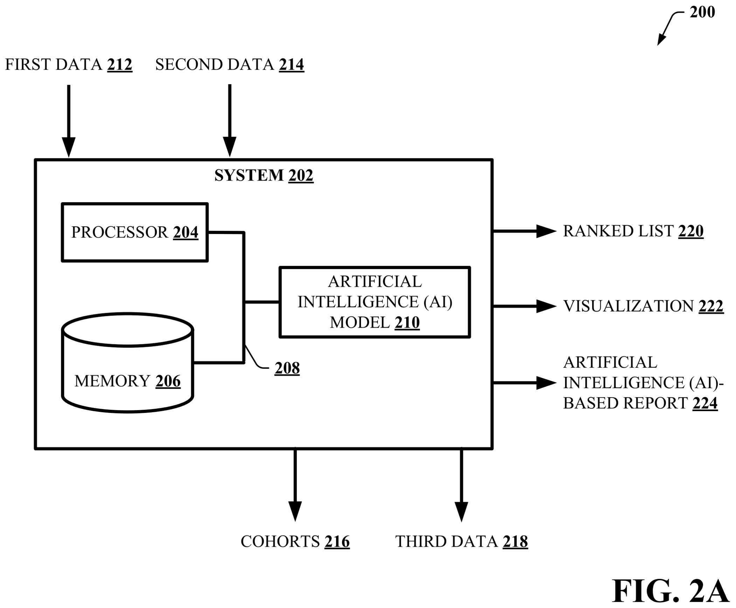

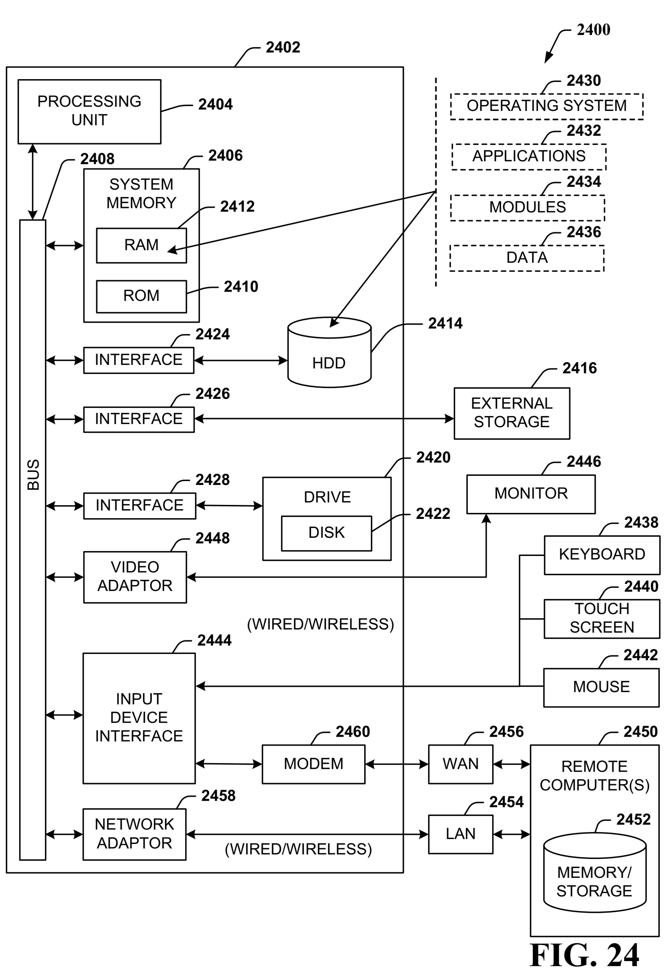

The embodiments depicted in one or more figures described herein are for illustration only, and as such, the architecture of embodiments is not limited to the systems, devices and/or components depicted therein, nor to any particular order, connection and/or coupling of systems, devices and/or components depicted therein. For example, in one or more embodiments, the non-limiting systems described herein, such as non-limiting system 200 as illustrated at FIG. 2A, and/or systems thereof, can further comprise, be associated with and/or be coupled to one or more computer and/or computing-based elements described herein with reference to an operating environment, such as the operating environment 2400 illustrated at FIG. 24. For example, non-limiting system 200 can be associated with, such as accessible via, a computing environment 2400 described below with reference to FIG. 24, such that aspects of processing can be distributed between non-limiting system 200 and the computing environment 2400. In one or more described embodiments, computer and/or computing-based elements can be used in connection with implementing one or more of the systems, devices, components and/or computer-implemented operations shown and/or described in connection with FIG. 2A and/or with other figures described herein.

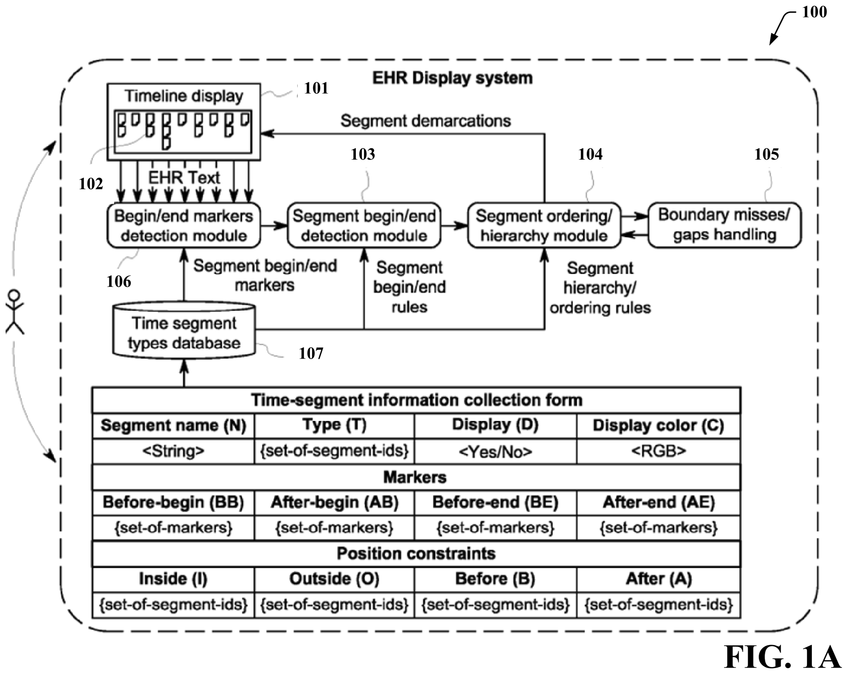

FIGS. 1A - 1E are intended to describe a technology background applicable to the one or more embodiments of the present disclosure. In this regard, at least FIGS. 1A - 1C describe systems and methods that can correspond to the existing software tool referenced in paragraph [0042] and to UI 300. Additionally, one or more systems and methods disclosed herein can interact with the systems and method described with reference to FIGS. 1A - 1E to enable the decision maker specific view described in various embodiments.

FIG. 1A illustrates a diagram of an example, non-limiting system 100 for patient history analysis and time-line segmentation using an EHR display system, in accordance with one or more embodiments described herein.

FIG. 1A shows a system (100) for patient history analysis and time-line segmentation using an EHR display system. The system (100) may include a display (101) configured to show the EHRs (102) of the patient according to an aspect of the disclosure. The EHRs of the patient may be stored in a memory of a computer system (107) or a server or any other electronic format known in the art or developed in the future. The computer system may be connected to a begin-end markers detection module (106). The begin-end markers detection module (106) may be configured to identify the beginning and end of a particular action or an event related to the patient and mark the beginning and end boundary of the action or the event. In one non-limiting example, the beginning event or the action may be admission of the patient to the hospital for diagnosis. Further, the patient may have been subject to magnetic resonance imaging (MRI) of the skull region. If no tumor was seen in the initial MR images, the patient may be discharged at a later point of time. This marks the end of imaging action and discharge of the patient. This scanning information of the patient in past may be time stamped and stored in the computer memory or any other suitable electronic storage medium. The begin-end markers detection module (106) may be configured to access the scanning information from the electronic storage medium. From the information stored on the electronic storage medium, the begin-end markers detection module (106) may be further configured to identify the scanning timeline including the date of admission of the patient, duration of the examination and display the date of discharge of the patient with a major conclusion that no tumor was initially detected. The begin-end markers detection module (106) may be configured to identify all such actions or events associated with the patient over the entire duration of the treatment. In one aspect of the disclosure, the begin-end markers detection module (106) may be configured to read the text information stored in the electronic storage medium and convert the information into a user viewable graph.

The system (100) further comprises a segment begin-end detection module (103). The segment begin-end detection module (103) may be configured to segment the various actions or events identified by the begin-end markers detection module (106). Segmenting the actions or the events identified by the begin-end markers detection module (106) by the segment begin-end detection module (103) comprises identifying the timeline boundaries of the actions or the events and arranging the segmented actions or the events according to the timeline of the action or the event. In one non-limiting example, the patient may have undergone a treatment for various unrelated medical conditions like brain tumor, chest infection, spinal dislocation and liver cirrhosis. According to an aspect of the disclosure, the segment begin-end detection module (103) may be configured to segment all the actions taken for these medical conditions and show the corresponding medication offered for it. The segment begin-end detection module (103) correlates the medical condition with the various other parameters including, for example, the timeline of the medical condition and medication offered against the medical condition. The segment begin-end detection module (103) further creates a visual graph showing the timelines of medications along with the overlaps in the treatment timelines. However, if the specific data related to end of the treatment timeline is not available, or there are breaks and restarts in the treatment, the boundaries of the graph for such treatment become uncertain and may get mixed with the other graphs. In system can further comprise a segment ordering/hierarchy module (104). The segment ordering/hierarchy module (104) may be configured to arrange the segments in order of the timeline. The gaps or the indefinite boundaries of the segments need to be identified for sequencing the segments in order of timeline.

A gap handling module (105) may be connected to the hierarchy module to identify and mitigate the gaps between two segments along the timeline wherever the timeline data is available. Further, an option may be provided to a clinician to manually fill the data and update the timelines based on the available medical records. This minimizes the timeline gaps and provides a more accurate picture to the user viewing the patient timeline. The demarcated segments may be sent to the display (101) by the segment ordering/hierarchy module (104) for showing the timeline sequence of the actions or the events related to the patient.

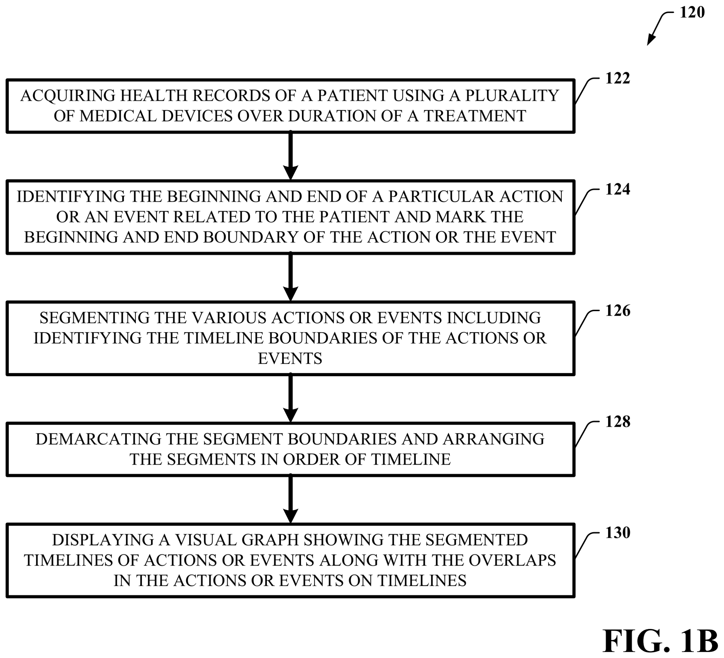

FIG. 1B illustrates a diagram of an example, non-limiting method 120 for timeline segmentation and display of the EHRs of the patient over the display device, in accordance with one or more embodiments described herein.

FIG. 1B shows a method (120) for patient history analysis and timeline segmentation of the EHRs and displaying segmented health records of the patient over the display device. The method (120) comprises acquiring (122) health records of a patient over a period of time and store the records for future records. The health records may be EHRs or physical health records that may be manually entered into the EHR systems. Although the reference is made to a single patient, it may be apparent to those skilled in the art that the health records of many such patients may be simultaneously acquired and stored. The EHRs of the patient may be stored in a memory of a computer system (not shown) or a server or any other electronic format known in the art or developed in the future. The method (120) further comprises identifying (124) the beginning and end of a particular action or an event related to the patient and marking the beginning and end boundary of the action or the event. Identifying (124) the beginning and end of a particular action or an event may be carried out using a begin-end markers detection module (106). Various treatments of a patient may have begun at various times and continued for a specific duration before the end of the treatment. The treatment course may include medication, visits to clinics, scanning, therapies, admission to the hospital, blood and other tests, discharge from the hospital and post discharge medication. The begin-end markers detection module (106) may be configured to access the scanning information from the electronic storage medium. From the information stored on the electronic storage medium, the begin-end markers detection module (106) may be further configured to identify the scanning timeline including the date of admission of the patient and the duration of the examination, and display the date of discharge of the patient with a major outcome or conclusion (e.g., no serious ailment was initially detected). The begin-end markers detection module (106) may be configured to identify all such actions or events associated with the patient over the entire duration of the treatment.

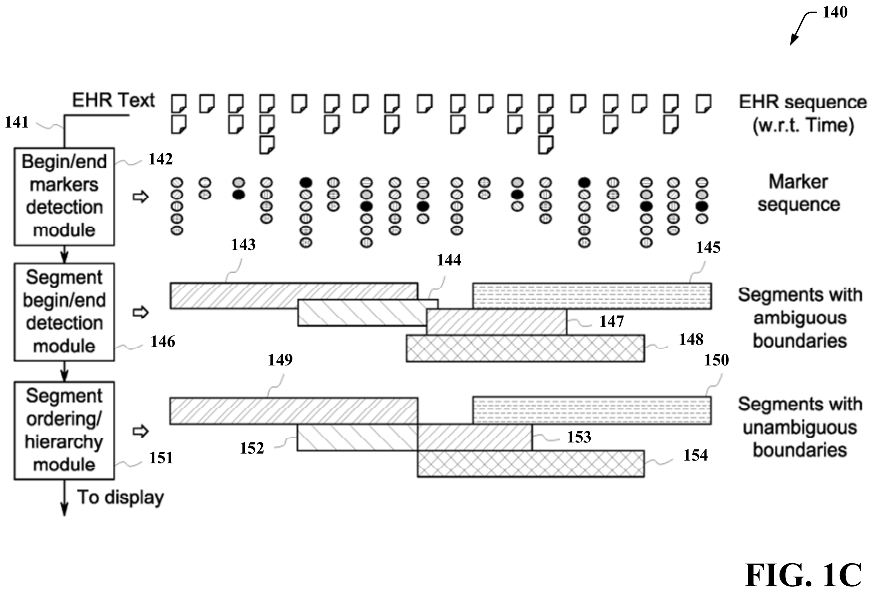

The method (120) further comprises segmenting (126) the various actions or events including identifying the timeline boundaries of the actions or the events. Segmenting (126) the various actions or the events may be carried out using a segment begin-end detection module (103). The segment begin-end detection module (103) may be configured to segment the various actions or the events identified by the begin-end markers detection module (106). Segmenting the actions or the events identified by the begin-end markers detection module (106) by the segment begin-end detection module (103) comprises identifying the timeline boundaries of the actions or events. In one non-limiting example, the patient may have undergone a treatment for various unrelated medical conditions like brain tumor, chest infection, spinal dislocation and liver cirrhosis. According to an aspect of the disclosure, segmenting (126) using the segment begin-end detection module (103) may be carried out to segment all the different actions taken for these medical conditions and show the corresponding medication offered for each medical condition. The segment begin-end detection module (103) correlates the medical condition with the various other parameters including, for example, the timeline of the medical condition and the medication offered against the medical condition. In instances where the end time of the action or the event is not properly identifiable, an ambiguous boundary may be generated. An ambiguous boundary is one where two segments overlap (shown in FIG. 1C) and demarcation of the segments is not possible. If the specific data related to end of the action or event timeline is not available, or there are breaks and restarts in the treatment, the boundaries of the graph for such treatment become uncertain and may get mixed with the other graphs.

The method (120) further comprises demarcating (128) the segment boundaries and arranging the segments in order of timeline. Demarcating (128) the segment boundaries comprises separating the segments from one another and creating identifiable boundaries. Arranging the segments in order of the timeline comprises identifying the sequence of the various actions or events related to a patient and arranging the sequence of actions or events along the timeline. In accordance with an aspect of the disclosure, a segment ordering/hierarchy module (104) may be provided. The segment ordering/hierarchy module (104) may be configured to arrange the segments in order of the timeline along with the clearly demarcated boundaries. Arranging the segments in sequential order or timeline creates the time sequence of the actions or events related to the patient. Arranging (128) the segments in order of the timeline may further comprise generating a visual graph showing the timelines of actions or events along with the overlaps in the action timelines.

The method (120) further comprises displaying (130) a visual graph showing the segmented timelines of actions or events containing the demarcated boundaries of the actions or events on timelines. The demarcated segments may be sent to the display device (101) by the segment ordering/hierarchy module (104) for showing the timeline sequence of the actions or the events related to the patient. The display (101) may be configured to show the timeline sequence of the actions or the events related to the patient along with the clinically important actions from every segment of the timeline. Displaying (130) may comprise showing the action or event segments in a variety of user-friendly display formats, for example, bars indicating beginning of the particular treatment and end of the treatment. When different treatments were offered simultaneously, the display will show two different segmented bars over a timeline with overlapping portions. The method (120) may be explained with an example of FIG. 1C.

FIG. 1C illustrates a diagram of an example, non-limiting method 140 for timeline segmentation and display of the EHRs of the patient over the display device, in accordance with one or more embodiments described herein.

In accordance with an aspect of the disclosure, FIG. 1C shows an example method (140) for timeline segmentation and display of the EHRs of the patient over the display device. The method (140) may include a display (not shown) configured to show the EHRs (141) of the patient according to an aspect of the disclosure. The EHRs (141) of the patient may be stored in a memory of a computer system (not shown) or a server or any other electronic format known in the art or developed in the future.

The computer system may be connected to a begin-end markers detection module (142). The begin-end markers detection module (142) may be configured to identify the beginning and end of a particular action or an event related to the patient and mark the beginning and end boundary of the action or the event. Various color schemes and shapes may be used to represent the type of disease, treatment offered and duration for a patient.

In one non-limiting example, the beginning event or the action may be admission of the patient to the hospital for diagnosis. A color-coded marker sequence may be generated corresponding to various EHRs by the begin-end markers detection module. Further, the patient may have been subject to magnetic resonance imaging (MRI) for the skull region. If no tumor was seen in the initial images, the patient may be discharged at a later point of time. This marks the end of imaging action and discharge of the patient. This scanning information of the patient in the past may be time stamped and stored in the computer memory or any other suitable electronic storage medium. The begin-end markers detection module (142) may be configured to access the scanning information from the electronic storage medium. From the information stored on the electronic storage medium, the begin-end markers detection module (142) may be further configured to identify the scanning timeline including the date of admission of the patient, duration of the examination and display the date of discharge of the patient with a major conclusion that no tumor was initially detected. The begin-end markers detection module (142) may be configured to identify all such actions or events associated with the patient over the entire duration of the treatment. In one aspect of the disclosure, the begin-end markers detection module (142) may be configured to read the text information stored in the electronic storage medium and convert the information into a user viewable graph.

The computer system further comprises a segment begin-end detection module (146). The segment begin-end detection module (146) may be configured to segment the various actions or events identified by the begin-end markers detection module (142). Segmenting the actions or events identified by the begin-end markers detection module (142) by the segment begin-end detection module (146) comprises identifying the timeline boundaries of the actions or events and arranging the segmented actions or events according to the timeline of the action or the event. In one non-limiting example, the patient may have undergone a treatment for various unrelated medical conditions like brain tumor, chest infection, spinal dislocation and liver cirrhosis. The segment begin-end detection module (146) may be configured to segment all the actions taken for these medical conditions and show the corresponding medication offered for it. The segment begin-end detection module (146) may generate segments (143, 145, 144, 147, 148) with ambiguous boundaries. Although only five segments (143, 145, 144, 147, 148) have been shown in the present example, it may be understood that there could be more or a smaller number of segments. The segment begin-end detection module (146) correlates the medical condition with the various other parameters including, for example, the timeline of the medical condition and medication offered against the medical condition. The segment begin-end detection module (146) further creates a visual graph showing the timelines of medications along with the overlaps in the treatment timelines. However, if the specific data related to the end of the treatment timeline is not available, or if there are breaks and restarts in the treatment, the boundaries of the graph for such treatment become uncertain and may get mixed with the other graphs. The system may also comprise a segment ordering/hierarchy module (151). The segment ordering/hierarchy module (151) may be configured to arrange the segments (149, 150, 152, 153, 154) in order of the timeline. The segment ordering/hierarchy module (151) may be connected to a gap handling module (105) to generate clearly demarcated boundaries. The demarcated segments (149, 150, 152, 153, 154) may be sent to the display (not shown) by the hierarchy module (151) for showing the timeline sequence of the actions or the events related to the patient. The segment begin-end detection module (146) may be further configured to highlight clinically important information related to each segment. This helps a clinician identify the relevant information from each segment within a short time duration as compared to manually finding out the relevant information from different treatments offered over time.

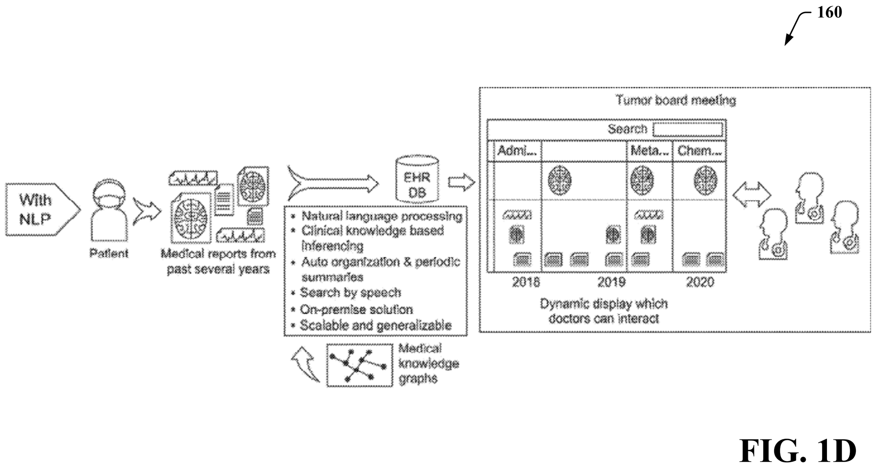

FIG. 1D schematically illustrates a diagram of an example, non-limiting 160 process employable to leverage the system of FIG. 1A (or another suitable system) to improve data access and integration for a multi-disciplinary team, in accordance with one or more embodiments described herein.

In the context of managing cancer treatment and as depicted visually by process 160 of FIG. 1D, a patient's medical reports from a segment in time (e.g., past several years) may be aggregated and analyzed using NLP. Clinical knowledge based inferencing may be applied. Auto-organization may be performed and periodic summaries may be generated.

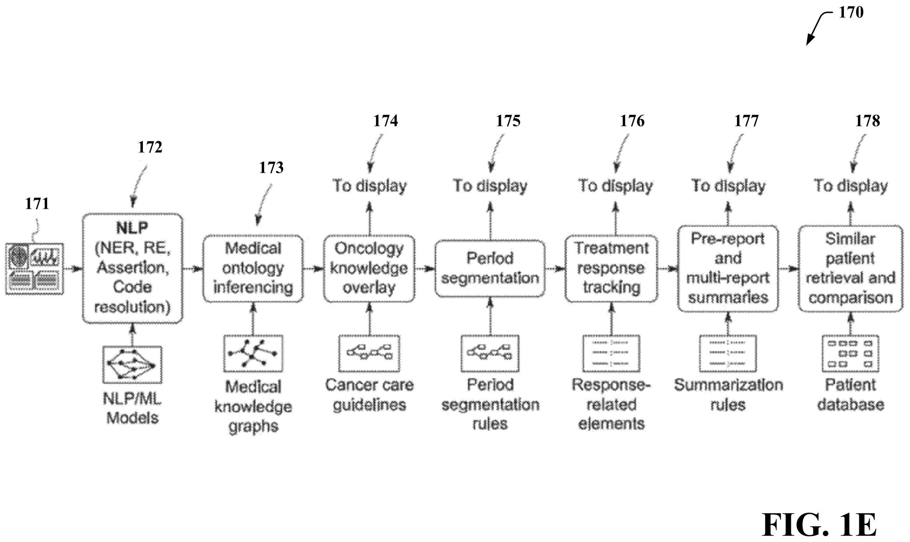

FIG. 1E illustrates a diagram of an example, non-limiting process 170 for utilizing the system of FIG. 1A (or another suitable system) to generate longitudinal data elements for a patient for display to a user, in accordance with one or more embodiments described herein.

FIG. 1E provides an example overview 170 of how system 100 or other systems described in the various figures may be utilized to generate timelines and associated display elements that may aid in delivering patient care. The overview 170 may represent a method for generating timelines that may be executed according to instructions stored in memory of a computing device, e.g., system 100 of FIG. 1A. As shown in FIG. 1E, the system 100 may ingest patient data 171, as described above with respect to FIG. 1A. The patient data may be in multiple different formats and obtained from different sources. The system 100 may utilize an NLP module to perform NLP on the patient data, as shown at 172. The NLP may include named entity recognition (NER), entity resolution, assertion, code resolution, and so on, as described above. Medical ontology inferencing may be performed on the processed data at 173, utilizing medical knowledge graphs. In this way, relevant clinical markers and information in the medical data may be identified and extracted, which may then be used for downstream display elements/overlays, as explained herein. For example, at 174, the processed patient data may be used to generate an oncology knowledge overlay based on cancer care guidelines, so that the guidelines relevant to the current patient status/condition may be displayed. At 175, period segmentation may be performed on the processed patient data according to period segmentation rules, and the period segmentation may be used to time order and segment the events of the patient data for display in the timeline. At 176, treatment response tracking may be performed on the patient data using response related elements, and any identified treatment responses may be displayed. Response related elements may include entities specific to a treatment response. For example, for cancer, response elements may include "stable response," "no response," etc., which may be determined following response evaluation criteria specific to a given condition or treatment, which allows clinicians to know if a treatment is working. At 177, per-report and multi-report summaries may be generated based on the relevant/extracted patient data (including treatment response) and summarization rules, and the summaries may be displayed on the timeline. At 178, the patient data may be used to identify similar patients from a patient database, and information about the similar patients may be retrieved and used to generate comparison summaries or other information that may be displayed.

FIG. 2A illustrates a block diagram of an example, non-limiting system 200 that can employ software and AI to present a decision maker specific visualization of the medical journey of a patient and generate alternative treatment plans employable for subsequent treatment of a disease, in accordance with one or more embodiments described herein.

Non-limiting system 200 and/or components of non-limiting system 200 can be employed to use hardware and/or software to solve problems that are highly technical in nature (e.g., related to medical information processing, generative AI, etc.), that are not abstract and that cannot be performed as a set of mental acts by a human. Further, some of the processes performed may be performed by specialized computers for carrying out defined tasks related to generative AI-based solutions to evaluate treatment options for patients. Non-limiting system 200 and/or components of non-limiting system 200 can be employed to solve new problems that arise through advancements in technologies mentioned above and/or the like. Non-limiting system 200 can provide technical improvements to medical information processing systems by improving the accuracy of and efficiency of processing analyzing clinical data and generating treatment plan alternatives based on the analyses. Non-limiting system 200 can provide additional improvements by reducing the financial costs to patients and medical facilities due to incorrect clinical decisions made by medical practitioners.

FIG. 2A further shows system 202, processor 204, memory 206, bus 208, AI model 210, first data 212, second data 214, cohorts 216, third data 218, ranked list 220, visualization 222, and AI-based report 224. System 202 can comprise processor 204 (e.g., computer processing unit, microprocessor, classical processor, and/or like processor). In one or more embodiments, a component associated with system 202, as described herein with or without reference to the one or more figures of the one or more embodiments, can comprise one or more computer and/or machine readable, writable and/or executable components and/or instructions that can be executed by processor 204 to enable performance of one or more processes defined by such component(s) and/or instruction(s).

In one or more embodiments, system 202 can comprise a computer-readable memory (e.g., memory 206) that can be operably connected to processor 204. Memory 206 can store computer-executable instructions that, upon execution by processor 204, can cause processor 204 and/or one or more other components of system 202 (e.g., artificial intelligence (AI) model 210, data access component 232, data generation component 234, display component 236, first artificial intelligence (AI) component 238, second artificial intelligence (AI) component 240, plan seeker component 242, detection component 244, evaluation component 246, merger component 248, selection component 250, prompt generation component 252, reporting component 254 and training component 256) to perform one or more actions. In one or more embodiments, memory 206 can store computer-executable components (e.g., AI model 210, data access component 232, data generation component 234, display component 236, first AI component 238, second AI component 240, plan seeker component 242, detection component 244, evaluation component 246, merger component 248, selection component 250, prompt generation component 252, reporting component 254 and training component 256). In one or more embodiments, one or more of the components of system 202 can reside in the cloud, and/or can reside locally in a local computing environment (e.g., at a specified location(s)).

Non-limiting system 200 and/or a component thereof as described herein, can be communicatively, electrically, operatively, optically and/or otherwise coupled to one another via bus 208. Bus 208 can comprise one or more of a memory bus, memory controller, peripheral bus, external bus, local bus, and/or another type of bus that can employ one or more bus architectures. One or more of these examples of bus 208 can be employed. In one or more embodiments, non-limiting system 200 can be coupled (e.g., communicatively, electrically, operatively, optically and/or via like function) to one or more external systems (e.g., a nonillustrated electrical output production system, one or more output targets, an output target controller and/or the like), sources and/or devices (e.g., classical computing devices, communication devices and/or like devices), such as via a network.

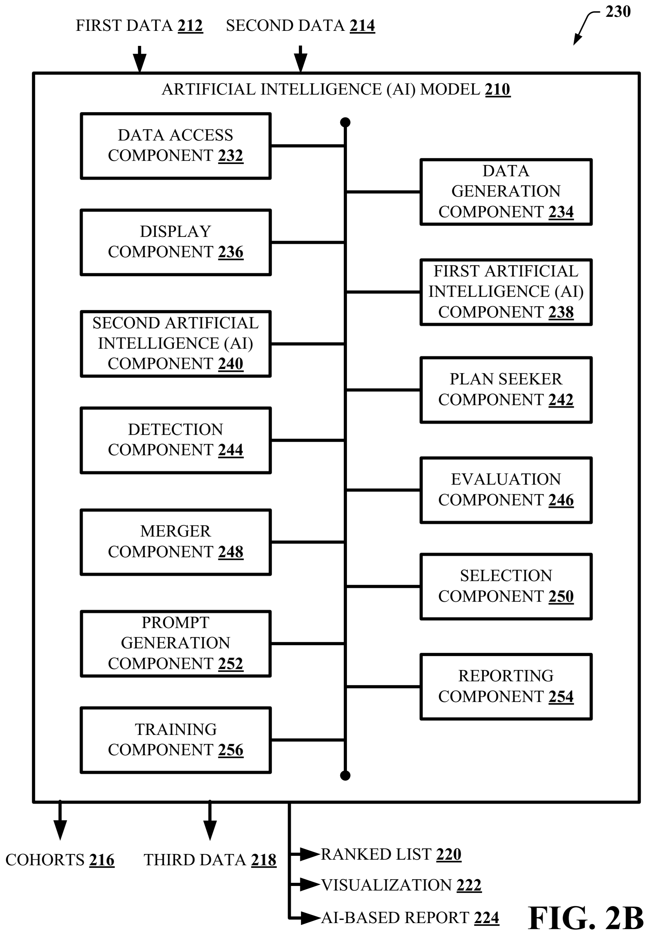

In one or more embodiments, system 202 can comprise AI model 210. In one or more embodiments, AI model 210 can comprise data access component 232, data generation component 234, display component 236, first AI component 238, second AI component 240, plan seeker component 242, detection component 244, evaluation component 246, merger component 248, selection component 250, prompt generation component 252, reporting component 254 and training component 256, as illustrated in FIG. 2B. In one or more embodiments, system 202 can also comprise, in memory 206, one or more software programs that can execute a set of algorithms or method steps to generate and output technical and medical outputs to a UI.

In one or more embodiments, AI model 210 can employ software steps and AI algorithms to present, at a UI, an interactive visualization of the medical journey of a patient and generate alternative treatment plans employable by end entities (e.g., hardware, software, machines, AI, neural networks and/or users) such as medical practitioners (e.g., doctors, surgeons, clinicians, etc.) to treat a medical condition (e.g., a disease) of a patient. The interactive visualization can be time-based, and a medical practitioner can review past decisions (e.g., go back in time) via the interactive visualization, with further assistance from the decision maker views discussed herein.

In one or more embodiments, data access component 232 can access first data 212 and second data 214, wherein first data 212 and second data 214 can be stored in a memory (e.g., memory 206) accessible to AI model 210. First data 212 can comprise first patient data associated with a patient, second patient data associated with one or more additional patients, a set of clinical rules, and treatment information related to a medical condition of the patient. Second data 214 can comprise one or more outcomes resulting from a clinical decision associated with the medical condition. First data 212 and second data 214 can be input to system 202 by an end entity (e.g., hardware, software, machine, AI, neural network and/or user) via the UI of a device (e.g., a computer, a laptop, a tablet, a smartphone, etc.). In one or more embodiments, the UI can comprise a command-line interface (CLI), a graphical user interface (GUI), a voice user interface (VUI) and/or a touch-base interface.

In one or more embodiments, data generation component 234 can analyze first data 212 and second data 214 via AI, a software rules engine, or specifically coded algorithm steps in a medical software program (e.g., human or machine-written source code). Based on analysis of first data 212 and second data 214, data generation component 234 can identify one or more cohorts to the medical journey of the patient (i.e., cohorts 216). Thereafter, based at least on first data 212, second data 214 and cohorts 216, data generation component 234 can generate, via the generative AI, third data 218 comprising at least two alternative treatment plans employable for subsequent treatment of the medical condition.

In one or more embodiments, display component 236 (e.g., a display module) can display on the UI of the device (e.g., a computer, a laptop, a tablet, a smartphone, etc.), a patient timeline comprising a temporal view of the medical story of a patient journey. This view can have disease specific views as well as decision maker specific views. This view may be a longitudinal patient timeline. Display component 236 can also extend the temporal view to a future date to display clinical decisions employable for subsequent treatment of the patient. Further, display component 236 can display on the UI, a visualization (i.e., visualization 222) of the one or more cohorts to the medical journey of the patient (i.e., cohorts 216). Visualization 222 can be impactful in evaluating the at least two alternative treatment plans with reference to cohort outcomes. Additionally, visualization 222 can be employable by a medical device to automatically execute, based on at least one cohort of the one or more cohorts to the medical journey of the patient, an action related to the subsequent treatment of the medical condition, if the at least one cohort satisfies a set of defined criteria. For example, in one or more embodiments, visualization 222 can be accessed by a medical device (e.g., a medical imaging device, or another type of medical device), and the medical device can be programmed by an end entity (e.g., hardware, software, machine, AI, neural network and/or user) to compare data from visualization 222 with a set of defined criteria (e.g., 90% predicted success rate for cohorts, lowest cost option, fastest response time, etc.) and to automatically execute one or more actions if at least one cohort comprised within cohorts 216 matches the set of defined criteria. For example, the medical device can be programmed to execute instructions to obtain a medical image, obtain test results and so on, if one or more of cohorts 216 match the set of defined criteria. As a result, the treatment for the patient can continue seamlessly and some of the human intervention can be automated and reduced. In one or more embodiments, display component 236 can function as an interactive module that can enable an end entity (e.g., hardware, software, machine, AI, neural network and/or user) to select options, decision points, drop-down menus, etc. on the UI via a cursor, touchscreen, voice commands, or other suitable functionality.

In one or more embodiments, first AI component 238 can detect, based on the first patient data and the treatment information, a plurality of decision points within the temporal view, wherein respective decision points can represent respective clinical decisions corresponding to the patient, and wherein the clinical decision can correspond to a decision point within the temporal view. In some embodiments, first AI component 238 can detect the plurality of decision points when a medical practitioner enters (e.g., via the UI) a treatment plan into non-limiting system 200 or prescribes a certain treatment that is entered into non-limiting system 200. The treatment plan can comprise, for example, a decision made by the medical practitioner related to specific treatment actions for a patient, wherein the treatment actions can comprise medical exams, medication, surgeries, and other types of treatment actions within the medical profession.

First AI component 238 can employ an AI agentic system (or a key decision moment detection agentic system) comprising various agents (e.g., plan seeker component 242, detection component 244, evaluation component 246 and merger component 248), wherein different agents can employ different approaches to detect the plurality of decision points. For example, plan seeker component 242 can identify a first set of decision points based on modifications within a treatment plan associated with the medical condition of the patient, detection component 244 can identify a second set of decision points based on inputs associated with treatment decisions comprised in the treatment plan, and evaluation component 246 can identify a third set of decision points based on the set of clinical rules, wherein the set of clinical rules can comprise rules configured to detect defined clinical patterns. Finally, merger component 248 can generate a list comprising the plurality of decision points by merging the first set of decision points, the second set of decision points and the third set of decision points. In this regard, respective decision points comprised in the list can represent key decision moments or key clinical decisions made by a physician to treat the medical condition of the patient, and detecting the respective decision points can be important for the decision maker view of the patient timeline described in one or more embodiments.

In one or more embodiments, first AI component 238 can further detect, based on the respective clinical decisions, one or more decision points of the plurality of decision points as respective key moments (also known as aha moments) indicative of non-standard insights identified by first AI component 238.

In one or more embodiments, second AI component 240 can detect the one or more outcomes comprised in second data 214 by employing selection component 250, prompt generation component 252 and merger component 248. For example, selection component 250 can select the decision point associated with the clinical decision corresponding to the one or more outcomes, prompt generation component 252 can generate a set of summarization prompts to identify a set of events representing consequences of the clinical decision, prompt generation component 252 can input the set of summarization prompts to a large language model (LLM), and merger component 248 can analyze results generated by the LLM upon processing the set of summarization prompts to detect the one or more outcomes associated with the decision point. Second AI component 240 can detect respective sets of outcomes for respective decision points in a similar manner. In one or more embodiments, display component 236 can highlight the decision point and the one or more outcomes via a bounding box, on the patient timeline, in response to the selecting. In one or more embodiments, first AI component 238, second AI component 240, and display component 236 can comprise a decision marker module that can identify and present, on the patient timeline, highlighted points (e.g., data points) representing significant clinical decisions or medical treatment plan decisions related to the medical condition of the patient.

Recall that data generation component 234 can identify, via the AI, one or more cohorts to the medical journey of the patient (i.e., cohorts 216). To identify cohorts 216, data generation component 234 can summarize the medical journey of the patient prior to the decision point, compare the medical journey of the patient to respective medical journeys of respective patients of the one or more additional patients, and select, based on relevant clinical criteria, a set of medical journeys from the respective medical journeys as the one or more cohorts to the medical journey of the patient (i.e., cohorts 216). In this regard, displaying visualization 222 can comprise outputting, by display component 236, on the UI of the device (e.g., a computer, a laptop, a tablet, a smartphone, etc.), first outcomes and outcome metrics for different choices of first treatment plans associated with respective cohorts of cohorts 216. In one or more embodiments, display component 236 can also display a cohort filter that can be employed by a physician to specify/enter additional details that data generation component 234 can consider when identifying cohorts 216.

In one or more embodiments, data generation component 234 can further generate, based on the first patient data and the treatment information, a ranked list of medical practitioners (i.e., ranked list 220), wherein ranked list 220 can be employable by an end entity (e.g., hardware, software, machine, AI, neural network and/or user) to explore second treatment plans employed by the medical practitioners to treat the medical condition, explore second outcomes associated with the second treatment plans, and contact the medical practitioners.

In one or more embodiments, reporting component 254 can generate AI-based report 224 comprising an explanation of information learned by data generation component 234 based on outcomes associated with the respective clinical decisions and recommendations for the subsequent treatment of the medical condition. In one or more embodiments, display component 236 can highlight the outcomes via visual indicators and scores displayed on the patient timeline on the UI.

In one or more embodiments, training component 256 can train data generation component 234 to iteratively analyze first data 212 and second data 214 for new information and to identify, based on the new information, new cohorts that are most relevant to the medical journey of the patient at a given point in time.

The functionalities of the components comprised in AI model 210 are described in greater detail with reference to at least the following example given by paragraphs [0094] - [00117]. It should be appreciated that, in addition to the data analysis and AI-based embodiments described in the present disclosure, the decision maker view described in various embodiments of the present disclosure can assist decision makers (e.g., physicians, surgeons, nurses, etc.) with making clinical decisions to treat patients as well as provide opportunities to the decision makers to learn from past clinical decisions (e.g., via comparison of treatment plans of different luminaries, medical journeys of patients with similar treatment journeys, aha moments, etc.). In this regard, the following example can involve aspects described with reference to FIGS. 1A - 1E.

### EXAMPLE:

In an embodiment, patient *A* can be a cancer patient being treated by oncologist *A.* First patient data comprised in first data 212 can comprise information such as the age, the gender, the ethnicity, the medical history, and other relevant details of patient A. Second patient data comprised in first data 212 can comprise the age, the gender, the ethnicity, the medical history, and other relevant details of one or more additional patients, for example, patients at the same medical facility (e.g., hospital, cancer institute, etc.) responsible for treating patient A. The treatment information comprised in first data 212 can comprise details from a treatment plan developed by oncologist *A* for patient *A.* The set of clinical rules comprised in first data 212 can comprise rules configured by the medical facility to detect defined clinical patterns in patients.

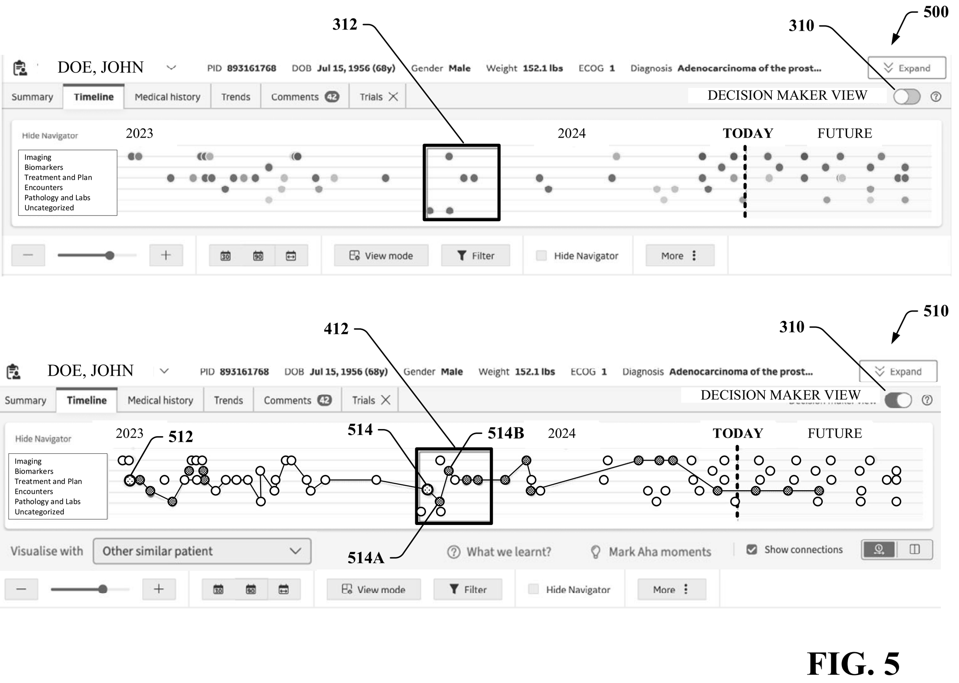

In an embodiment, data access component 232 can access the first patient data and the treatment information related to patient A, based on which, display component 236 can display an initial patient timeline (e.g., non-limiting patient timeline 500 of FIG. 5) comprising a temporal view of the medical history of patient A and the treatment plan developed by oncologist A. The initial patient timeline can extend from a specific point in time in the past (e.g., a specific date) to a future point in time (e.g., a specific date). The initial patient timeline can be displayed on the UI of a device (e.g., a computer, a laptop, a tablet, a smartphone, etc.) accessible oncologist A. Additionally, the initial patient timeline can be displayed with an interactive toggle switch such that an end entity (e.g., hardware, software, machine, AI, neural network and/or user) can turn the toggle switch on to generate a disease-specific view. For example, oncologist A can turn the toggle switch on by selecting the toggle switch via a cursor, touchscreen or other suitable functionality. As a result, display component 236 can display a patient timeline (e.g., non-limiting patient timeline 510 of FIG. 5) with a cancer-specific view showing only the cancer-specific medical journey corresponding to patient *A*. For example, patient *A* can also be a diabetic patient, and the initial patient timeline can display events such as laboratory tests, biomarker data, etc. related to a diabetes treatment as well as the cancer-treatment of patient *A*, whereas the patient timeline with the cancer-specific view can display events such as laboratory tests, imaging data, biomarker data, etc. related to only the cancer-treatment of patient *A*. Further, each event on the initial patient timeline and on the patient timeline comprising the cancer-specific view can be displayed as a point (e.g., a data point), and different events can be highlighted by different colors and/or visual indicators. For example, in the initial patient timeline, events in identical categories can be displayed with the same color and events in unidentical categories can be displayed with different colors.

In one or more embodiments, first AI component 238 can employ plan seeker component 242, detection component 244, evaluation component 246 and merger component 248 to detect a plurality of decision points on the patient timeline. Recall that respective decision points on the patient timeline can represent respective key decision moments. To detect a decision point (i.e., a key decision moment), plan seeker component 242 (e.g., agent 1) can access and read medical notes for patient A stored in a memory (e.g., memory 206) and identify when oncologist *A* has planned treatment for patient *A.* Each plan determination or modification comprised in the treatment plan developed by oncologist *A* for patient *A* can represent a key decision moment. Further, detection component 244 (e.g., agent 2) can apply a trained model that can identify key decision moments. The trained model can be trained (e.g., by training component 256) on data annotated by physicians to identify events that represent key decision moments according to the physicians. For example, in an embodiment, each line of treatment and dose or adjustment based on tolerance or comorbidities can be a clinical decision made by a physician, and key decision moments can be the inputs employed to make the clinical decision. In one or more embodiments, detection component 244 can be a decision moment detection model based on deep learning (DL). Evaluation component 246 (e.g., agent 3) can be a rulebased model that can monitor the patient timeline according to the set of clinical rules, wherein the set of clinical rules can comprise a set of hospital/physician configured rules that can detect well-defined patterns. For example, evaluation component 246 can be configured to alert oncologist *A* if patient *A* experiences a weight reduction greater than 5 percent (5 %). Finally, merger component 248 can access/gather the key decision moments detected by the agents (i.e., plan seeker component 242, detection component 244 and evaluation component 246), merge the key decision moments and return a list comprising the decision points.

In one or more embodiments, first AI component 238 can further detect one or more decision points of the plurality of decision points as aha moments. Aha moments represent key moments when a non-standard insight is identified by an end entity (e.g., hardware, software, machine, AI, neural network and/or user). For example, an aha moment can be a moment in time when a physician suddenly understands or recognizes a piece of information, has a good idea or finds an answer to a problem. In an embodiment, first AI component 238 can detect a decision point and determine that the decision point represents an aha moment. In another embodiment, oncologist *A* or another care giver for patient *A* can determine, upon viewing the patient timeline, that a decision point represents an aha moment, and oncologist *A* or the other care giver can select a button/an option on the UI via a cursor, touchscreen or other suitable functionality to indicate to first AI component 238 that the decision point represents an aha moment. Software that can identify an aha moment can typically comprise user behavior analytics tools such as Mixpanel^{®}, Fullstory^{®}, Highlight, Pendo^{®}, ClickUp^{®}, and UserGuiding^{®} that can allow an end entity (e.g., hardware, software, machine, AI, neural network and/or user) to track user interactions within a product to pinpoint the specific actions or features that trigger a sudden understanding or realization of value, wherein the specific actions or the features can be considered the aha moment for the end entity. The key features of such software tools that can identify aha moments include:
User session recording: Watching video replays of user sessions to see exactly how users navigate and interact with a company's product.
Event tracking: Monitoring specific user actions like button clicks, form submissions, or feature usage to identify patterns related to aha moments.
Heatmaps: Visualizing where users click or hover on an interface to understand areas of interest and potential aha moment triggers.
Funnels and cohorts: Analyzing user flows to identify drop-off points and where users might be experiencing an aha moment.
In-app surveys and feedback mechanisms: Collecting direct feedback from users about their experience to understand when and how they perceived value.

Some additional techniques to detect aha moments can include:
Setting up tracking for key actions: Identifying potential aha moments in a product and tracking the user interactions associated with them.
Analyzing user behavior patterns: Looking for patterns in user data that can indicate a significant shift in engagement or understanding, suggesting an aha moment.
Segment user groups: Analyzing data based on different user demographics or behaviors to understand if aha moments vary across different user types.
Iterating and refining user data: Using insights from user data to optimize a product onboarding and user experience to guide users more effectively towards their aha moment.

In one or more embodiments, first AI component 238 can employ one or more of such features to detect the one or more decision points as aha moments, with can be marked on a navigator on the patient timeline.

In one or more embodiments, data access component 232 can further access second data 214 comprising one or more outcomes resulting from a clinical decision, wherein the clinical decision can correspond to a decision point detected by first AI component 238 within the temporal view of the patient timeline. For example, the decision point can be a decision made by oncologist *A* in the past to administer a drug to patient *A,* and the one or more outcomes can represent the consequences of the drug on patient *A*. In one or more embodiments, second AI component 240 can detect the one or more outcomes comprised in second data 214 by employing selection component 250, prompt generation component 252 and merger component 248. For example, selection component 250 can select the decision point. Based on the decision point, prompt generation component 252 can develop summarization prompts describing a list or a summary of events that stem from the decision point. For example, prompt generation component 252 can develop a first prompt for summarization based on the decision point, a second prompt to select events that are consequences of the decision point (i.e., the corresponding clinical decision), a third prompt to summarize potential consequences of a different/alternative clinical decision, a fourth prompt to select an event or a data point (e.g., surgery, medicine, diet, etc.) and summarize the causes and/or clinical decisions responsible for the event, a fifth prompt for response tracking to help create a key decision moment, and additional prompts comprising additional questions.

Response tracking refers to the process of monitoring, storing and analyzing responses generated by a system such as an LLM, an AI model, etc. For example, detection of toxicities by an LLM can be a negative response, detection of efficacy by an LLM can be a positive response, and so on, and input parameters to a response can comprise clinical, biochemical and radiological response parameters. Such details can be combined by prompt generation component 252 to generate the fifth prompt. Additionally, prompt generation component 252 can employ Retrieval Augmented Generation (RAG) to develop the second prompt and the additional prompts. RAG combines a retrieval mechanism with a generative model such that the generative model can employ relevant information from an external knowledge base to generate a response.

In one or more embodiments, data generation component 234 can analyze, via generative AI, first data 212 and second data 214. Based on analysis of first data 212 and second data 214, data generation component 234 can identify, via generative AI, cohorts 216, wherein cohorts 216 can comprise one or more cohorts to the medical journey of patient *A*. For example, data generation component 234 can comprise algorithms that can identify patients having identical medical conditions to that of patient *A* within the same institution (e.g., hospital, cancer institute, etc.) responsible for treating patient *A*. In an embodiment, data generation component 234 can employ existing cohort algorithms and/or systems. In another embodiment, data generation component 234 can employ an algorithm based on decision making (i.e., a decision-making algorithm). For example, an end entity (e.g., hardware, software, machine, AI, neural network and/or user) can select a decision point on the UI via a cursor, touchscreen or other suitable functionality. For example, oncologist *A* can select a decision point for patient *A.*

Based on the selection, the decision-making algorithm can summarize the medical journey of patient *A* up to the decision point, and for each patient in the institution, the decision-making algorithm can detect (e.g., from the second patient data comprised in first data 212), a decision moment (if such a decision moment exists) that is the most similar to the decision point selected for patient *A* (e.g., a most-similar decision moment). Upon detecting a most-similar decision moment for a patient (e.g., patient *B*), the decision-making algorithm can summarize the medical journey of patient *B* up to the most-similar decision moment. The decision-making algorithm can repeat this process for patients (e.g., patient *C*, patient *D*, etc.) for whom a most-similar decision moment can be detected. The decision-making algorithm can compare the medical journey of patient *A* with the respective medical journeys of patients *B, C, D,* etc. and select the top *N* matches as cohorts 216, wherein *N* represents a positive integer.

In one or more embodiments, display component 236 can display (e.g., output, present) the outcomes for the respective cohorts comprised in cohorts 216 and the outcome metrics for the respective choices of treatments (e.g., Overall Survival (OS), Disease-Free Survival (DFS)/Progression-Free Survival (PFS), radiographic Progression-Free Survival (rPFS), prostate-specific antigen Progression-Free Survival (pPFS), etc.) as visualization 222 (e.g., a parallel view of other patients) on the UI. In an embodiment, display component 236 can display visualization 222 in response to a selection made by oncologist *A* on the UI to visualize the medical journey of patient *A* alongside cohorts 216. In one or more embodiments, an end entity (e.g., hardware, software, machine, AI, neural network and/or user) can select the most suitable outcomes metrics based on visualization 222. For example, oncologist *A* can select the best outcome metrics based on OS, DFS, PFS, etc. to determine the subsequent treatment for patient *A*. Additionally, in one or more embodiments, data generation component 234 can generate, based on first data 212, second data 214 and cohorts 216, at least two alternative treatment plans for patient *A*, and visualization 222 can be impactful in evaluating the alternative treatment plans with reference to the cohort outcomes. That is, in addition to employing visualization 222 to determine the subsequent treatment for patient *A,* oncologist *A* can employ visualization 222 to compare the alternative treatment plans with cohorts 216 and the corresponding cohort outcomes.

In one or more embodiments, data generation component 234 can further generate, based on the first patient data and the treatment information, ranked list 220 comprising a ranked list of medical practitioners (e.g., luminaries) specializing in treatment of a disease, wherein the medical practitioners can be ranked according to various criteria such as recent citations, positive results, guidelines, age, ethnicity, etc. For example, ranked list 220 can comprise a ranked list of oncologists (e.g., Dr. John Doe at Hospital *M* in Ohio, Dr. Jane Doe at Hospital *N* in China, etc.) specializing in the treatment of cancer. In an embodiment, data generation component 234 can generate ranked list 220 in response to a selection made by oncologist *A* on the UI to generate ranked list 220. In one or more embodiments, display component 236 can display ranked list 220 on the UI, such that an end entity (e.g., hardware, software, machine, AI, neural network and/or user) can select the name of a medical practitioner comprised in ranked list 220 to generate a parallel view of medical journeys of patients treated by the medical practitioner. For example, oncologist *A* can select the names of one or more medical practitioners comprised in ranked list 220 on the UI via a cursor, touchscreen or other suitable functionality, and display component 236 can display a parallel/overlapping view of medical journeys of patients associated with the medical practitioners, the decisions made by the medical practitioners during treatment of their patients, and the corresponding outcomes. Thus, oncologist A can view the decisions that other medical practitioners may have made for patients similar to patient *A* and/or for situations similar to those experienced by patient *A.* Display component 236 can also display a "contact luminary" button, a "video call luminary" button and/or a "send luminary my patient journey" button that can be employed by oncologist *A* to extract further assistance from one or more medical practitioners at a given time for the treatment of patient *A*.

In one or more embodiments, reporting component 254 can generate AI-based report 224 comprising an explanation of information learned by data generation component 234. For example, reporting component 254 can employ a generative AI model that can provide LLM-based outputs comprising the learnings of data generation component 234 during a previous leg (e.g., the last leg) of the medical journey of patient *A* from a decision point to a corresponding outcome, information about patient *A*, industry trends, electronic medical records (EMR) of patient *A,* guidelines, data available with oncologist *A,* explanations for one or more outcomes related to a decision point, etc. Accordingly, AI-based report 224 can describe, for example, whether patient *A* followed a treatment plan developed by oncologist *A,* whether a clinical decision made by oncologist *A* was a good decision, a bad decision or neither a good nor a bad decision based on effectiveness (e.g., 80% effective, 90% effective, etc.). AI-based report 224 can additionally comprise recommendations for data generation component 234 to improve future decisions based on past decisions, clickable hyperlinks that can allow oncologist *A* to directly view a specific visual point on the patient timeline related to a particular discussion or summarization in AI-based report 224, and so on. In one or more embodiments, reporting component 254 can also employ algorithms to detect adherence of patient *A* to a treatment plan (e.g., to determine how much patient *A*'s actions/adherence impacted the outcome of the treatment plan), to determine how patient *A* fares as compared to average patients in the same category and why, and to identify similarities in known patient parameters (e.g., via a slider displayed on the UI to see that the patient parameters are 80% similar, 90% similar, etc. to other patients). Such additional information can also be included in AI-based report 224 to provide multiple layers of information to oncologist *A,* which can direct oncologist *A* towards higher-order decision making or higher-order knowledge.

In general, AI-based report 224 can assist an end entity (e.g., hardware, software, machine, AI, neural network and/or user) in learning from good decisions and employing the learnings to make clinical decisions in the future. In an embodiment, reporting component 254 can generate AI-based report 224 in response to a selection made by oncologist *A* on the UI to generate AI-based report 224. In one or more embodiments, display component 236 can display AI-based report 224 on the UI.

In one or more embodiments, data generation component 234 can also employ the generative AI to perform continuous/high-frequency monitoring of first data 212 and second data 214 to ensure that the treatment plans recommended by data generation component 234 can remain within desirable medical limits. Contrary to existing method of monitoring patients, for example, wherein doctors check-up on patients at defined time intervals (e.g., every six hours, etc.), the generative AI employed by data generation component 234 can provide a more efficient method of monitoring patients. For example, data generation component 234 can continuously parse the first patient data and determine whether patient *A* should be included in a different cohort such that another oncologist can benefit from the clinical decisions made by oncologist *A* for patient *A*.

The continuous monitoring can also assist a medical practitioner in determining how often the medical practitioner should monitor the patient. For example, in medical practice, Eastern Cooperative Oncology Group Performance Status (ECOG) is a clinical tool that is typically employed in oncology to assess a patient's functional status on a scale from zero (0) to five (5). ECOG can be employed alongside factors such as a patient's organ functions, existing comorbidities, physical well-being, drug safety, etc. to guide treatment decisions. For example, ECOG results for a patient in combination with the patient's organ functions, existing comorbidities, etc. can be matched with certain guidelines, results of other patients, labeled warning on drugs, etc. to determine whether a drug can be administered to a patient. For example, a medical practitioner can determine that a drug can cause anemia, upregulate a medical condition, cause dizziness, etc. Further, drugs can have their own toxicities or unwanted side effects.

In one or more embodiments, data generation component 234 can perform a juxtaposition to compare the ECOG results, medical journeys, medical contexts, organ functions, non-commercialities, etc. of different patients to determine specific medical conditions of patients and how different patients respond to different treatments. Based on the comparison, data generation component 234 can employ a filtering process to exclude patients having medical conditions that can render the patients unsuitable for a medication. For example, data generation component 234 can determine that a medication for anemia cannot be administered to a patient because the medication can cause the patient's health to worsen and cause the patient to return to the emergency room. Further, data generation component 234 can output the determination on the UI wherein the determination can be viewed by the patient's physician. Based on the determination, the patient's physician can determine how frequently the patient's condition should be monitored because the medication for anemia, although unsuitable for the patient, may need to be administered to the patient under certain circumstances. However, by monitoring the patient more frequently, the physician can be prepared to intervene instead of prematurely administering the anemia medication to the patient without accurate evidence.

In medical practice, physicians typically make clinical decisions by combining information received from multiple sources such as radiology, laboratory test results, genomic reports, patient preferences (e.g., preferences for avoiding side effects such as further loss of hair, loss of weight, loss of taste, burning the esophagus, etc.) and correlating the combined information with a patient's clinical symptoms and clinical responses instead of relying on information from a single source. Additionally, certain legal factors can also prevent physicians from intervening prematurely, for example, without concrete evidence. By monitoring a patient more closely based on the determinations made by data generation component 234, a physician can review past data related to multiple patients, superimpose the past data on the available treatment choices via AI model 210, and determine the most appropriate clinical decision by weighing the risks and the trade-offs. For example, instead of administering a medicine that is unsuitable for a patient, a physician can administer a supporting medication and monitor the patient frequently so that the physician can be prepared to intervene.

Additional embodiments of the present disclosure are described with reference to the subsequent figures.

FIG. 2B illustrates another block diagram of an example, non-limiting system 230 that can present a visualization of the medical journey of a patient, including a decision maker specific visualization, and that can have the ability to generate alternative treatment plans employable for subsequent treatment of a disease, in accordance with one or more embodiments described herein. Repetitive description of like elements and/or processes employed in respective embodiments is omitted for sake of brevity.

Non-limiting system 230 illustrates an exemplary system of AI model 210. As discussed with reference to FIG. 2A. AI model 210 may comprise data access component 232, data generation component 234, display component 236, first AI component 238, second AI component 240, plan seeker component 242, detection component 244, evaluation component 246, merger component 248, selection component 250, prompt generation component 252, reporting component 254 and training component 256.

In one or more embodiments, training component 256 can train data generation component 234 to iteratively analyze first data 212 and second data 214. For example, over time, new information can be added to first data 212 and second data 214 as the treatment for a patient progresses, and some of the new information can become training data for data generation component 234. For example, the one or more outcomes of a clinical decision can be added as feedback to a training dataset employed by training component 256 to train data generation component 234. Based on such iterative training, data generation component 234 can identify the most relevant cohorts (e.g., cohorts 216) and generate the alternative treatment plans based on the most up-to-date information related to a patient. Further, in one or more embodiments, relevant cohorts can be visualized in the decision maker view, via display component 236, to compare past treatment decisions related to similar patients (i.e., patients with similar medical conditions, characteristics, etc.) and to compare the resulting outcomes of the past treatment decisions. Such embodiments can further assist decision makers improve their decision making and learn from past decisions.

Training component 256 can iteratively train first AI component 238 and second AI component 240 by employing new relevant information as training data for first AI component 238 and second AI component 240.
In one or more embodiments, training component 256 can also train data generation component 234 to mitigate hallucinations. In machine learning, hallucinations refer to instances when a model generates outputs that are incorrect or biased. Hallucinations are commonly caused when models are trained on biased or incorrect data. For example, some of the training data employed to train data generation component 234 can comprise sparse data sets that can be biased. For example, a first demographic can be prone to a greater number of immune issues than a second demographic. If the training dataset comprises a disproportionate amount of data from the first demographic, data generation component 234 can become trained on features or correlations that are overly representative of the first demographic. As a result, when data generation component 234 is applied to a patient from the second demographic, data generation component 234 can generate results that reflect the skewed patterns.

To prevent such scenarios, training component 256 can train data generation component 234 to employ real world evidence in generating results. For example, if a hospital does not have enough data sets of a particular group (e.g., the first demographic), data generation component 234 can access publications from external sources (e.g., online publications, publications from other institutions, etc.) to include more relevant data and support information for an end entity (e.g., hardware, software, machine, AI, neural network and/or user). To achieve this, data generation component 234 can employ an LLM and one or more methods to prompt the LLM with the most accurate questions based on the context of a medical condition of a patient and a context available for the patient (e.g., first patient data). By fanning out to a publication search, data generation component 234 can provide real world evidence, for example, when identifying cohorts 216 for a patient.

Data generation component 234 can also be trained by training component 256 to notify an end entity (e.g., hardware, software, machine, AI, neural network and/or user) about the availability of sparse data. For example, data generation component 234 can notify a physician that due to the availability of sparce evidence in the context of a patient, the search for cohorts 216 was expanded to a publication search, and data generation component 234 can direct the physician to one or more publications. Thus, although the physician can make the final decision regarding how a patient's treatment should progress, data generation component 234 can present the most relevant, accurate and unbiased data (e.g., data about how previous clinicians arrived at a clinical decision, whether the clinical decision was based on guidelines, a second opinion, a multi-disciplinary team, a board meeting for a tumor, a cohort outcome, etc.) that can assist the physician with the decision-making process. As stated elsewhere herein, the data presented by data generation component 234 can comprise past clinical decisions made by a physician for a patient and outcomes of the past clinical decisions, as well as the clinical decisions and corresponding outcomes associated with other patients in similar situations. In one or more embodiments, such information can be overlaid or otherwise displayed on the UI as a comparative view with respect to the medical journey of a current patient. Based on the information displayed on the UI, a physician can make data-driven clinical decisions to treat a patient.

For example, with continued reference to the exemplary scenario involving patient *A* and oncologist *A* discussed with reference to FIG. 2A, data generation component 234 can identify that cohorts 216 had a 90% success rate with treatment *T1* and a 30% success rate with treatment *T2.* Accordingly, oncologist *A* can choose treatment *T1* (the popular choice) to determine the subsequent treatment for patient *A.* However, oncologist *A* can also decide to forego treatment *T1* due to contradictions related to patient *A.* For example, due to patient *A*'s preference for a certain quality of life, oncologist *A* can forego treatment *T1* if treatment *T1* is likely to reduce the quality of life of patient *A.* Similarly, oncologist *A* can forego treatment *T1* due to a contraindication. For example, treatment *T1* can be suitable for patient *A* in the short term, but treatment *T1*'s regimen can worsen some conditions in patient *A* that can eventually distract patient *A* from the cancer treatment. For example, patient *A* can have weak liver, and although the liver issues can be within acceptable clinical bounds, they can affect the lower quartile of the liver function. As a result, adopting treatment *T1* for patient *A* can increase the possibility of liver failure for patient *A.* Accordingly, oncologist *A* can decide to choose treatment *T2* to determine the subsequent treatment for patient *A* because the liver is one of the most vital organs in the human body, and a liver failure can heavily compromise the subsequent treatment for patient *A*. In oncology, surgery is often the only curative solution. Non-surgical alternatives are often directed to the prolongation of life with some defined quality of life. For example, despite being associated with the intent to cure cancer, non-surgical alternatives typically involve repeated cycles of treatment, recurrence, and diagnosis of cancer, and oncologists attempt to keep their patients disease free for longer durations before another diagnosis is needed. Thus, oncologist *A* can choose treatment *T2* (e.g., the minority case) because it can be more suitable for patient *A*.

In one or more embodiments, the data accessed by data generation component 234, for example, to identify cohorts 216, generate alternative treatment plans, etc. can comprise controlled datasets, have a high veracity, and be anonymized or de-identified to ensure that the data is not skewed with respect to a diagnosis. For example, data generation component 234 can also be trained by training component 256 to access real world data from within a hospital network, reputed universities, etc. to mitigate instances of hallucinations. In doing so, data generation component 234 can be confined to search for data within a defined knowledge space or a defined parameter space based on the context applicable to a particular patient. For example, in one or more embodiments, first AI component 238 can employ generative AI and natural extraction to parse the medical journey of a patient to a granular level, and create a set of data points for the patient. Based on the data points, data generation component 234 can execute a multi-dimensional query or a multi-parameter search, followed by relaxing the search filters employed for the multi-dimensional query or a multi-parameter search based on the context of the patient.

In one or more embodiments, to extract data that is not readily available as discrete data elements, data generation component 234 can employ a generative AI model because such data extraction involves knowledge of the data to be extracted to prevent an asymptotic data search. For example, the generative AI model employed by data generation component 234 can have access to a knowledge source such as a set of guidelines or prior data to define the data extraction process. For example, the guidelines can indicate to the generative AI model that if a patient exhibits certain parameters or attributes of certain parameters, then the generative AI model should extend the parameters. For example, if a patient has stage-2 lung cancer, the guidelines can limit the generative AI model's search to parameters, treatments, diagnostic content, etc. related to stage-2 lung cancer and stage-3 lung cancer. Limiting the generative AI model's search in this manner can ensure that the generative AI model searches for data within the confines of oncology while adhering to a context relevant to the patient. For example, the generative AI model can begin searching for data related to stage-2 and stage-3 lung cancer, and thereafter, relax the filters employed for the search based on the discrete data elements that characterize the stage-2 and stage-3 lung cancer. Such a knowledge-backed search can mitigate the hallucinations to a very small non-zero percentage.

Recall that data generation component 234 can employ first data 212, second data 214 and cohorts 216 to generate at least two alternative treatment plans employable to treat a patient. In one or more embodiments, data generation component 234 can also employ a utilitybased analysis to factor the cost of an incorrect recommendation versus the benefit of a correct recommendation. For example, patient *A* can be a 90-year old individual, and data generation component 234 can conclude that that although a particular treatment plan generated positive results in 90% of cohorts 216, the treatment plan is unsuitable for patient *A* given the frailty of patient *A* and can result in the death of patient *A.* In generating treatment plan recommendations, data generation component 234 can factor a tuple with two parameters, wherein one parameter can be the costs associated with different treatment plans and another parameter can be the quality of life (QOL). The costs can be measured in terms of the financial costs of different treatments or costs in terms of life lost, where life lost is defined by disability-adjusted life year (DALY). DALY can account for the years of a patient's life lost due to a wrong decision as a parameter in evaluating a treatment plan. The QOL can be measured in terms of toxicities, the quality-adjusted life years (QALY), and other similar parameters. QALY can determine disease-free progression or overall survival, which is the length of time that a patient remains disease free before a recurrence of disease. DALY and QALY are typically employed in medical practice, and data generation component 234 can employ DALY, QALY and/or additional parameters when recommending treatment plans.

In one or more embodiments, data generation component 234 can be trained by training component 256 to ensure that a patient's condition remains within a narrow swim lane instead of oscillating/swinging widely between extremes, because a wide oscillation/gamble can dent a patient's immune system. For example, a wide oscillation can cause the organs of the patient to experience high levels of toxicity thereby causing a natural reduction in the life of the organs, and ultimately, in the life of the patient. Parameters such as DALY, QALY, etc. can assist data generation component 234 in determining whether a patient's condition oscillated/swung widely between extremes, for example, whether the patient exhibited a good response or a bad response due to a clinical decision, even in treatment plans involving trial and error solutions. By factoring parameters such as the costs, DALY, QALY, etc., data generation component 234 can recommend treatment plans that can be tailored to a patient because even though two patients can be very similar in many aspects, they can respond differently to a treatment plan. For example, a patient can be low on appetite, low on weight and anemic. Data generation component 234 can determine that a potential treatment for the patient can affect a patient's salivary glands and prevent the patient from producing saliva, a condition known as Xerostomia, where a patient can experience difficulty in swallowing food and may need artificial saliva. Thus, recommending the treatment for the patient can worse the patient's condition when instead the patient can benefit from a protein-rich diet and some dietary restrictions.

In one or more embodiments, an end entity (e.g., hardware, software, machine, AI, neural network and/or user) can select a treatment plan on the UI via a cursor, touchscreen or other suitable functionality to view additional information about the parameters considered by data generation component 234 in recommending the treatment plan. For example, a medical practitioner can view the financial cost associated with a treatment plan.

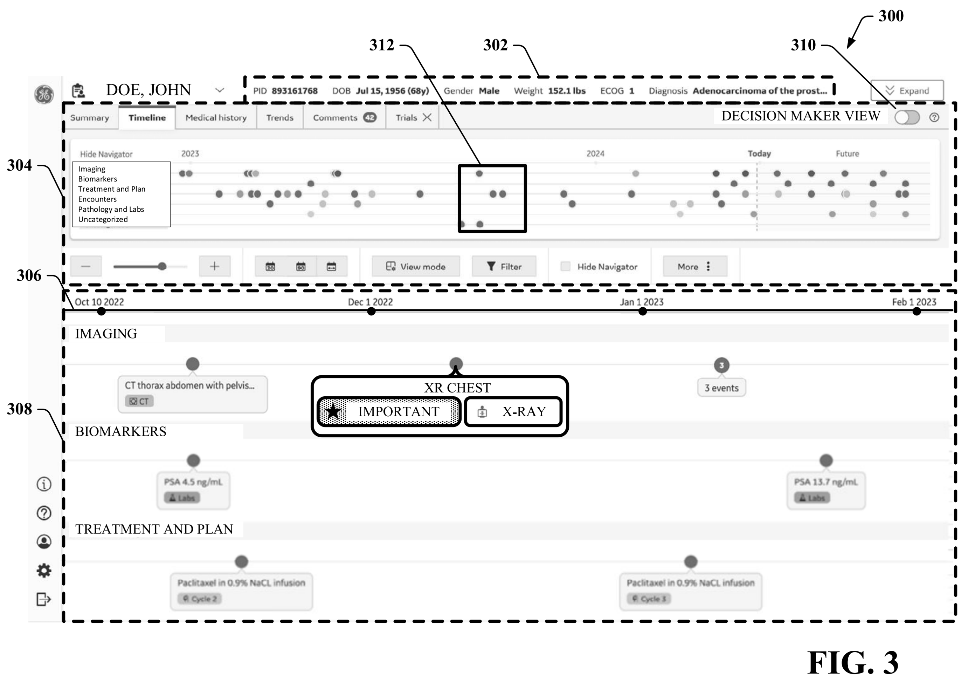

FIG. 3 illustrates a diagram of an example, non-limiting UI 300 displaying a temporal view of the medical journey of a patient, in accordance with one or more embodiments described herein. Repetitive description of like elements and/or processes employed in respective embodiments is omitted for sake of brevity.

Non-limiting UI 300 can be an interactive UI that can be displayed on a device (e.g., a computer, a laptop, a tablet, a smartphone, etc.) accessible to a medical practitioner, clinician, physician, nurse, and other medical professionals who may impact patient care. Non-limiting UI 300 can comprise, in the top margin, the name of a patient and information such as the date of birth (DOB), gender, and other relevant details of the patient, as illustrated by block 302.

UI 300 can also comprise an initial patient timeline, as illustrated by block 304, wherein the initial patient timeline can be displayed by display component 236 on the UI, based on first patient data and the treatment information comprised in first data 212. The initial patient timeline can comprise a temporal longitudinal view of the medical history of a patient and the treatment plan developed by a physician responsible for treating the patient. Additionally, the initial patient timeline can extend from a specific point in time in the past (e.g., 1 January 2023) to a future point in time (e.g., 1 January 2027). The initial patient timeline can display events such as imaging data, biomarker data, treatment and plan data, encounters, pathology and laboratory tests, uncategorized events, etc. as part of the medical care journey of the patient, wherein each event on the initial patient timeline can be displayed as a point (e.g., a data point), and different events can be displayed in different colors. For example, events in identical categories can be displayed with the same color and events in unidentical categories can be displayed with different colors. The information about the events displayed by the initial patient timeline can be pulled from disparate data sources such as EMR data, smartphone data, radiology information systems, urology and oncology information systems, and other sources. Further, the information can be pulled from various medical providers, different institutions, physicians, and other sources with different technology systems. Thus, embodiments of the present disclosure can provide a single, viewable patient journey based on information from multiple disparate data sources, which is a technical outcome that is useful for patients as well as clinicians.

UI 300 can further comprise an expanded view based on the initial patient timeline, as illustrated by block 308, wherein the expanded view can provide detailed information on the specific events displayed on the initial patient timeline. For example, in one or more embodiments, an end entity (e.g., hardware, software, machine, AI, neural network and/or user) can interact with non-limiting UI 300 to select one or more events to view additional details related to the selected events in the expanded view. Upon selection of the events, bounding box 312 can appear around the selected events to highlight the events. The expanded view can also comprise a date display, as illustrated at 306.

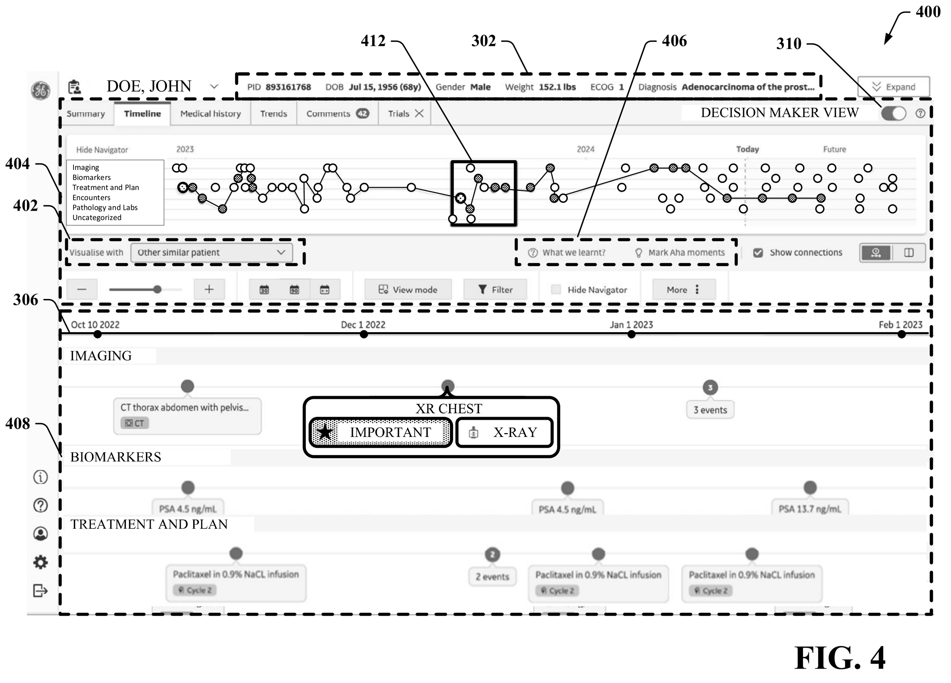

UI 300 can be generated by a software tool (e.g., the existing software tool referenced in paragraph [0042]) stored on a computer readable medium and executed via one or more computer processing units. In one or more embodiments, UI 300 can be updated via the provision of toggle switch 310, in addition to interactive components such as the menus, icons and other visual cues illustrated in FIG. 3. In one or more embodiments, end entity (e.g., hardware, software, machine, AI, neural network and/or user) can interact with toggle switch 310 to generate a specific view (which can comprise a disease-specific view, a decision maker view, or other updated views). For example, the physician can turn the toggle switch on via a cursor, touchscreen or other suitable functionality. As a result, display component 236 can generate a patient timeline showing only the decision maker view including the specific medical history and treatment plan corresponding to the patient, which is described further with reference to FIG. 4. Additionally, in one or more embodiments, UI 300 can comprise additional toggle switches 310, such as an oncology-focus toggle switch, a cardiology-focus toggle switch, a neurology-focus toggle switch, and so on. In FIG. 3, toggle switch 310 is illustrated in the inactive or "off" position. In one or more embodiments, non-limiting UI 300 can be viewed via a slider button instead of toggle switch 310.

FIG. 4 illustrates a diagram of an example, non-limiting UI 400 displaying a temporal view of the medical journey of a patient specific to a medical condition, in accordance with one or more embodiments described herein. Repetitive description of like elements and/or processes employed in respective embodiments is omitted for sake of brevity.

With continued reference to at least FIGS. 1 and 3, non-limiting UI 400 illustrates a decision maker view of non-limiting UI 300, wherein the decision maker view can be a longitudinal view comprising rows corresponding to different categories of medical information. For example, in addition to the name of the patient, block 302, the date display illustrated at 306, toggle switch 310 (here, in the active or "on" position), and the interactive components such as the menus, icons and other visual cues illustrated in FIG. 3, non-limiting UI 400 can comprise the patient timeline illustrated in block 404, wherein the patient timeline can display events such as imaging data, biomarker data, treatment and plan data, encounters, pathology and laboratory tests, uncategorized events, etc. specific to a medical condition of the patient. For example, in the exemplary scenario discussed earlier, wherein patient A can be a cancer patient being treated by oncologist A, the patient timeline illustrated in block 404 can display cancer-specific events related to patient *A.* For example, patient *A* can undergo a scan, take medications, receive chemotherapy, receive injections, etc. over a period of time as part of a cancer treatment plan prescribed by oncologist *A.* Oncologist *A* or another end entity (e.g., hardware, software, machine, AI, neural network and/or user) can monitor the cancer treatment plan for patient *A* and input details of the cancer-specific events into system 202 by employing non-limiting UI 400, based on which, display component 236 can display the events as points on the patient timeline comprised in non-limiting UI 400. Oncologist *A* can employ the cancer-specific view to travel backwards and forward on the patient timeline to different events and decision points (i.e., key decision moments) made over time. In some embodiments, non-limiting UI 400 can comprise a vertical view or another type of view as opposed to a longitudinal view.

As discussed elsewhere herein, first AI component 238 can detect decision points on the patient timeline illustrated in FIG. 4, wherein respective decision points can represent respective clinical decisions corresponding to patient A. The respective decision points can also represent key decision moments in the medical journey of patient A. The respective decision points can be moments in time when a clinical decision is made by a physician for the treatment of the patient such as, for example, a prescription approved, a treatment plan entered into a software program, the ordering of specific tests or procedures to be completed, and other key decision moments in the treatment of a disease. The patient timeline for a patient can comprise multiple decision points, and there can be multiple decision makers for a patient (e.g., a radiologist, a primary care physician, an oncologist, etc.). Display component 236 can highlight/emphasize respective decision points on the patient timeline via visual indicators (e.g., via blinking points, colored points, points larger than other points, a change in color of the points, etc.).

Additionally, second AI component 240 can detect one or more outcomes (e.g., patient journey points, events, clinical outcomes) resulting from a decision point. For example, in an embodiment, oncologist A can interact with non-limiting UI 400 to select a decision point of interest. In another embodiment, second AI component 240 can select the decision point automatically or based on instructions provided by oncologist A via interaction with non-limiting UI 400. Upon selection of the decision point, second AI component 240 can detect the one or more outcomes (e.g., patient journey points, events, clinical outcomes) resulting from the decision point. Display component 236 can also highlight the one or more outcomes (e.g., patient journey points, events, clinical outcomes) via visual indicators. In one or more embodiments, display component 236 can highlight the selected decision point and the one or more outcomes (e.g., patient journey points, events, clinical outcomes) resulting from the selected decision point via bounding box 412 and show visual connections (e.g., edges, lines) between decision points and outcomes resulting from the decision points. That is, display component 236 can highlight key decision moments in the medical journey of patient A, and when a key decision moment is clicked on or selected, display component 236 can box the related treatment. In one or more embodiments, display component 236 can also update the data displayed in block 408, based on bounding box 412, to show more detailed information (e.g., an expanded view), including visual connections, related to only the selected key decision moment. For example, block 408 can display a zoomed-in (e.g., more detailed) view of the selected key decision moment and the corresponding outcomes that are within bounding box 412.

In FIG. 4, each circle illustrated on the patient timeline can represent an event that can be selected by oncologist *A* to learn more about the event, the outcomes related to the event, patient A's adherence to the event and other information related to the event, such as information about chemotherapy, surgery, medicine, diet, etc., and such details can be reviewed by oncologist *A* in a very visual and helpful manner via non-limiting UI 400. The expanded view illustrated by block 408 can provide detailed information on the specific events displayed on the patient timeline in connection with a specific decision point. For example, oncologist *A* can interact with UI 400 to select a decision point (i.e., a key decision moment) to view additional details related to the selected decision point in the expanded view. In one or more embodiments, once a decision point is selected (e.g., by selection component 250 or oncologist *A*), the other parts (e.g., events) of the patient timeline can disappear or become grayed out to allow oncologist *A* to focus on the outcomes resulting from the decision point.

Non-limiting UI 400 can also comprise interactive components that can be employed by an end entity (e.g., hardware, software, machine, AI, neural network and/or user) to generate visualization 222, ranked list 220 and AI-based report 224. For example, non-limiting UI 400 can comprise a drop-down menu, as illustrated in block 402, that can be employed by oncologist *A* to select the option to visualize the medical journey of patient *A* with medical journeys of other cancer patients that have cancer received treatment in the past. In an embodiment, the drop-down menu can show a default selection to visualize the medical journey of patient *A* upon activating toggle switch 310, as illustrated in FIGS. 4 and 5. Non-limiting UI 400 can also comprise the option to generate a report (e.g., AI-based report 224) via the "What we learnt?" option and the option to mark aha moments via the "Mark Aha moments" option, as illustrated in block 406.

In one or more embodiments, non-limiting UI 400 can additionally comprise a second toggle switch or drop-down menu (not illustrated) that can allow an end entity (e.g., hardware, software, machine, AI, neural network and/or user) to view second opinions. For example, oncologist *A* can select the second toggle switch to view second opinions received by other cancer patients in the past, wherein such second opinions can assist oncologist *A* in determining the subsequent treatment for patient *A*. The second toggle switch can be particularly useful in oncology and other areas where patients commonly get second, third or even fourth opinions. Publications in journals such as the Wall Street Journal indicate that decision makers (e.g., medical practitioners) often do not possess all the knowledge, for example, knowledge about decisions made by other decision makers or latest medical guidelines because medical guidelines typically change rapidly. Thus, the second toggle switch can act as a tool that can allow physicians to view second opinions based on the latest guidelines. In general, non-limiting UI 400 can be employable to view multiple types of focus views.

These embodiments benefit decision makers by allowing them to learn more about other ways in which they could have made a previous or upcoming clinical decision and to think through their clinical decisions with more information presented in an easy to digest manner. In addition, the decision maker view can be useful to medical payment companies, payors, healthcare agencies, and others looking at the quality of decision making and outcomes for patient care. If a hospital leader is trying to improve clinical outcomes, they can use the embodiments of the present disclosure to learn specifics based on general benchmarking. For example, the embodiments of the present disclosure can assist hospital leaders review specific decisions and decision-makers. This can also be done at the national or international level. The systems and methods disclosed herein can also be used for training and improving junior medical professionals, such as medical residents. Trying to understand how past clinical decisions were made by a physician and comparing the past clinical decisions to specific outcomes can provide significant value in improving medical care, as such analysis can be enabled by the systems and methods disclosed herein.

FIG. 5 illustrates diagrams of example, non-limiting patient timelines 500 and 510 to show a comparison between the two patient timelines, in accordance with one or more embodiments described herein. Repetitive description of like elements and/or processes employed in respective embodiments is omitted for sake of brevity.

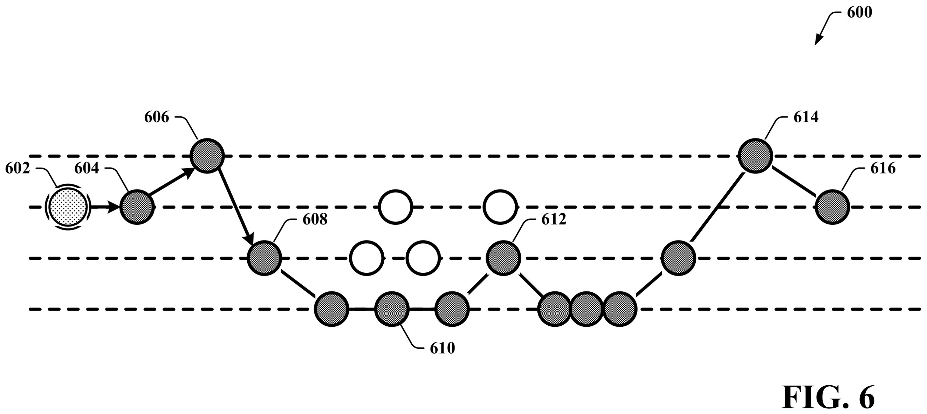

Non-limiting patient timeline 500 illustrates the patient timeline from FIG. 3, and non-limiting patient timeline 510 illustrates the patient timeline from FIG. 4. Non-limiting patient timeline 500 (FIG. 3) and non-limiting patient timeline 510 (FIG. 4) are intended to show a comparison between the generic view/default view of the patient timeline and the decision maker specific view of the patient timeline. Highlighted in non-limiting patient timeline 510 are decision point 512 and decision point 514 that can be decision points detected by first AI component 238 on non-limiting patient timeline 510. As described with reference to FIG. 4, display component 236 can display visual connections (e.g., edges, lines) between decision points and outcomes resulting from the decision points such as, for example, the visual connections between decision point 514 and outcomes 514A and 514B. The outcomes resulting from the decision points can be any events, patient journey communications, data collections, procedures, and other specific outcomes related to the medical/healthcare journey of a patient. The visual connections can originate from decision point 512 and travel towards other decision points and their outcomes to highlight a sequence of the decisions made by oncologist *A* after decision point 512 as part of the medical journey of patient *A*. In one or more embodiments, non-limiting patient timeline 510 can also show overlays of outcome metrics of other patients for certain decision points, for example, based on cohorts 216. As discussed with reference to FIG. 4, once a decision point is selected (e.g., by oncologist *A*), the other events of non-limiting patient timeline 510 can disappear or become grayed out to allow oncologist *A* to focus on the outcomes resulting from the decision point. This concept is illustrated in greater detail with reference to FIG. 6.

FIG. 6 illustrates a diagram of an example, non-limiting patient timeline 600 displaying a portion of the medical journey of a patient, in accordance with one or more embodiments described herein. Repetitive description of like elements and/or processes employed in respective embodiments is omitted for sake of brevity.

With continued reference to at least FIGS. 1 and 3 - 5, non-limiting patient timeline 600 is intended to illustrate some aspects of patient timeline 510 in greater detail. In patient timeline 600, event 602 can represent a decision point, the additional events illustrated by the patterned circles can represent outcomes of the decision point, and the events illustrated by the hollowed circles can represent grayed out events. For example, non-limiting patient timeline 600 can illustrate the main progression of the medical journey of patient *A*. Event 602 can be a decision made by oncologist *A* to prescribe chemotherapy to patient *A.* Accordingly, event 604 can represent a blood test performed on patient *A*, for example, to check tubular Reabsorption of Phosphorous (TRP) levels, event 606 can represent a scan or imaging performed on patient *A*, event 608 can represent the first dose of chemotherapy administered to patient *A*, event 610 (and other the events to the immediate left-hand side and right-hand side of event 610) can represent respective diet checks performed by oncologist *A* via oncologist *A*'s smartphone. Event 612 can represent a second dose of chemotherapy administered to patient *A*, event 614 can represent a second scan or imaging performed on patient *A* to monitor the effects of the chemotherapy, and event 616 can be an outcome based on the preceding events which can be followed by the next decision point. In an embodiment, oncologist *A* can select event 602 to view overlays of outcome metrics related to clinical decisions made for other patients. It should be appreciated that in FIG. 6, the hollowed circles not connected to other events or to each other via lines can represent additional parts of the medical journey of the patient, such as parts not related to event 602.

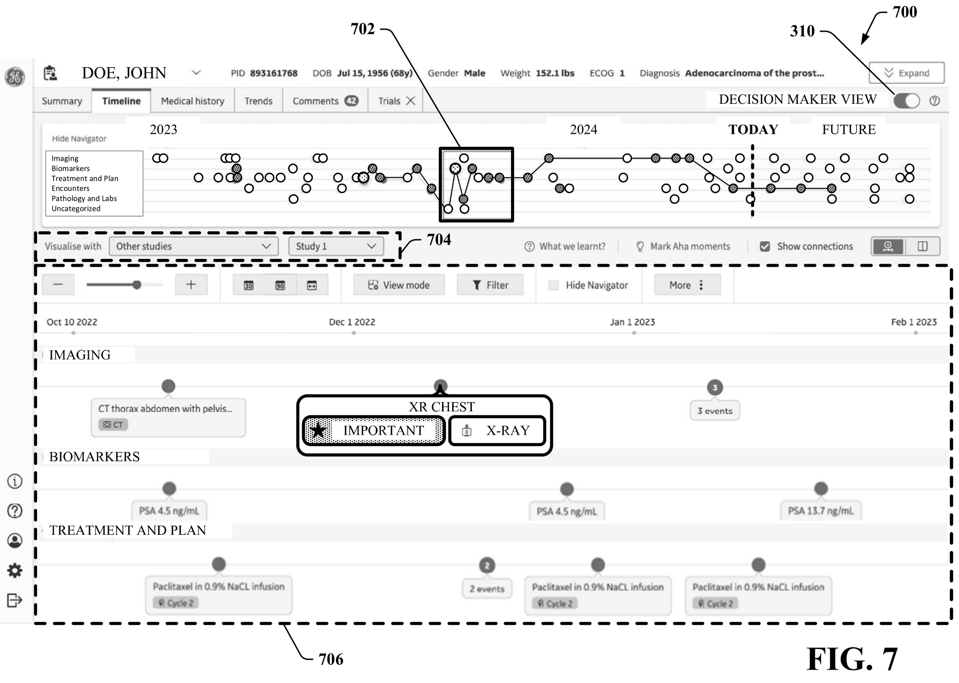

FIGS. 7 - 11 illustrated additional aspects of non-limiting UI 400 and non-limiting patient timeline 510.

FIG. 7 illustrates a diagram of an example, non-limiting UI 700 that can be employed to visualize medical studies related to the medical condition of a patient, in accordance with one or more embodiments described herein. Non-limiting UI 700 can be analogous to non-limiting UI 400 with toggle switch 310 in the "on" position. Additionally, the visualization drop-down menu in block 704 can be set to view other medical studies (e.g., cancer research studies) similar to the medical journey of patient A, which can allow the viewing of separate visuals related to the other medical studies, in block 706. Alternatively, one or more other medical studies can be visually overlaid or superimposed with the current patient information for a direct comparison view. The similarities and differences between clinical decisions and outcomes for a current patient and clinical decisions and outcomes for patients with similar circumstances discovered via the "Other studies" option, in addition to the information that can be generated via the "What we learnt?" feature, can be employed by a clinician to investigate reasons for why certain clinical decision may have generated certain outcomes. Bounding box 702 can highlight a decision point (e.g., different from decision point 514) and outcomes of the decision point, wherein the decision point can be selected by oncologist *A* or by selection component 250, and block 706 can represent an expanded view that can display additional details of one or more events selected by oncologist *A* on the patient timeline.

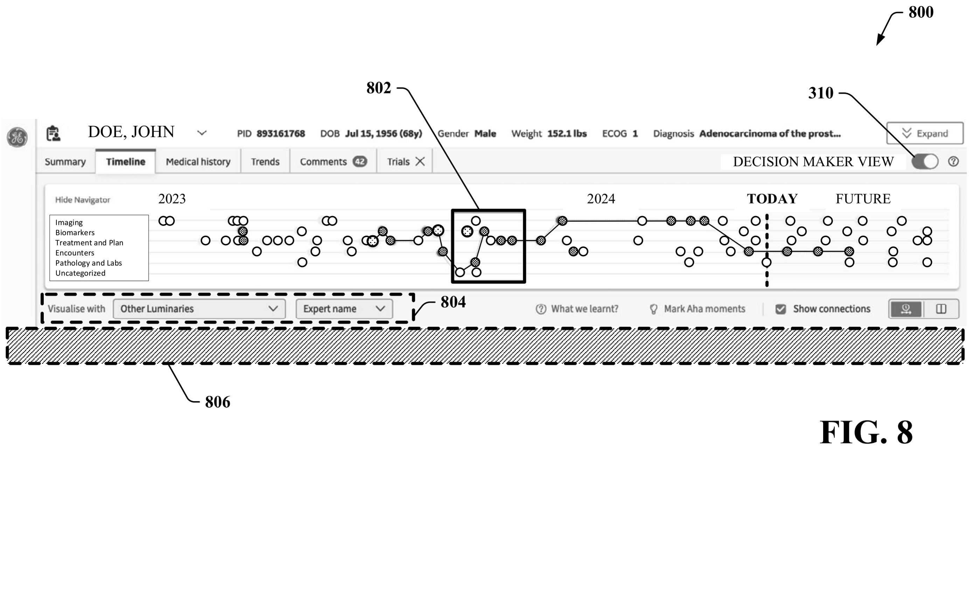

FIG. 8 illustrates a diagram of an example, non-limiting UI 800 that can be employed to visualize treatment plans employed by medical practitioners specializing in the treatment of the medical condition of a patient, in accordance with one or more embodiments described herein. Repetitive description of like elements and/or processes employed in respective embodiments is omitted for sake of brevity.

Non-limiting UI 800 can be analogous to UI 400 with toggle switch 310 in the "on" position, and the visualization drop-down menu in block 804 set to view treatment plans employed by medical practitioners (e.g., luminaries or experts) in a medical field related to a disease that a patient has such as, for example, in the field of oncology to treat the type of cancer that patient *A* has. Bounding box 802 can highlight outcomes of a decision point selected by oncologist *A* or selection component 250. As illustrated in block 804, non-limiting UI 800 can comprise a second drop-down menu that can display ranked list 220, wherein ranked list 220 can be generated by data generation component 234. Oncologist *A* can select an expert's name in the second drop-down menu to view one or more treatment plans employed by the expert and the corresponding outcomes. In one or more embodiments, upon selecting an expert's name, additional options can be displayed on non-limiting UI 800 that can be employed by oncologist *A* to contact the expert for additional details.

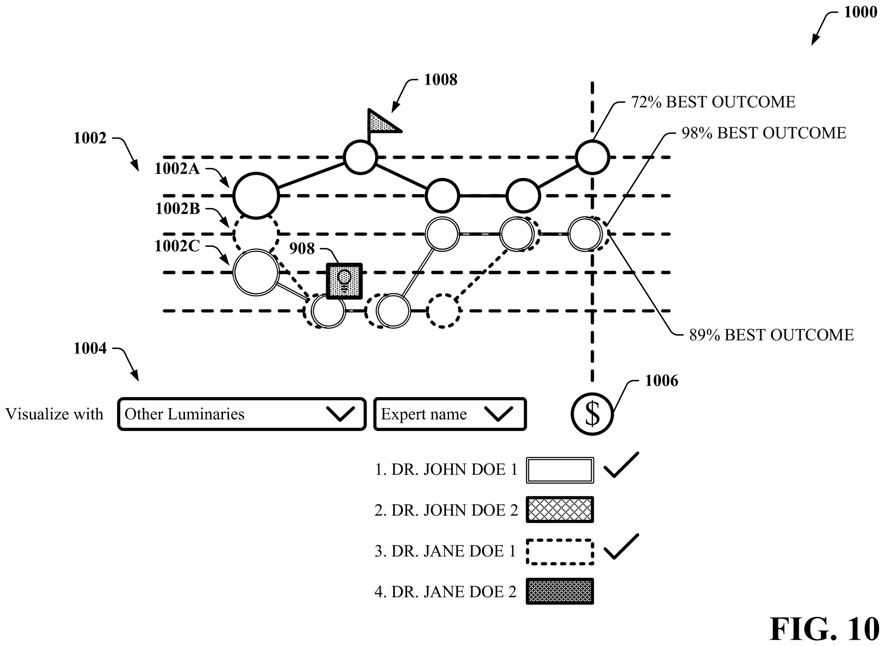

The ability of data generation component 234 to present the most relevant, accurate and unbiased data can be especially useful in situations where a medical practitioner does not have significant experience in treating a medical condition or a special case of a medical condition. For example, a general oncologist practicing in a rural area can develop a treatment plan with a 72% best outcome for an uncommon form of cancer, whereas data generation component 234 can determine that luminaries in the field have developed treatment plans with 89% best outcomes or even 93% best outcomes. The information learnt by data generation component 234 can be displayed by display component 236 on non-limiting UI 800 in a visual manner to assist the general oncologist in adjusting the treatment plan based on the treatment plans employed by the luminaries. In one or more embodiments, display component 236 can also display on non-limiting UI 800, reminders generated by data generation component 234 for the general oncologist, in addition to ranked list 220, to assist the general oncologist in making better clinical decisions. The details of an example output that can be generated by employing the "Other luminaries" options of block 804 are discussed with reference to FIG. 10, and although not illustrated in FIG. 8, such an example output can be displayed within block 806. It should be appreciated that in FIG. 8, block 806 is intended to represent an expanded view of UI 800, similar to the expanded view illustrated by block 408 in FIG. 4.

In general, the visualization drop-down menu illustrated in at least FIGS. 7 and 8 can be employed by an end entity (e.g., hardware, software, machine, AI, neural network and/or user) to view additional data that can be useful in determining the subsequent treatment for a patient. In one or more embodiments, upon selecting a desirable option via the drop-down menu, data generation component 234 can employ the digital twin of a patient and other technologies available to show suggested paths forward (e.g., alternative treatment plans) based on factors related to decision-making suggestions. For example, data generation component 234 can consider factors such as cancer guidelines, relevant laws and regulations, data from other experts in a field, wherein the experts can be ranked or assigned a rating (e.g., based on the specific form of cancer in a patient), outcomes of treatment plans employed by other experts (e.g., in the form of actual data, stories, online data, medical trials, etc.), past errors made by a clinician, data from the patient (e.g., test data, scans, experiences or other relevant data), aha moments, and so on. Additionally, data generation component 234 can rank the factors via a neural network with weighting to identify the alternative treatment plans.

In one or more embodiments, the visualization drop-down menu can also be employed by an end entity (e.g., hardware, software, machine, AI, neural network and/or user) to view recommendations by institutions or exemplary cases based on ever changing and improving guidelines for a certain type of cancer and the related medications. For example, oncologist A can employ the drop-down menu to select organizations such as the National Comprehensive Cancer Network (NCCN), a governing medical body of a country, etc. Upon selecting an organization, data generation component 234 can access the relevant guidelines from the organization based on factors such as the medical condition of patient A, etc., and display component 236 can display the guidelines on the UI where the guidelines can be viewed by oncologist A. Typically, clinical decisions are based on the relevant guidelines. Thus, the ability of data generation component 234 to access various guidelines can be very valuable for medical practitioners.

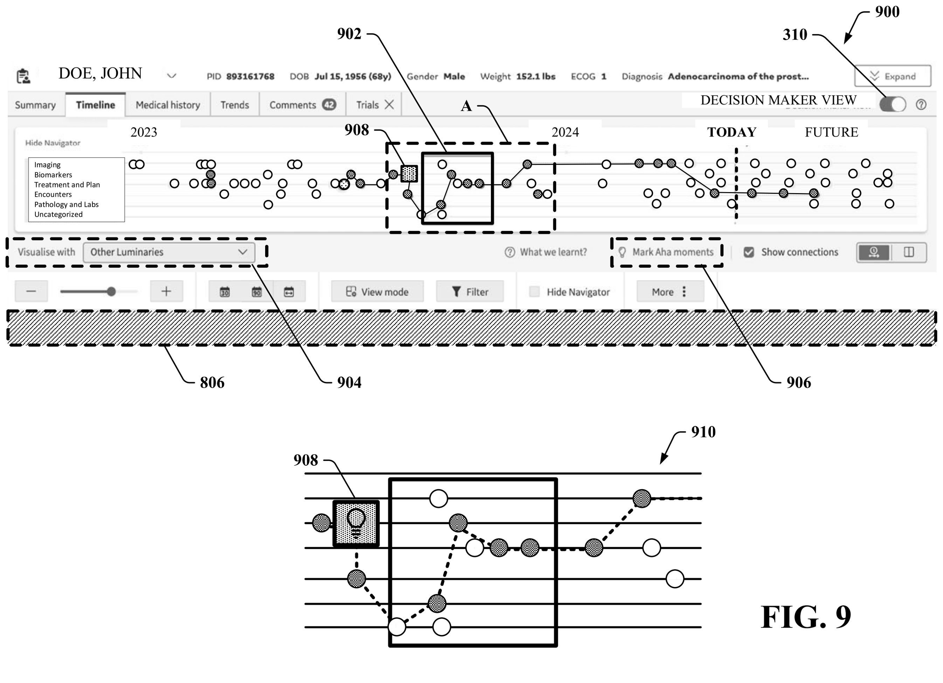

FIG. 9 illustrates a diagram of an example, non-limiting UI 900 that can be employed to mark aha moments in the medical journey of a patient, in accordance with one or more embodiments described herein. Repetitive description of like elements and/or processes employed in respective embodiments is omitted for sake of brevity.

Non-limiting UI 900 can be analogous to non-limiting UI 400 with toggle switch 310 in the "on" position, and the visualization drop-down option in block 904 selected to view treatment plans employed by medical practitioners (e.g., luminaries or experts) in the field of oncology to treat the type of cancer that patient A has. In FIG. 9, block 806 is representative of block 806 from FIG. 8.

In non-limiting UI 900, the events (e.g., procedures, outcomes, follow ups, tests, etc.) resulting from a decision point (e.g., a decision point selected by oncologist A) are highlighted via bounding box 902 on the patient timeline. The patient timeline further illustrates block A. An expanded view of block *A* is illustrated at 910 in FIG. 9 and displays aha moment 908. Recall that in one or more embodiments, first AI component 238 can detect one or more decision as aha moments, wherein aha moments represent key moments when a non-standard insight is identified by an end entity (e.g., hardware, software, machine, AI, neural network and/or user). Aha moments can be thought of as improved thinking on the part of a chess player in a game of chess as a result of the chess player deviating from the norm during a move. For example, oncologist *A* can select a decision point followed by selecting the "Mark Aha moments" button illustrated in block 906 to capture a good decision made during the medical journey of patient *A,* or oncologist *A* can have a sudden realization to alter the treatment plan for patient *A* and mark aha moments based on the sudden idea. Thus, aha moments can comprise good decisions captured and moments that can be valuable to oncologist *A* in the treatment of cancer for patient *A* and to the medical industry, for example, in treating a particular type of cancer. Aha moments marked by oncologist *A* can also become available to other medical practitioners practicing oncology, dealing with cohorts similar to cohorts 216, and so on. Aha moments can also be interesting or unique learnings that could be harvested, along with other aha moments identified by other decision makers managing treatments for the same disease, to potentially detect new medicines or treatments for that disease.

In one or more embodiments, aha moments can also be automatically detected and marked by first AI component 238. For example, first AI component 238 can employ software/algorithms to detect decision points of sudden realizations by a physician, abnormal occurrences, actions taken by the physician that fall outside of typical practice, etc. and mark such decision points as key moments or aha moments that can be useful for oncologist *A* or other physicians. Thus, aha moments can be described as a collection or recording of wisdom over time. Aha moments can include detection, via voice input, of a conversation between a decision maker and a patient, or a decision maker and a nurse, for example, where a reflection by the decision maker is verbally communicated and includes a non-standard insight related to the patient, disease, treatment plan, and/or medical care. In addition, the closer that a patient and the medical condition of the patient is to another patient and their medical condition, the more likely it is for the aha moment from another medical professional or the other patient to be relevant to the patient. In such scenarios, UI 900 can display on a screen (in faded view or other highlighting) potential relevant aha moments for the decision maker to review. Thus, individual aha moments as well as patterns found in aha moments detected by first AI component 238, via analysis or artificial intelligence, can be useful to decision makers.

FIG. 10 illustrates a diagram of an example, non-limiting UI 1000 displaying an aha moment and a parallel view of medical journeys of multiple patients and aha moments, in accordance with one or more embodiments described herein. Repetitive description of like elements and/or processes employed in respective embodiments is omitted for sake of brevity.

With continued reference to at least FIGS. 1, 8 and 9, non-limiting UI 1000 is intended to illustrate some aspects of non-limiting UIs 800 and 900 in greater detail. In non-limiting UI 1000, patient timeline 1002 displays a parallel view of alternate medical journeys of patients treated by different luminaries. For example, medical journey 1002A can represent the medical journey of patient *A*, medical journey 1002B can represent the medical journey of patient *B,* and medical journey 1002C can represent the medical journey of patient *C,* wherein patient *B* can be Dr. Jane Doe 1's patient and patient *C* can be Dr. John Doe 1's patient. The respective medical journeys of patients *A, B* and *C* can be distinguished by different colors, patterns and/or other visual indicators. Medical journeys 1002B and 1002C can be displayed by display component 236 on patient timeline 1002 in response to a selection made by oncologist *A* to visualize the decision points and the corresponding outcomes in patients of other luminaries specializing in the treatment of cancer, as illustrated at 1004 in FIG. 10. For example, oncologist *A* can select "Other luminaries" in the first drop-down menu to view ranked list 220 comprising names of cancer experts identified by data generation component 234 based on factors, situations, etc. similar to those of patient *A.* Thereafter, oncologist *A* can select (e.g., by clicking on check boxes) the names of cancer experts Dr. John Doe 1 and Dr. Jane Doe 1 in the second drop-down menu to generate the view displayed by patient timeline 1002. In one or more embodiments, oncologist *A* can also manually rank the luminaries by interacting with non-limiting UI 1000. In one or more embodiments, oncologist *A* can employ the visualization drop-down menu to view the medical journeys of cancer patients previously treated by oncologist *A.*

In one or more embodiments, oncologist *A* can select decision points in medical journeys 1002B and 1002C to view additional details such as outcomes of the respective medical journeys, progression of the respective treatments, etc. in the expanded view of non-limiting UI 1000. In one or more embodiments, patient timeline 1002 can further display an outcome results review comprising scores for outcomes of medical journeys 1002A, 1002B and 1002C. In one or more embodiments, oncologist *A* can select a score to view how each score was calculated. In one or more embodiments, respective scores for respective medical journeys can be calculated by data generation component 234 or another component of AI model 210, wherein the respective scores can indicate the success rates of the respective medical journeys. For example, data generation component 234 can calculate a 72% best outcome score for medical journey 1002A, a 98% best outcome score for medical journey 1002B, and an 89% best outcome score for medical journey 1002C. In one or more embodiments, data generation component 234 can generate percentile scores or other types of scores to indicate the success rates of the respective medical journeys.

Thus, non-limiting UI 1000 can assist an end entity (e.g., hardware, software, machine, AI, neural network and/or user) to visualize the medical journeys of multiple patients by overlaying the medical journeys across patient timeline 1002. For example, oncologist *A* can select an option from the visualization drop-down menu to view medical journeys 1002B and 1002C and deselect the option to remove medical journeys 1002B and 1002C from patient timeline 1002. The overlayed medical journeys can assist oncologist *A* in viewing cause and effect relationships and correlations between parameters for different patient groups.

In one or more embodiments, the generative AI employed by data generation component 234 can continuously index patient timeline 1002 with new data from first data 212 and second data 214, differentiated data, connected/related data or suggestive data as the medical journey of a patient evolves. As stated elsewhere herein, the overlayed medical journeys of different patients can present various treatment options to a physician to determine subsequent treatment for a patient, and the physician can interact with non-limiting UI 1000 to further explore individual treatment options and the probabilities associated with the individual treatment options. For example, to ensure that patient *A* can remain in remission, oncologist *A* can review basic outcome metrics such as DALY, QALY, etc. associated with each treatment option. For example, a first treatment option can be associated with a two year progression-free survival period, a second treatment option can be associated with a progression-free survival period of one year and eight months, and a third treatment option can be associated with a progression-free survival period of three years. Progression-free survival can refer to a time duration during which a patient can survive without needing clinical intervention. Oncologist A can select the third treatment option with the longest progression-free survival duration, or oncologist A can select a different treatment option depending on other factors. For example, patient A may have experienced six emergency situations within a two year progression-free survival period, leading to a poorer QOL, and the QOL information can be one of the basic outcome metrics displayed by display component 236, on non-limiting UI 1000, alongside each treatment option. Based on the QOL information for patient *A* and/or patient *A*'s preference for a certain QOL, oncologist *A* can determine that the second treatment option is more suitable for patient A despite the third treatment option being associated with the longest progression-free survival period. Thus, the generative AI can continuously operate in the background to extract information from first data 212 and second data 214, compute the relevant outcome metrics and employ the outcome metrics to overlay data in a visual manner on non-limiting UI 1000.

In one or more embodiments, the option to mark aha moments can also be employed by an end entity (e.g., hardware, software, machine, AI, neural network and/or user) to mark decision points appearing in the medical journeys of other patients as aha moments. For example, oncologist *A* can mark a decision point in the medical journey of patient C as aha moment 908 by selecting the "Mark Aha moments" button illustrated in FIG. 9 to capture a different decision made by Dr. John Doe 1 as compared to oncologist *A.*

In some embodiments, UI 1000 as well as other UIs illustrated in other figures can comprise an option to generate cost information. For example, as illustrated at 1006 in FIG. 10, UI 1000 can comprise a button that can be selected by an end entity (e.g., hardware, software, machine, AI, neural network and/or user) to view cost information related to different treatment plans. Upon selection of the button by the end entity (e.g., hardware, software, machine, AI, neural network and/or user), data generation component 234 can generate the cost information, and display component 236 can visually display the cost information on UI 1000, wherein the cost information can be displayed for the various types of visualizations that can be generated by the embodiments of the present disclosure as well as for respective medical journeys comprised in a cohort. The cost information can indicate the costs billed for respective events (e.g., scans, medications, doctor visits, etc.) in the medical journey of a patient and the overall treatment cost to a patient. For example, the cost information can indicate the cost associated with each event on patient timeline 1002 as well as the cost for an entire section of patient timeline 1002 from a first decision point to a second decision point. In some scenarios, a luminary can employ the cost information and develop a treatment plant that can result in the same outcome as that resulting from another treatment plan developed by another luminary, but cost significantly less (e.g., $100,000 less) than the treatment plan developed by the other luminary. Thus, the cost information can be very valuable for decision makers (e.g., oncologist *A*) and for other end entities (e.g., hardware, software, machines, AI, neural networks and/or users) that may desire the cost information. For example, the cost information can be valuable to leaders (e.g., managers, presidents, etc.) of medical facilities and institutions for making large equipment purchases and designing medical facilities (e.g., hospitals, clinics, medical research institutions, etc.). In general, the cost information can assist end entities (e.g., hardware, software, machines, AI, neural networks and/or users) with small and large decisions, assist medical systems (e.g., medical facilities and institutions) with reducing costs and saving money, and assist decision makers with achieving better clinical outcomes.

In one or more embodiments, data generation component 234 can recommend (e.g., to a decision maker), based on the cost information, treatment avenues or treatment plans having high (e.g., highly positive) outcomes and lower costs. For example, a software algorithm and/or AI model employed by data generation component 234 can analyze data points on patient timeline 1002 and synthesize a recommendation/recommend an action or clinical decision for the decision maker. If the decision maker desires to understand why the action is being recommended by data generation component 234, the decision maker can evaluate the decision maker views described herein to understand the specific events and clinical decisions that result in the lower costs and high outcomes. For example, oncologist *A* may need to select a treatment option from surgery, therapies, transplants, theranostics, medication, and so on, and an analysis comparing the cost information and outcomes associated with different medical journeys comprised within cohorts 216 and clinical decisions made by other luminaries can be valuable in selecting the treatment option. In one or more embodiments, data generation component 234 can also analyze other quality metrics to perform comparative analyses of costs and outcomes associated with different treatment plans, wherein such quality metrics can comprise time to diagnosis, time to treatment initiation, disease free interval, progression free survival, overall survival and so on.

An additional technical feature of the systems and methods described herein is comparing the treatment plans associated with different patients within UI 1000 and determining whether the treatment plan for a patient at a first hospital can be employed for another patient (e.g., a local patient) at a second hospital. For example, when performing the comparative analyses of cost information and outcomes associated with different treatment plans, data generation component 234 can answer questions such as, "Is a patient eligible for certain discounts that another patient received?" or "Does this hospital have the specific medical equipment employed at a luminary's location to implement the treatment plan developed by the luminary or will different equipment be needed?", and so no. Such evaluations can be beneficial to decision makers because one patient's medical journey typically cannot be exactly replicated for another patient even if the medical journey is known to have generated positive outcomes. In one or more embodiments, data generation component 234 can also assign flags or other visual indicators on a comparative or overlaid view of the relevant information for a decision maker. For example, data generation component 234 can assign a flag to an event on patient timeline 1002, such as the flag illustrated at 1008, with a note that says, "This scan was performed with a new theranostics machine that our hospital does not have." The flag can be displayed by display component 236 in response to a decision maker clicking on a notification icon or a decision point on UI 1000, and the note can be displayed by display component 236 in response to the decision maker further hovering a cursor over the flag or otherwise selecting the flag via UI 1000. Such flags and the associated notes can be very valuable because different treatment plans may have been implemented in different ways and with different techniques at various medical facilities. Additionally, some medications and equipment may not be available at certain medical facilities. For example, certain rural hospitals or hospitals in lower income areas may not have access to medical professionals or equipment, and the embodiments disclosed herein can assist such medical facilities in making the best decisions given the facilities and personnel available.

FIG. 10 shows clear benefits to the improved technology of certain embodiments. Instead of flying across the country and paying thousands of dollars to talk with a certain luminary, or waiting three months to get into a luminary's schedule, UI 1000 can show helpful information related to clinical decisions and insights of the luminary on a useful screen. This is beneficial for patients as well as decision makers. The visualization tool may also allow conversation between luminaries where they can talk to each other digitally about why certain patient timelines and related decisions were made and share thoughts and ideas. Such functionalities can be enabled in a manner that can be easily understood by an end entity (e.g., hardware, software, machine, AI, neural network and/or user) based on the visual and technological aspects of the embodiments of the present disclosure.

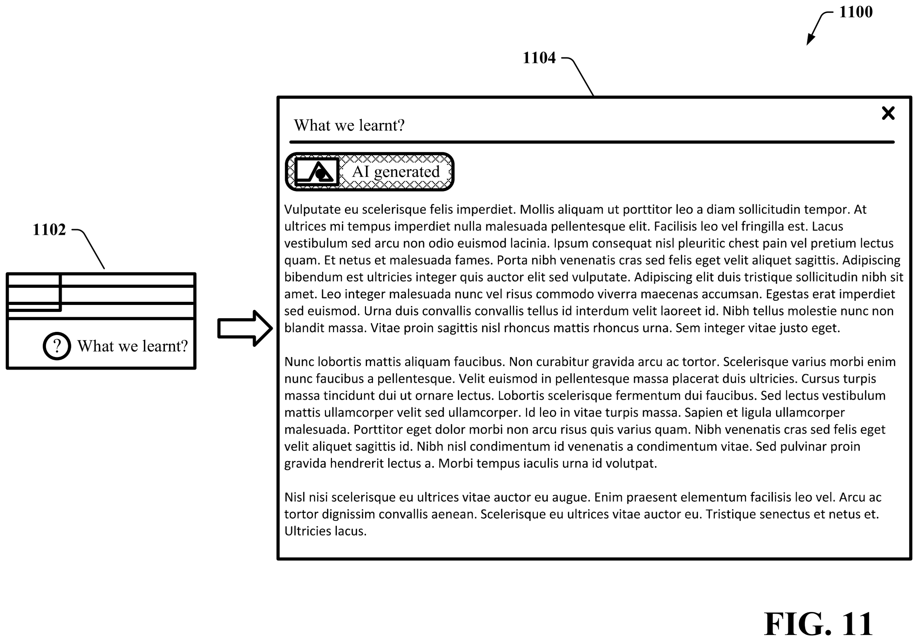

FIG. 11 illustrates a flow diagram of an example, non-limiting process 1100 that can generate an AI-based report to summarize the medical journey of a patient, in accordance with one or more embodiments described herein. Repetitive description of like elements and/or processes employed in respective embodiments is omitted for sake of brevity.

With continued reference to FIG. 2A, non-limiting process 1100 illustrates how AI-based report 224 (e.g., a generative AI report) can be generated. For example, at 1102, an end entity (e.g., hardware, software, machine, AI, neural network and/or user) can select the "What we learnt?" button, in response to which, reporting component 254 can generate, at 1104, AI-based report 224 comprising an explanation of information learned by data generation component 234 based on outcomes associated with the respective clinical decisions and recommendations for the subsequent treatment of the medical condition. For example, reporting component 254 can act as an outcome explanation module that can employ generative AI to explain the outcomes of clinical decisions made by oncologist *A* for patient *A* in AI-based report 224. For example, AI-based report 224 can comprise an explanation of information learnt by data generation component 234 in a prior round of chemotherapy for patient *A,* how the chemotherapy impacted the subsequent treatment for patient *A,* whether the chemotherapy was a good or a bad decision, whether the decision to alter the treatment plan mid-way through the treatment based on a complaint from patient *A* was a good decision, etc.

Typically, oncologists and other specialists are highly accomplished and very well compensated for their work, which can lead to some specialists viewing their opinions in high regard. However, specialists can sometimes make wrong treatment decisions, and the feedback from AI-based report 224 can prevent such misdirection. For example, AI-based report 224 can inform an oncologist dealing with a rare form of cancer that a clinical decision made by the oncologist in the past was not the best decision. For example, reporting component 254 can mine for the relevant data and determine that the clinical decision made by oncologist A to alter the treatment for patient *A* mid-way through the treatment plan was a poor decision.

In one or more embodiments, AI-based report 224 can comprise summaries of summaries that can be generated by reporting component 254, via generative AI, by summarizing and combining various parts of the medical journey of a patient. Such a report can reduce the cognitive footprint of a medical practitioner because in some situations, a physician is not aware of the various types of information available with a patient and the patient is not aware of the information that can be useful to the physician. In such situations, AI-based report 224 comprising, for example, a summarization of the medical journey of the patient during a previous clinic visit and further comprising keywords, sources of the keywords, sources for other types of data, events (e.g., two imaging scans, two blood tests, a tumor marker test, a visit to the emergency department, a visit to the endocrinology clinic, etc.) occurred since a previous clinic visit, and so on, can provide useful information to the physician. For example, oncologist *A* can titrate medication for patient *A* based on AI-based report 224.

In one or more embodiments, AI-based report 224 can generate a coarse summary and a fine summary, wherein the coarse summary can describe a patient overview and the fine summary can describe the patient overview at a finer level of detail, such as in terms of events related to the medical journey of patient *A* that may have occurred since patient *A* last visited oncologist *A.* For example, the fine summary can describe events occurring over the three weeks, one month, two months, etc. prior to a scheduled visit for patient *A* with oncologist *A.* In this manner, AI-based report 224 can allow oncologist A to prepare for each future encounter with patient *A,* and the details of each encounter between oncologist *A* and patient *A* can also be summarized by reporting component 254 in AI-based report 224. As a result, AI-based report 224 can be iteratively updated, which can allow oncologist *A* to closely monitor how patient *A* responds to each clinical decision made by oncologist A.

In general, AI-based report 224 can allow an end entity (e.g., hardware, software, machine, AI, neural network and/or user) to observe cause and effect relationships between clinical decisions and the resulting outcomes, draw specific questions, identify good clinical decisions, and even produce additional works (e.g., papers, publications, etc.). Since AI-based report 224 can also describe the basis for a clinical decision, AI-based report 224 can be employed for educational purposes, for example, by a senior clinician to educate junior clinicians. In one or more embodiments, reporting component 254 can combine textual data and visuals in AI-based report 224 to enhance the usability of AI-based report 224.

AI model 210 and AI-based report 224 can be powerful tools for clinical trials. A clinical trial can typically involve a large number of discoveries and analysis modes, and a clinical trial is bound by a defined standard of care. A clinical trial also has a wider latitude, for example, as compared to the treatment process for a single patient. For example, researchers involved in a clinical trial tend to look for known clinical effects, known responses, known interventions, correlations, causes, potential drug descriptions employable to label a drug, the percentage of hypertensive patients in a cohort, outcomes of respective patients in the cohort, etc. Accordingly, employing AI model 210 in clinical trials can allow for more discovery, for example, via AI-based report 224 and/or other results that can be generated by AI model 210.

With continued reference to FIG. 2A, FIGS. 12 - 15 describe how AI model 210 can detect decision points and outcomes of decision points in the medical journey of a patient, identify cohorts to the medical journey of the patient, and generate a ranked list of luminaries specializing in the treatment of the medical condition of the patient.

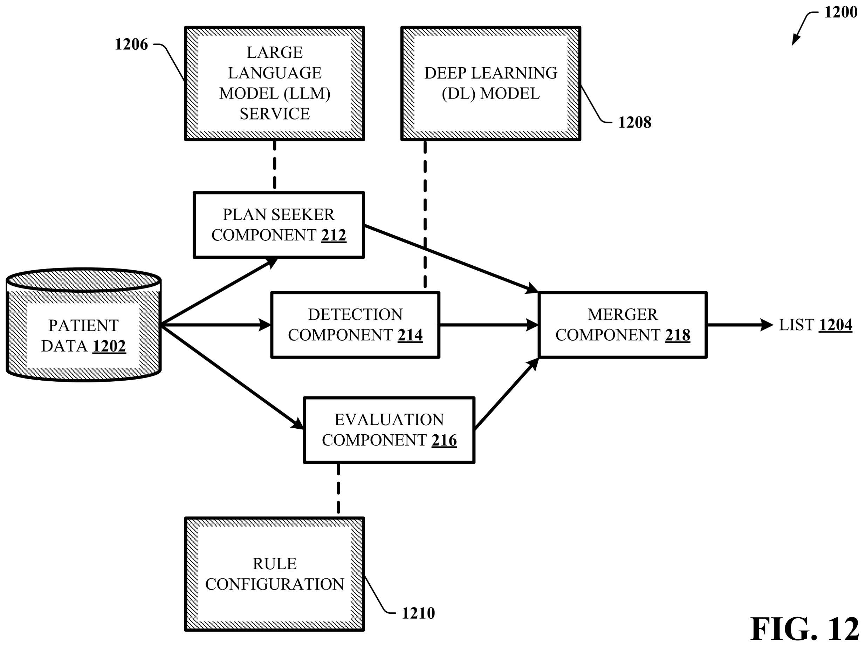

FIG. 12 illustrates a flow diagram of an example, non-limiting method 1200 that can detect decision points in the medical journey of a patient, in accordance with one or more embodiments described herein. Repetitive description of like elements and/or processes employed in respective embodiments is omitted for sake of brevity.

In one or more embodiments, first AI component 238 can detect a plurality of decision points within the temporal view displaying the decision maker specific medical journey of a patient on a patient timeline. To detect the plurality of decision points, first AI component 238 can employ an AI agentic system comprising plan seeker component 242, detection component 244, evaluation component 246 and merger component 248. For example, patient data 1202 can comprise first data 212 and second data 214. At 1206, plan seeker component 242 can employ an LLM service to identify a first set of decision points within the patient timeline, based on the determination of and/or modifications within a treatment plan associated with the medical condition of the patient. At 1208, detection component 244 can employ a deep learning (DL) model to identify a second set of decision points within the patient timeline, based on inputs associated with the events within the medical journey of the patient. The DL model can extract features from images as well as text. At 1210, evaluation component 246 can employ rule configuration to identify a third set of decision points based on a set of clinical rules, wherein the set of clinical rules can comprise rules configured to detect defined clinical patterns. Finally, merger component 248 can generate list 1204 comprising a plurality of decision points by merging the first set of decision points, the second set of decision points and the third set of decision points. As described with reference to FIG. 2A, respective decision points comprised in list 1204 can represent key decision moments or key clinical decisions made by an end entity (e.g., hardware, software, machine, AI, neural network and/or user) to treat the medical condition of the patient.

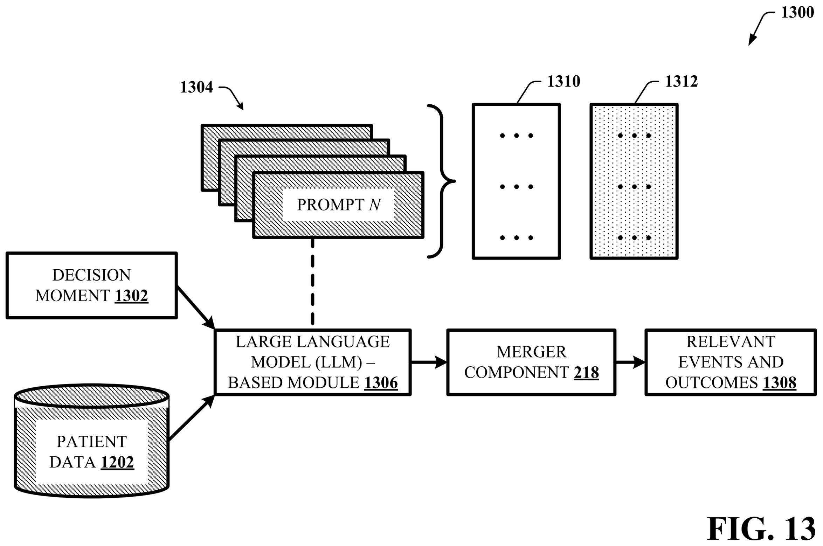

FIG. 13 illustrates a flow diagram of an example, non-limiting method 1300 that can detect events and outcomes resulting from a decision point in the medical journey of a patient, in accordance with one or more embodiments described herein. Repetitive description of like elements and/or processes employed in respective embodiments is omitted for sake of brevity.

With continued reference to FIGS. 1, 4 and 12, in one or more embodiments, second AI component 240 can detect one or more outcomes comprised in second data 214. To detect the one or more outcomes, second AI component 240 can employ an event and outcome determination system comprising selection component 250, prompt generation component 252 and merger component 248. For example, selection component 250 can select decision point 1302, wherein decision point 1302 can be detected by first AI component 238 on the patient timeline. For example, decision point 1302 can represent a first chemotherapy event (chemo1), a second chemotherapy event (chemo2), a surgery, a hormone test, adjuvant radiotherapy (adj RT), etc. Thereafter, at 1304, prompt generation component 252 can generate, based on decision point 1302 and patient data 1202, a set of summarization prompts to identify a set of events representing consequences of the clinical decision. For example, prompt generation component 252 can generate the summarization prompts to down-select data based on criteria such as patient parameters (block 1310) and drug indications (block 1312) for each decision moment. Prompt generation component 252 can input the set of summarization prompts to an LLM (e.g., LLM-based module 1306), and merger component 248 can analyze results generated by the LLM upon processing the set of summarization prompts to detect relevant events and outcomes 1308 resulting from decision point 1302. Second AI component 240 can detect respective sets of outcomes for respective decision points in a similar manner.

In one or more embodiments, prompt generation component 252 can also employ a Computer-Aided Diagnosis (CAD) model to generate the summarization prompts. For example, the CAD model can access a knowledge source, such as decision point 1302, and a medical context related to the patient, such as patient data 1202, and auto-generate questions directed to specific outcomes. The questions can be the summarization prompts that can be processed by LLM-based module 1306 to generate responses to the questions. In one or more embodiments, LLM-based module 1306 can be prompted by the summarization prompts generated by prompt generation component 252 as well and by prompts generated by an end entity (e.g., hardware, software, machine, AI, neural network and/or user). For example, prompt generation component 252 can auto-generate some summarization prompts, and the UI (e.g., non-limiting UI 400) can comprise a text box where oncologist *A* can manually enter additional questions to prompt LLM-based module 1306. Prompt generation component 252 can access the questions from oncologist *A* and prompt LLM-based module 1306 with the auto-generated summarization prompts and the questions input by oncologist A, while remaining within the confines of the knowledge base and the medical context related to patient *A.* Such a functionality can allow the end entity (e.g., hardware, software, machine, AI, neural network and/or user) to arrive at a desirable end point in terms of the responses generated by LLM-based module 1306.

In some situations, the treatment for a patient can become palliative, that is, the aggression level of a treatment previously adopted for the patient can change. In such situations, the provision of the text box can allow the end entity (e.g., hardware, software, machine, AI, neural network and/or user) to switch the treatment plan to palliative care. For example, a physician can upload new guideline documents, a paper, a publication, additional knowledge source and/or other data via the text box and prompt LLM-based module 1306 to determine how the physician can switch the treatment plan for a patient to a treatment plan suitable for palliative care.

Thus, in one or more embodiments, first AI component 238 and second AI component 240 can enhance the clinical insights provided to a medical practitioner by AI model 210 by identifying the causes and effects of parameters related to the medical condition of a patient, wherein the parameters can be continuously monitored. The enhanced clinical insights can assist the medical practitioner in providing improved healthcare to the patient by reviewing the consequences of clinical decisions and subsequently making better clinical decisions.

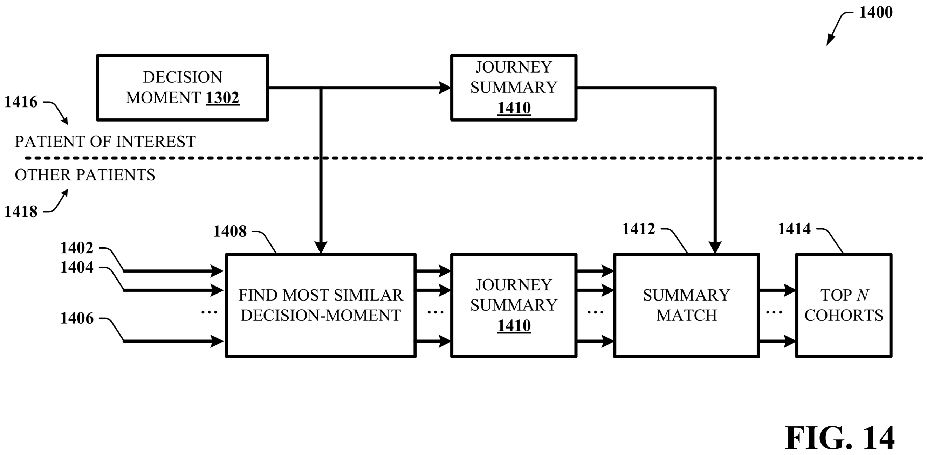

FIG. 14 illustrates a flow diagram of an example, non-limiting method 1400 that can identify cohorts to the medical journey of a patient, in accordance with one or more embodiments described herein. Repetitive description of like elements and/or processes employed in respective embodiments is omitted for sake of brevity.

In one or more embodiments, data generation component 234 can identify, via generative AI, cohorts 216 corresponding to a patient of interest. To identify cohorts 216, data generation component 234 can summarize, at 1416, the medical journey of the patient prior to decision point 1302 and generate journey summary 1410 based on the summarization.

Thereafter, data generation component 234 can compare journey summary 1410 to respective medical journeys of respective patients of one or more additional patients, for example, patients in the same medical facility responsible for treating the patient. For example, at 1418, data generation component 234 can access second patient data comprising data related to the one or more additional patients, and at 1408, data generation component 234 can identify decision points (i.e., key decision moments) comprised in the second patient data that are most similar to decision point 1302 (i.e., most-similar decision points). In this regard, the arrow at 1402 can represent a first decision point (e.g., Pt. #1) comprised in the second patient data, the arrow at 1404 can represent a second decision point (e.g., Pt. #2) comprised in the second patient data, the arrow at 1406 can represent an *M*^{th} decision point (e.g., Pt. #M) comprised in the second patient data, and so on, wherein *M* represents a positive integer. One or more of the decision points comprised in the second patient data can be most similar to decision point 1302.

At 1412, data generation component 234 can perform a summary match, wherein data generation component 234 can compare journey summary 1410 with the most-similar decision points. At 1414, data generation component 234 can select, based on the summary match, a set of patients as cohorts to the patient. Specifically, data generation component 234 can select a set of medical journeys from the second patient data as the top *N* cohorts to the medical journey of the patient, wherein *N* represents a positive integer. The top *N* cohorts can comprise cohorts 216.

In one or more embodiments, data generation component 234 can employ existing cohort algorithms and/or systems or decision-making-based algorithms specific to a disease to identify cohorts 216. For example, to identify cohorts 216 for a cancer patient, data generation component 234 can employ a cancer-specific data model to perform the summary match based on the type and subtype of cancer, the age, the gender, the weight, the molecular profile of the patient, the tumor, node, metastasis (TNM) stage of the cancer, neo adjuvant (Neo Adj) therapies, adjuvant (Adj) therapies, 1^{st} line, 2^{nd} line, co-morbidities and allergies of the patient, etc.

In some situations, data generation component 234 can factor new disease outbreaks in identifying cohorts 216. For example, most cancer patients are generally immune suppressed and possess high risks for infections, including infections acquired at a medical facility. An infection can have a profound influence on a cancer patient. For example, any infection can further weaken the immune system of a cancer patient and distract the immune system of the cancer patient from the cancer treatment, for example, due to drugs administered to the cancer patient to treat the infection. If a cancer patient becomes infected with a fairly new disease (e.g., a week-old disease), new data can become available for the cancer patient on the patient timeline (e.g., non-limiting patient timeline 510 of FIG. 5). For example, the new data can become available in the form of events such as tests for the infection, treatments, etc. In one or more embodiments, data generation component 234 can employ generative AI to determine whether the new data for the cancer patient comprises enough information to identify cohorts 216 since the infection is fairly new. If the new data does not comprise enough information, data generation component 234 can exclude/filter out the new data when identifying cohorts 216 until enough decision points related to the infection become available for the cancer patient.

In one or more embodiments, the cancer patient can be associated with a cohort, and data generation component 234 can continuously update the cohort as additional decision points become available in the cancer-specific medical journey of the cancer patient. For example, at certain times, data generation component 234 can include the medical journey of the cancer patient in a large cohort, and at other times, data generation component 234 can include the medical journey of the cancer patient in a small cohort. In such situations, if any of the patients associated with a cohort develop the infection, data generation component 234 can apply a new disease code to the patients and flag/alert the concerned oncologists about the new disease code. As a result, the concerned oncologists can monitor the infected patients' conditions to determine whether the infection is prevalent and can potentially spread to other patients associated with the cohort because some aspects of the cancer patients associated with the cohort can be immune suppressed, making the cancer patients predisposed to the infection. Stated differently, in one or more embodiments, data generation component 234 can factor new disease outbreaks and alert the concerned oncologists only if the patients they are responsible for treating are in a group in which other patients are infected with the new disease. Since cohorts can be highly matched with several shared characteristics among the patients associated with the cohort, the oncologist can benefit from being alerted by data generation component 234 if one or more patients associated with the cohort acquire the infection. For example, the oncologist can try to determine why the one or more patients acquired the infection, whether the infection is an isolated issue or an immune modulated situation, how the infection can be prevented from spreading to other patients associated with the cohort, etc.

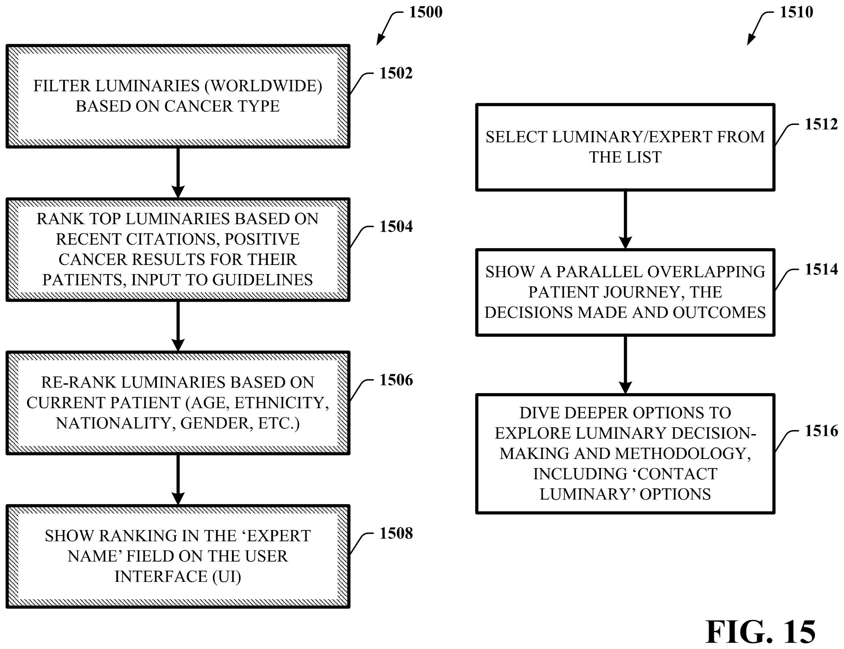

FIG. 15 illustrates flow diagrams of example, non-limiting methods 1500 and 1510 that can generate a ranked list of medical practitioners specializing in the treatment of a medical condition and generate additional data corresponding to respective medical practitioners in the ranked list, in accordance with one or more embodiments described herein.

In one or more embodiments, data generation component 234 can generate, based on the first patient data and the treatment information comprised in first data 212, ranked list 220, wherein ranked list 220 can be employable by an end entity (e.g., hardware, software, machine, AI, neural network and/or user) to explore treatment plans (e.g., second treatment plans) employed by medical practitioners (e.g., luminaries or experts) specializing in the treatment of the medical condition of a patient, explore outcomes (e.g., second outcomes) associated with the treatment plans, and contact the medical practitioners.

In one or more embodiments, non-limiting method 1500 can be employed by data generation component 234 to generate ranked list 220 by listing expert names and ranking the experts. For example, at 1502, data generation component 234 can perform a global search to find luminaries based on the type of cancer that a patient has. For example, data generation component 234 can identify the potential experts in oncology that frequently publish research papers, are frequently approached for treatments, etc. from online sources, digital libraries, etc. For example, for certain cancers or diagnoses, a very clear list of luminaries can sometimes be accessed from online sources.

At 1504, data generation component 234 can rank top luminaries based on recent citations of the works of the luminaries, positive cancer results for patients of the luminaries, and inputs to guidelines provided by a physician responsible for treating the patient. Additionally, as end users compare their decision maker views on the patient timeline with other luminaries, data generation component 234 can identify the luminaries that are selected by the end users more frequently (e.g., more number of times than other luminaries) and suggest those luminaries more prominently, for example, by listing the names of those luminaries higher up within ranked list 220. Further, surveys that clinicians fill out can also identify who the top luminaries in the medical field for a particular disease are. For example, third parties (e.g., agencies), independent groups (e.g., medical professional associations), and even government entities (e.g., The Department of Health) may have preferences or rankings for top medical professionals for certain diseases and patient conditions. In one or more embodiments, data generation component 234 can employ a rules engine or an AI model to identify top luminaries based on the factors listed herein and/or other factors. The ranking employed by data generation component 234 can be similar to how magazines can rank top lawyers for different legal fields or to how awards can be given to the best professionals in a certain career. At 1506, data generation component 234 can re-rank the luminaries based on the age, the gender, the ethnicity, etc. of a patient to generate ranked list 220. At 1508, display component 236 can display the ranking in the "Expert name" field on the UI.

In some embodiments, the names of the patients treated by the luminary can be hidden from the physician by display component 236 due to legal and privacy reasons, for example, if the patients are from a different medical facility than the one where the patient is being treated. In other embodiments, the names of the patients can be displayed to the physician by display component 236, for example, if the patients are from the same medical facility where the patient is being treated. In one or more embodiments, the names of medical professionals may also be hidden. For example, the name of a medical professional can be hidden depending on the country of residence, geographic reasons and/or legal reasons.

Non-limiting method 1510 can describe a process that can be executed (e.g., by display component 236) when the physician selects the name of a luminary on the UI via a cursor, touchscreen or other suitable functionality. For example, at 1512, the physician can select the name of a luminary from ranked list 220. In some embodiments, the name of the luminary can be automatically selected by display component 236 based on audio instructions received by display component 236 from the physician such as, for example, via the microphone of a device. At 1514, display component 236 can display a parallel view of overlapping medical journeys of patients treated by the selected luminary alongside the medical journey of the patient, on the UI. At 1516, display component 236 can display options on the UI that can allow the physician to dive deeper (e.g., "Dive Deeper" options) to explore the decision-making processes, methodologies, etc. employed by the luminary and to contact the luminary. The "Dive Deeper" options can include options to read notes, EMR records, or other information that the luminary may have specifically entered within the medical record of a different patient, a research paper or a scientific paper authored by the luminary, wherein the research paper or the scientific paper can be related to the clinical decisions being made by the luminary at the time of authoring and to a patient state (e.g., a disease state, gender, age, and/or other factors), and so on.

Ranked list 220 can provide an efficient method of analyzing alternate treatment plans to the physician, for example, as opposed to attending a conference or other elaborate methods. In one or more embodiments, the physician can contact the luminary and the luminary can describe a particular case or methodology in greater detail, or the physician can forward to the luminary, a paragraph worth of a snippet related to the patient along with one or more questions, over a secure chat or communication line. Thus, the physician can access offline, multidisciplinary inputs from different luminaries (e.g., an offline tumor board) based on ranked list 220.

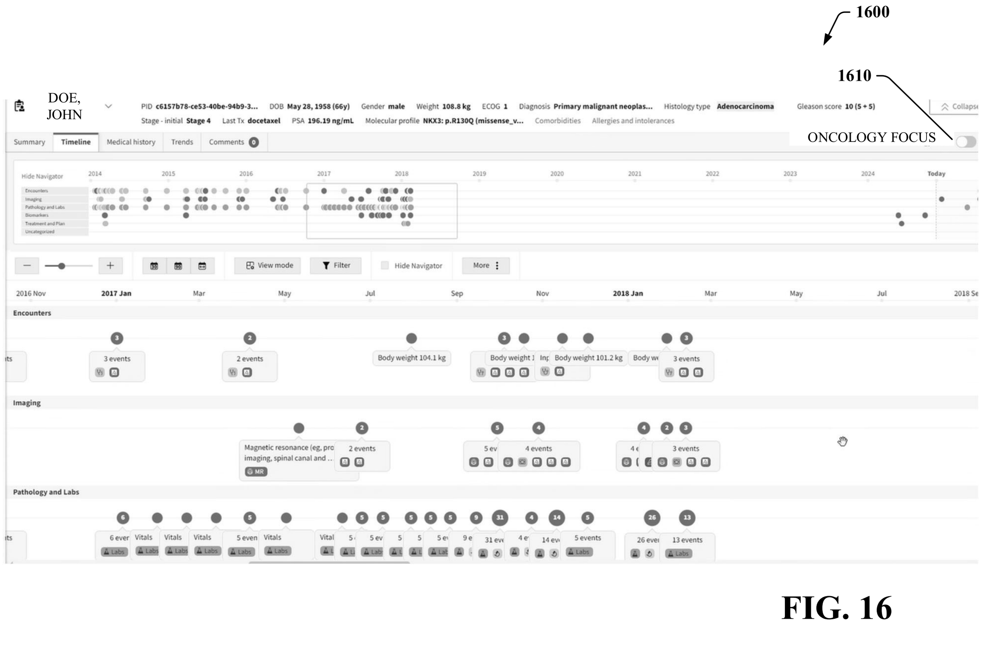

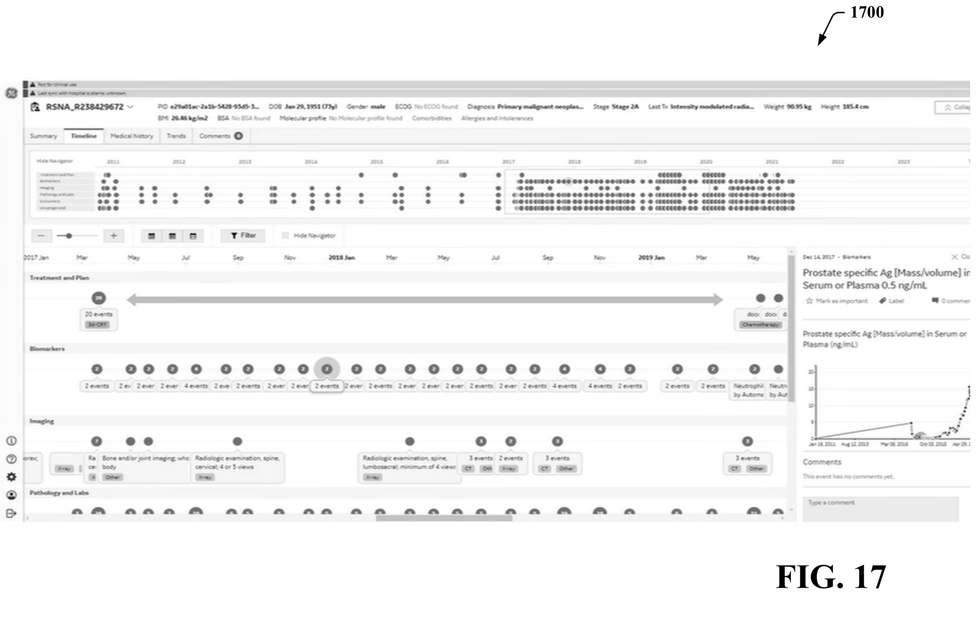

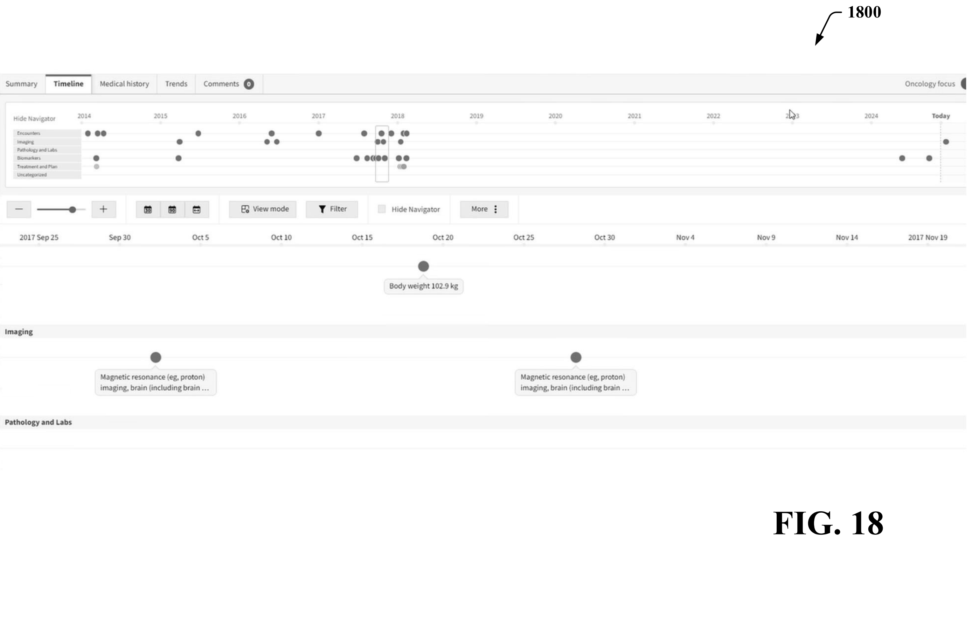

FIGS. 16 and 17 illustrate diagrams of example, non-limiting UIs 1600 and 1700, respectively, that can display the medical journey of a patient, in accordance with one or more embodiments described herein. FIG. 18 illustrates a diagram of an example, non-limiting UI 1800 that can display the decision maker specific view of the medical journey of a patient, in accordance with one or more embodiments described herein. Repetitive description of like elements and/or processes employed in respective embodiments is omitted for sake of brevity.

Non-limiting UI 1600 and non-limiting UI 1700 can be analogous to non-limiting UI 300 of FIG. 3. Toggle switch 1610 can allow an end entity (e.g., hardware, software, machine, AI, neural network and/or user) to transition from the default view of the medical journey of a patient illustrated in FIGS. 16 and 17 to the cancer-specific view (e.g., "Oncology focus") illustrated in FIG. 18. For example, non-limiting UI 1800 can display the portions of patient *A'*s medical journey that are focused on oncology-related events and clinical decisions. Evidently, the default view can display numerous data points which can become confusing for oncologist *A* concerned only with the cancer treatment of patient *A.* On the contrary, the cancer-specific view can provide an oncology focused user experience (UX) by zooming into the most helpful aspects of the cancer treatment and assist oncologist *A* in learning from past clinical decisions and making more informed clinical decisions in the future.

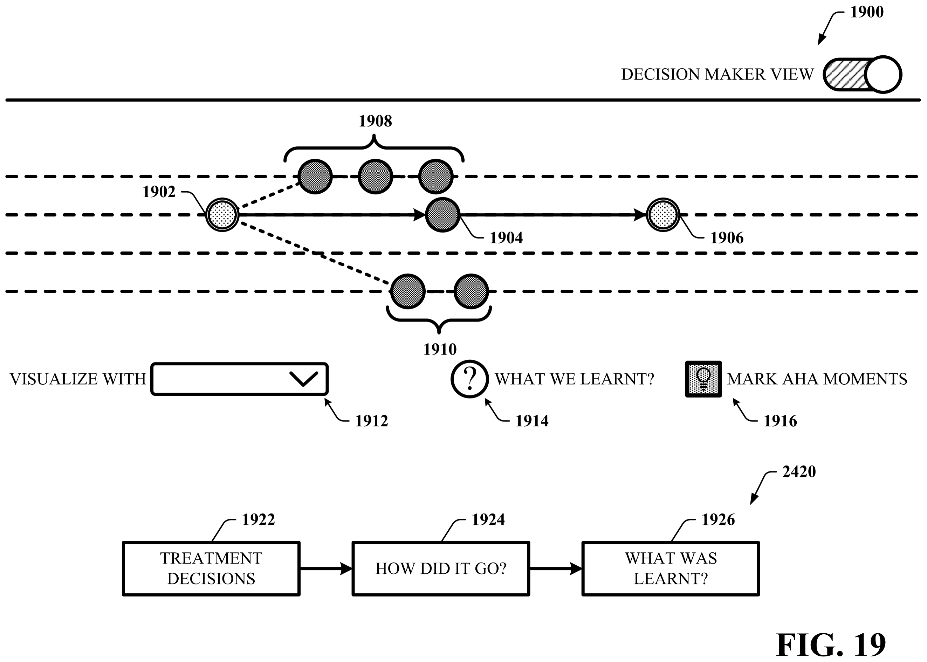

FIG. 19 illustrates a diagram of an example, non-limiting UI 1900 that can display a cancer-specific view of the medical journey of a patient, in accordance with one or more embodiments described herein. Repetitive description of like elements and/or processes employed in respective embodiments is omitted for sake of brevity.

With continued reference to at least FIGS. 1 and 18, non-limiting UI 1900 is intended to illustrate additional aspects of non-limiting UI 1800. In one or more embodiments, event 1902 can be a decision point detected by first AI component 238, and event 1904 can be the outcome of event 1902, wherein event 1904 can be detected by second AI component 240. Events 1904 and 1906 can describe the main progression of event 1902. For example, event 1904 can describe whether patient *A* adhered to the clinical decision of event 1902, and event 1906 can describe another clinical decision made by oncologist *A* for patient *A,* based on event 1904. In this regard, FIG. 19 further illustrates a diagram of non-limiting process 1920 that oncologist *A* can employ to the progression of evaluate event 1902. For example, at 1922, oncologist *A* can evaluate the clinical decision associated with event 1902, at 1924, oncologist *A* can evaluate, based on event 1904, how the clinical decision progressed (e.g., whether patient *A* adhered to the clinical decision), and at 1926, oncologist A can identify valuable learnings based on events 1902 and 1904. Events 1908 and events 1910 can represent clinical decisions associated with the medical journeys of other patients (e.g., cohorts 216), clinical decisions made by luminaries (e.g., cancer specialists) for their patients, clinical decisions from medical studies performed in the past, etc. For example, events 1908 can represent clinical decisions associated with a first medical journey comprised in cohorts 216, and events 1908 can represent clinical decisions associated with a second medical journey comprised in cohorts 216. Events 1908 and events 1910 can be displayed by display component 236 on non-limiting UI 1900 via branching paths, as illustrated in FIG. 19, wherein the branching paths can be generated by suitable algorithms. In general, display component 236 can display, on UI 1900, respective medical journeys of respective patients comprised in cohorts 216 via branching paths that branch from a decision point associated with the medical journey of a patient, such that the branching paths highlight an alternative progression of the medical journey of the patient resulting from an alternative clinical decision as opposed to the clinical decision associated with the decision point. Events 1908 and events 1910 can assist oncologist *A* to view how the medical journey for patient *A* could have progressed differently, had oncologist *A* made a different clinical decision.

As described elsewhere herein, display component 236 can visually distinguish decision points from other points on the patient timeline, for example, by making the decision points larger than other points, applying different colors to the decision points versus the other points, or otherwise emphasizing the decision points. Thus, UI 1900 can show the actual past paths of the patient timeline alongside other potential paths that the decision maker could have taken. In FIG. 19, the solid line can highlight the medical journey of a patient based on the clinical decisions made by a decision maker, and the dashed line can highlight other potential clinical decisions and medical journey options.

As described elsewhere herein, drop-down menu 1912 can be employed by an end entity (e.g., hardware, software, machine, AI, neural network and/or user) to generate visualization 222, view ranked list 220, compare the medical journeys of patients with studies conducted in the past, view guidelines from institutions, etc. Additionally, the "What we learnt" button at 1914 can be selected by the end entity (e.g., hardware, software, machine, AI, neural network and/or user) to view AI-based report 224, and the "Mark Aha moment" button at 1916 can be selected by the end entity (e.g., hardware, software, machine, AI, neural network and/or user) to mark valuable decision points as aha moments for future reference. As described with reference to FIG. 2A, display component 236 can function as an interactive module that can enable an end entity (e.g., hardware, software, machine, AI, neural network and/or user) to select options, decision points, drop-down menus, etc. on the UI via a cursor, touchscreen, voice commands, or other suitable functionality. In one or more embodiments, a generative AI model employed by first AI component 238 can automatically suggest prospective aha moments to the end entity (e.g., hardware, software, machine, AI, neural network and/or user).

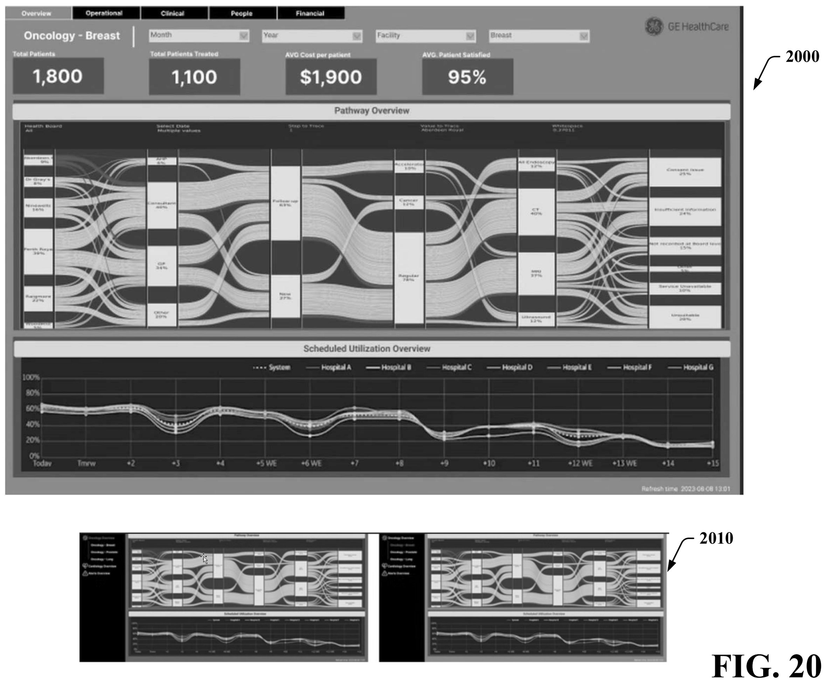

FIG. 20 illustrates diagrams of example, non-limiting cohort analysis visuals 2000 and 2010, in accordance with one or more embodiments described herein. Repetitive description of like elements and/or processes employed in respective embodiments is omitted for sake of brevity.

Non-limiting cohort analysis visual 2000 shows a cohort screen which is also shown in non-limiting cohort analysis visual 2010 alongside a second cohort screen. Such cohort screens can show information about multiple patients, for example, how patient behavior can move outside of standard expected behavior. With continued reference to FIG. 2A, in one or more embodiments, display component 236 can display cohort screens similar to the cohort screens shown in non-limiting cohort analysis visuals 2000 and 2010 on the UI, for example, as a pop-up screen or window that can be employed by an end entity (e.g., hardware, software, machine, AI, neural network and/or user) to determine the normal behavior of patients when comparing the medical journey of a patient with oncology guidelines or with healthcare outcomes of other patients treated by various luminaries.

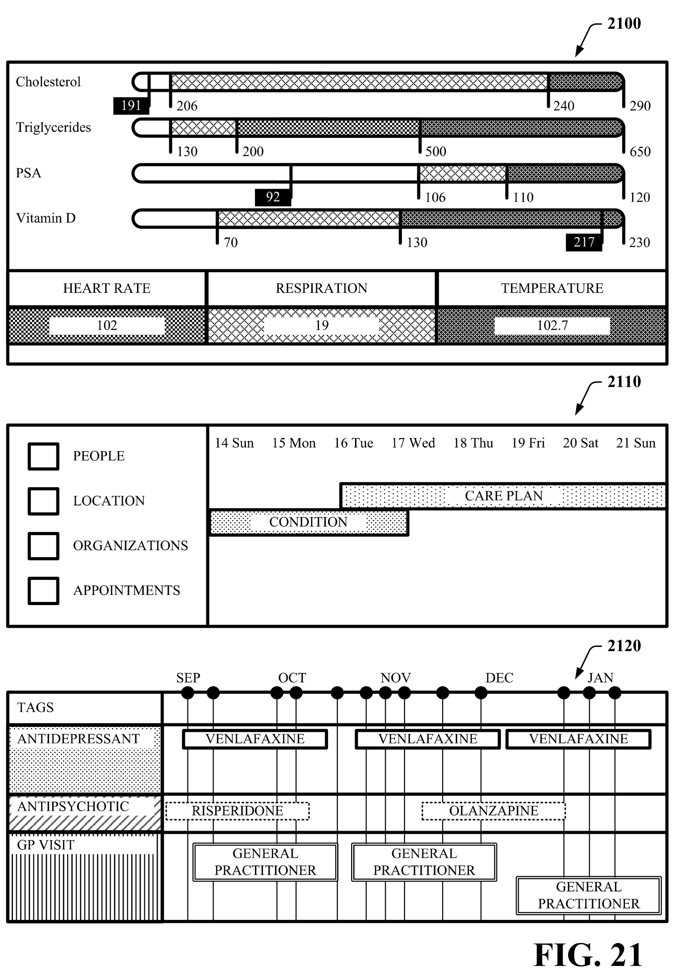

FIG. 21 illustrates diagrams of example, non-limiting patient timelines 2100, 2110 and 2120 that can display aspects of the medical journey of a patient. Repetitive description of like elements and/or processes employed in respective embodiments is omitted for sake of brevity.

Non-limiting patient timeline 2100, non-limiting patient timeline 2110 and non-limiting patient timeline 2120 are intended to illustrate patient timelines presented by existing software. Evidently, non-limiting patient timelines 2100, 2110 and 2120 do not show decision maker specific views or toggle switches, such as those presented by the embodiments of the present disclosure, to assist a physician or another end entity (e.g., hardware, software, machine, AI, neural network and/or user) in switching from a default view to a more focused view of a patient's medical journey.

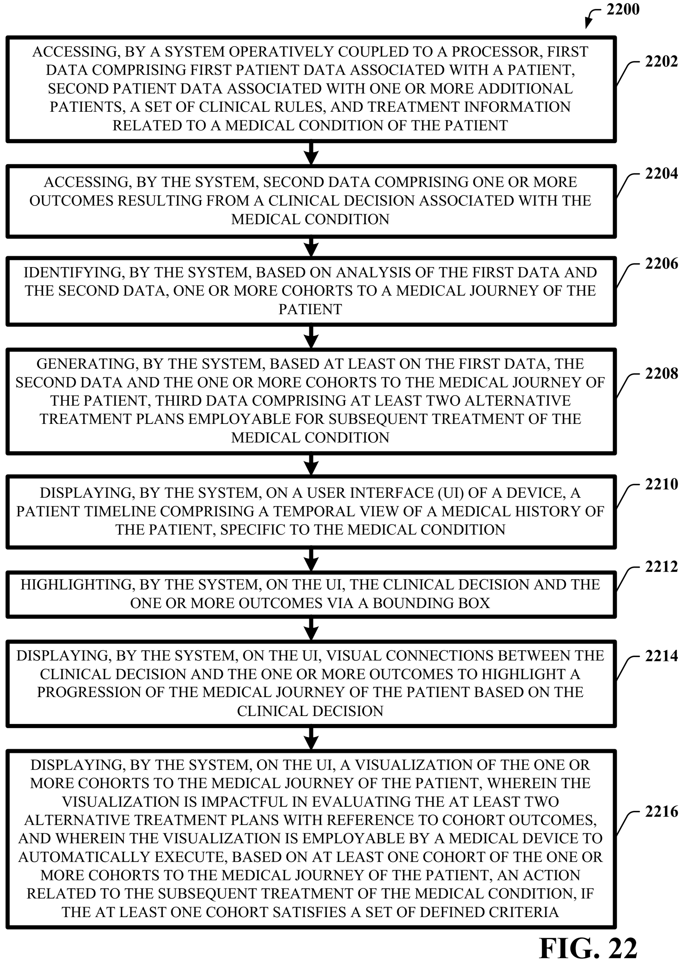

FIG. 22 illustrates a flow diagram of an example, non-limiting method 2200 that can present a decision maker specific visualization of the medical journey of a patient and generate alternative treatment plans employable for subsequent treatment of a disease, in accordance with one or more embodiments described herein. Repetitive description of like elements and/or processes employed in respective embodiments is omitted for sake of brevity.

At 2202, non-limiting method 2200 can comprise accessing (e.g., by data access component 232), by a system operatively coupled to a processor, first data comprising first patient data associated with a patient, second patient data associated with one or more additional patients, a set of clinical rules, and treatment information related to a medical condition of the patient.

At 2204, non-limiting method 2200 can comprise accessing (e.g., by data access component 232), by the system, second data comprising one or more outcomes resulting from a clinical decision associated with the medical condition.

At 2206, non-limiting method 2200 can comprise identifying (e.g., by data generation component 234), by the system, based on analysis of the first data and the second data, one or more cohorts to a medical journey of the patient.

At 2208, non-limiting method 2200 can comprise generating (e.g., by data generation component 234), by the system, based at least on the first data, the second data and the one or more cohorts to the medical journey of the patient, third data comprising at least two alternative treatment plans employable for subsequent treatment of the medical condition.

At 2210, non-limiting method 2200 can comprise displaying (e.g., by display component 236), by the system, on a UI of a device, a patient timeline comprising a temporal view of a medical history of the patient, specific to the medical condition.

At 2212, non-limiting method 2200 can comprise highlighting (e.g., by display component 236), by the system, on the UI, the clinical decision and the one or more outcomes via a bounding box.

At 2214, non-limiting method 2200 can comprise displaying (e.g., by display component 236), by the system, on the UI, visual connections between the clinical decision and the one or more outcomes to highlight a progression of the medical journey of the patient based on the clinical decision.

At 2216, non-limiting method 2200 can comprise displaying, (e.g., by display component 236), by the system, on the UI, a visualization of the one or more cohorts to the medical journey of the patient, wherein the visualization is impactful in evaluating the at least two alternative treatment plans with reference to cohort outcomes, and wherein the visualization is employable by a medical device to automatically execute, based on at least one cohort of the one or more cohorts to the medical journey of the patient, an action related to the subsequent treatment of the medical condition, if the at least one cohort satisfies a set of defined criteria.

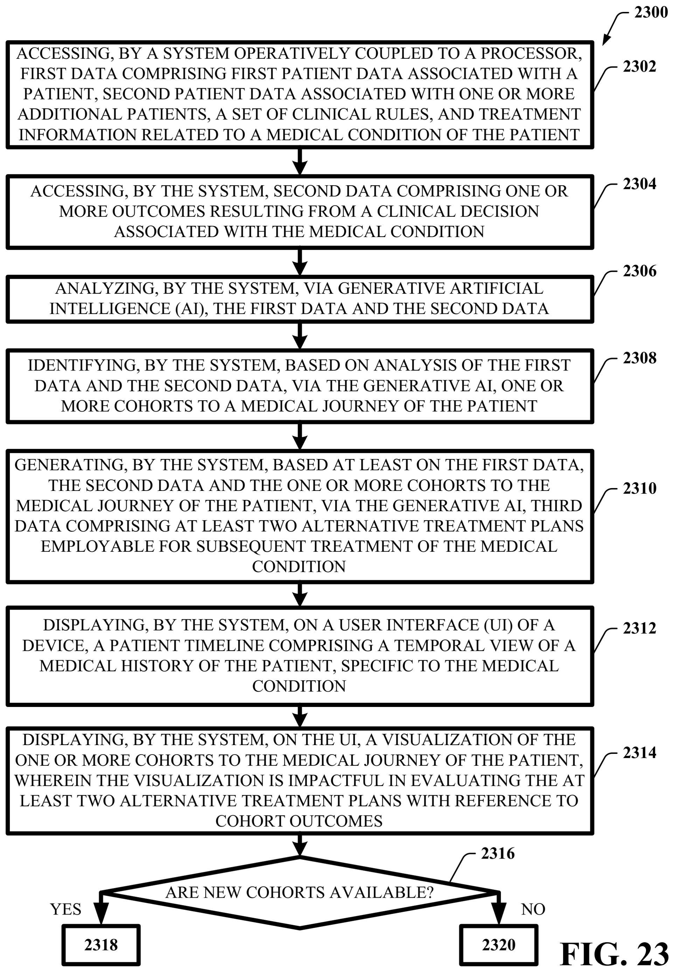

FIG. 23 illustrates a flow diagram of an example, non-limiting method 2300 that can employ generative AI to present a decision maker specific visualization of the medical journey of a patient and generate alternative treatment plans employable for subsequent treatment of a disease, in accordance with one or more embodiments described herein. Repetitive description of like elements and/or processes employed in respective embodiments is omitted for sake of brevity.

At 2302, non-limiting method 2300 can comprise accessing (e.g., by data access component 232), by a system operatively coupled to a processor, first data comprising first patient data associated with a patient, second patient data associated with one or more additional patients, a set of clinical rules, and treatment information related to a medical condition of the patient.

At 2304, non-limiting method 2300 can comprise accessing (e.g., by data access component 232), by the system, second data comprising one or more outcomes resulting from a clinical decision associated with the medical condition.

At 2306, non-limiting method 2300 can comprise analyzing (e.g., by data generation component 234), by the system, via generative AI, the first data and the second data.

At 2308, non-limiting method 2300 can comprise identifying (e.g., by data generation component 234), by the system, based on analysis of the first data and the second data, via the generative AI, one or more cohorts to a medical journey of the patient.

At 2310, non-limiting method 2300 can comprise generating (e.g., by data generation component 234), by the system, based at least on the first data, the second data and the one or more cohorts to the medical journey of the patient, via the generative AI, third data comprising at least two alternative treatment plans employable for subsequent treatment of the medical condition.

At 2312, non-limiting method 2300 can comprise displaying (e.g., by display component 236), by the system, on a UI of a device, a patient timeline comprising a temporal view of a medical history of the patient, specific to the medical condition.

At 2314, non-limiting method 2300 can comprise displaying (e.g., by display component 236), by the system, on the UI, a visualization of the one or more cohorts to the medical journey of the patient, wherein the visualization is impactful in evaluating the at least two alternative treatment plans with reference to cohort outcomes.

At 2316, non-limiting method 2300 can comprise determining, (e.g., by display component 236), by the system, whether new cohorts (i.e., cohorts to the medical journey of the patient) have been identified/are available.

If yes, then at 2318, non-limiting method 2300 can comprise updating (e.g., by display component 236), by the system, the visualization to include the new cohorts.

If not, then at 2320, non-limiting method 2300 can comprise monitoring (e.g., by display component 236), by the system, for new cohorts.

In various instances, machine learning algorithms or models can be implemented in any suitable way to facilitate any suitable aspects described herein. To facilitate some of the above-described machine learning aspects of various embodiments, consider the following discussion of artificial intelligence (AI). Various embodiments described herein can employ AI to facilitate automating one or more features or functionalities. The components can employ various AI-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (z₁, z₂, z₃, z₄, *zₙ*), to a confidence that the input belongs to a class, as by f(z) = *confidence*(*class*)*.* Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naive Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

In order to provide additional context for various embodiments described herein, FIG. 24 and the following discussion are intended to provide a brief, general description of a suitable computing environment 2400 in which the various embodiments described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 24, the example environment 2400 for implementing various embodiments of the aspects described herein includes a computer 2402, the computer 2402 including a processing unit 2404, a system memory 2406 and a system bus 2408. The system bus 2408 couples system components including, but not limited to, the system memory 2406 to the processing unit 2404. The processing unit 2404 can be any of various commercially available processors. Dual microprocessors and other multi-processor architectures can also be employed as the processing unit 2404.

The system bus 2408 can be any of several types of bus structure that can further interconnect to a memory bus (with or without a memory controller), a peripheral bus, and a local bus using any of a variety of commercially available bus architectures. The system memory 2406 includes ROM 2410 and RAM 2412. A basic input/output system (BIOS) can be stored in a non-volatile memory such as ROM, erasable programmable read only memory (EPROM), EEPROM, which BIOS contains the basic routines that help to transfer information between elements within the computer 2402, such as during startup. The RAM 2412 can also include a high-speed RAM such as static RAM for caching data.

The computer 2402 further includes an internal hard disk drive (HDD) 2414 (e.g., EIDE, SATA), one or more external storage devices 2416 (e.g., a magnetic floppy disk drive (FDD) 2416, a memory stick or flash drive reader, a memory card reader, etc.) and a drive 2420, e.g., such as a solid state drive, an optical disk drive, which can read or write from a disk 2422, such as a CD-ROM disc, a DVD, a BD, etc. Alternatively, where a solid state drive is involved, disk 2422 would not be included, unless separate. While the internal HDD 2414 is illustrated as located within the computer 2402, the internal HDD 2414 can also be configured for external use in a suitable chassis (not shown). Additionally, while not shown in environment 2400, a solid state drive (SSD) could be used in addition to, or in place of, an HDD 2414. The HDD 2414, external storage device(s) 2416 and drive 2420 can be connected to the system bus 2408 by an HDD interface 2424, an external storage interface 2426 and a drive interface 2428, respectively. The interface 2424 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 2402, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 2412, including an operating system 2430, one or more application programs 2432, other program modules 2434 and program data 2436. All or portions of the operating system, applications, modules, or data can also be cached in the RAM 2412. The systems and methods described herein can be implemented utilizing various commercially available operating systems or combinations of operating systems.

Computer 2402 can optionally comprise emulation technologies. For example, a hypervisor (not shown) or other intermediary can emulate a hardware environment for operating system 2430, and the emulated hardware can optionally be different from the hardware illustrated in FIG. 24. In such an embodiment, operating system 2430 can comprise one virtual machine (VM) of multiple VMs hosted at computer 2402. Furthermore, operating system 2430 can provide runtime environments, such as the Java runtime environment or the .NET framework, for applications 2432. Runtime environments are consistent execution environments that allow applications 2432 to run on any operating system that includes the runtime environment. Similarly, operating system 2430 can support containers, and applications 2432 can be in the form of containers, which are lightweight, standalone, executable packages of software that include, e.g., code, runtime, system tools, system libraries and settings for an application.

A user can enter commands and information into the computer 2402 through one or more wired/wireless input devices, e.g., a keyboard 2438, a touch screen 2440, and a pointing device, such as a mouse 2442. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 2404 through an input device interface 2444 that can be coupled to the system bus 2408, but can be connected by other interfaces, such as a parallel port, an IEEE 1394 serial port, a game port, a USB port, an IR interface, a BLUETOOTH^{®} interface, etc.

A monitor 2446 or other type of display device can be also connected to the system bus 2408 via an interface, such as a video adapter 2448. In addition to the monitor 2446, a computer typically includes other peripheral output devices (not shown), such as speakers, printers, etc.

The computer 2402 can operate in a networked environment using logical connections via wired or wireless communications to one or more remote computers, such as a remote computer(s) 2450. The remote computer(s) 2450 can be a workstation, a server computer, a router, a personal computer, portable computer, microprocessor-based entertainment appliance, a peer device or other common network node, and typically includes many or all of the elements described relative to the computer 2402, although, for purposes of brevity, only a memory/storage device 2452 is illustrated. The logical connections depicted include wired/wireless connectivity to a local area network (LAN) 2454 or larger networks, e.g., a wide area network (WAN) 2456. Such LAN and WAN networking environments are commonplace in offices and companies, and facilitate enterprise-wide computer networks, such as intranets, all of which can connect to a global communications network, e.g., the Internet.

When used in a LAN networking environment, the computer 2402 can be connected to the local network 2454 through a wired or wireless communication network interface or adapter 2458. The adapter 2458 can facilitate wired or wireless communication to the LAN 2454, which can also include a wireless access point (AP) disposed thereon for communicating with the adapter 2458 in a wireless mode.

When used in a WAN networking environment, the computer 2402 can include a modem 2460 or can be connected to a communications server on the WAN 2456 via other means for establishing communications over the WAN 2456, such as by way of the Internet. The modem 2460, which can be internal or external and a wired or wireless device, can be connected to the system bus 2408 via the input device interface 2444. In a networked environment, program modules depicted relative to the computer 2402 or portions thereof, can be stored in the remote memory/storage device 2452. It will be appreciated that the network connections shown are examples and other means of establishing a communications link between the computers can be used.

When used in either a LAN or WAN networking environment, the computer 2402 can access cloud storage systems or other network-based storage systems in addition to, or in place of, external storage devices 2416 as described above, such as but not limited to a network virtual machine providing one or more aspects of storage or processing of information. Generally, a connection between the computer 2402 and a cloud storage system can be established over a LAN 2454 or WAN 2456 e.g., by the adapter 2458 or modem 2460, respectively. Upon connecting the computer 2402 to an associated cloud storage system, the external storage interface 2426 can, with the aid of the adapter 2458 or modem 2460, manage storage provided by the cloud storage system as it would other types of external storage. For instance, the external storage interface 2426 can be configured to provide access to cloud storage sources as if those sources were physically connected to the computer 2402.

The computer 2402 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

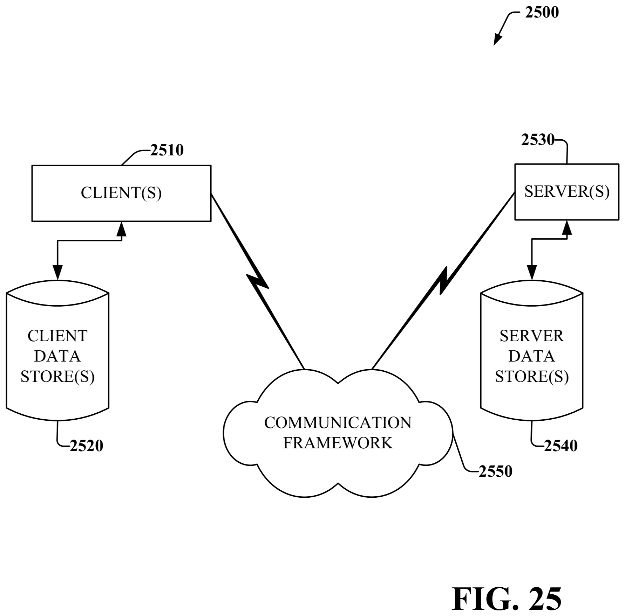

FIG. 25 is a schematic block diagram of a sample computing environment 2500 with which the disclosed subject matter can interact. The sample computing environment 2500 includes one or more client(s) 2510. The client(s) 2510 can be hardware or software (e.g., threads, processes, computing devices). The sample computing environment 2500 also includes one or more server(s) 2530. The server(s) 2530 can also be hardware or software (e.g., threads, processes, computing devices). The servers 2530 can house threads to perform transformations by employing one or more embodiments as described herein, for example. One possible communication between a client 2510 and a server 2530 can be in the form of a data packet adapted to be transmitted between two or more computer processes. The sample computing environment 2500 includes a communication framework 2550 that can be employed to facilitate communications between the client(s) 2510 and the server(s) 2530. The client(s) 2510 are operably connected to one or more client data store(s) 2520 that can be employed to store information local to the client(s) 2510. Similarly, the server(s) 2530 are operably connected to one or more server data store(s) 2540 that can be employed to store information local to the servers 2530.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A system, comprising:
a memory (206) that stores computer-executable components; and
a processor (204) that executes the computer-executable components stored in the memory (206), wherein the computer-executable components comprise:
a data access component (232) that:
accesses first data (212) comprising first patient data associated with a patient, second patient data associated with one or more additional patients, a set of clinical rules, and treatment information related to a medical condition of the patient, and
accesses second data (214) comprising one or more outcomes resulting from a clinical decision associated with the medical condition;
a data generation component (234) that:
identifies, based on analysis of the first data (212) and the second data (214), one or more cohorts (216) to a medical journey of the patient, and
generates, based at least on the first data (212), the second data (214) and the one or more cohorts (216) to the medical journey of the patient, third data (218) comprising at least two alternative treatment plans employable for subsequent treatment of the medical condition; and
a display component (236) that:
displays, on a user interface (UI) of a device, a patient timeline comprising a temporal view of a medical history of the patient, specific to the medical condition,
highlights, on the UI, the clinical decision and the one or more outcomes via a bounding box,
displays, on the UI, visual connections between the clinical decision and the one or more outcomes to highlight a progression of the medical journey of the patient based on the clinical decision,
displays, on the UI, a visualization (222) of the one or more cohorts (216) to the medical journey of the patient, wherein the visualization (222) is impactful in evaluating the at least two alternative treatment plans with reference to cohort outcomes, and wherein the visualization (222) is employable by a medical device to automatically execute, based on at least one cohort of the one or more cohorts (216) to the medical journey of the patient, an action related to the subsequent treatment of the medical condition, if the at least one cohort satisfies a set of defined criteria.

2. The system according to claim 1, wherein the computer-executable components further comprise:
a first artificial intelligence (AI) component (238) that detects, based on the first data (212), a plurality of decision points within the temporal view, wherein respective decision points represent respective clinical decisions corresponding to the patient, and wherein the clinical decision corresponds to a decision point within the temporal view.

3. The system according to claim 2, wherein detecting the plurality of decision points comprises:
identifying, by a plan seeker component (242), a first set of decision points based on modifications within a treatment plan associated with the medical condition;
identifying, by a detection component (244), a second set of decision points based on inputs associated with treatment decisions comprised in the treatment plan;
identifying, by an evaluation component (246), a third set of decision points based on the set of clinical rules, wherein the set of clinical rules comprises rules configured to detect defined clinical patterns; and
generating, by a merger component (248), a list comprising the plurality of decision points by merging the first set of decision points, the second set of decision points and the third set of decision points.

4. The system according to claim 3, wherein the computer-executable components further comprise:
a second artificial intelligence (AI) component (240) that detects the one or more outcomes, wherein detecting the one or more outcomes comprises:
selecting, by a selection component (250), the decision point associated with the clinical decision;
generating, by a prompt generation component (252), a set of summarization prompts to identify a set of events representing consequences of the clinical decision;
inputting, by the prompt generation component (252), the set of summarization prompts to a large language model (LLM); and
analyzing, by the merger component (248), results generated by the LLM upon processing the set of summarization prompts.

5. The system according to claim 2, wherein identifying the one or more cohorts (216) to the medical journey of the patient comprises:
summarizing, by the data generation component (234), the medical journey of the patient prior to the decision point;
comparing, by the data generation component (234), the medical journey of the patient to respective medical journeys of respective patients of the one or more additional patients; and
selecting, by the data generation component (234), based on relevant clinical criteria, a set of medical journeys from the respective medical journeys as the one or more cohorts (216) to the medical journey of the patient.

6. The system according to claim 5, wherein the data generation component (234) further generates cost information describing respective costs associated with the medical journey of the patient and with the respective medical journeys of the respective patients.

7. The system according to claim 5, wherein the display component (236) further displays, on the UI, the respective medical journeys of the respective patients via branching paths that branch from the decision point, such that the branching paths highlight an alternative progression of the medical journey of the patient resulting from an alternative clinical decision.

8. The system according to claim 1, wherein displaying the visualization (222) on the UI comprises:
outputting, by the display component (236), on the UI, first outcomes and outcome metrics for different choices of first treatment plans associated with respective cohorts of the one or more cohorts (216) to the medical journey of the patient.

9. The system according to claim 1, wherein the data generation component (234) further generates, based on the first patient data and the treatment information, a ranked list (220) of medical practitioners, wherein the ranked list (220) of medical practitioners is employable by an end entity to explore second treatment plans employed by the medical practitioners to treat the medical condition, explore second outcomes associated with the second treatment plans, and contact the medical practitioners.

10. The system according to claim 2, wherein the first AI component (238) further detects, based on the respective clinical decisions, one or more decision points of the plurality of decision points as respective key moments indicative of non-standard insights identified by the first AI component (238).

11. The system according to claim 2, wherein the computer-executable components further comprise:
a reporting component (254) that generates an AI-based report (224) comprising an explanation of information learned by the data generation component (234) based on outcomes associated with the respective clinical decisions and recommendations for the subsequent treatment.

12. The system according to claim 11, wherein the display component (236) highlights the outcomes via visual indicators and scores displayed on the patient timeline on the UI.

13. The system according to claim 1, wherein the computer-executable components further comprise:
a training component (256) that trains the data generation component (234) to iteratively analyze the first data (212) and the second data (214) for new information and identify, based on the new information, new cohorts that are most relevant to the medical journey of the patient at a given point in time.

14. A computer-implemented method, comprising:
accessing, by a system operatively coupled to a processor (204), first data (212) comprising first patient data associated with a patient, second patient data associated with one or more additional patients, a set of clinical rules, and treatment information related to a medical condition of the patient;
accessing, by the system, second data (214) comprising one or more outcomes resulting from a clinical decision associated with the medical condition;
analyzing, by the system, via generative AI, the first data (212) and the second data (214);
identifying, by the system, based on analysis of the first data (212) and the second data (214), via the generative AI, one or more cohorts (216) to a medical journey of the patient;
generating, by the system, based at least on the first data (212), the second data (214) and the one or more cohorts (216) to the medical journey of the patient, via the generative AI, third data (218) comprising at least two alternative treatment plans employable for subsequent treatment of the medical condition;
displaying, by the system, on a UI of a device, a patient timeline comprising a temporal view of a medical history of the patient, specific to the medical condition; and
displaying, by the system, on the UI, a visualization (222) of the one or more cohorts (216) to the medical journey of the patient, wherein the visualization (222) is impactful in evaluating the at least two alternative treatment plans with reference to cohort outcomes.

15. The computer-implemented method according to claim 14, further comprising:
detecting, by the system, based on the first data (212), via the generative AI, a plurality of decision points within the temporal view, wherein respective decision points represent respective clinical decisions corresponding to the patient, and wherein the clinical decision corresponds to a decision point within the temporal view.
